(19) Europäisches Patentamt
European Patent Office
Office européen des brevets

(11) EP 0 650 953 B1

(12) # FASCICULE DE BREVET EUROPEEN

(45) Date de publication et mention de la délivrance du brevet:
**04.06.1997 Bulletin 1997/23**

(51) Int Cl.[6]: **C07C 59/48**, A61K 31/19, C07C 59/56

(21) Numéro de dépôt: **94402399.3**

(22) Date de dépôt: **26.10.1994**

(54) **Analogues cyclisés de métabolites d'acides gras, leur procédé de préparation et les compositions pharmaceutiques les renfermant**

Cyclizierte Analoge der Fettsäurenmetaboliten, Verfahren zu ihrer Herstellung und diese enthaltende pharmazeutische Zusammensetzungen

Cyclised analogs of metabolites of fatty acids, process for their preparation and pharmaceutical compositions containing them

(84) Etats contractants désignés:
**AT BE CH DE DK ES FR GB GR IE IT LI LU NL PT SE**

(30) Priorité: **28.10.1993 FR 9312858**

(43) Date de publication de la demande:
**03.05.1995 Bulletin 1995/18**

(73) Titulaire: **ADIR ET COMPAGNIE**
**92415 Courbevoie Cédex (FR)**

(72) Inventeurs:
- **Gree, René**
  **F-35510 Cesson-Sevigne (FR)**
- **Hachem, Ali Mohammed**
  **F-35000 Rennes (FR)**
- **Gree, Danielle**
  **F-35510 Cesson-Sevigne (FR)**
- **Le Floc'h, Yves**
  **F-35830 Betton (FR)**
- **Rolland, Yves**
  **F-92170 Vanves (FR)**
- **Simonet, Serge**
  **F-78700 Conflans-St-Honorine (FR)**
- **Verbeuren, Tony**
  **F-78540 Vernouillet (FR)**

(56) Documents cités:
**US-A- 3 352 901**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 650 953 B1

## Description

La présente invention concerne de nouveaux analogues de métabolites d'acides gras, leur procédé de préparation ainsi que les compositions pharmaceutiques qui les contiennent.

De récentes études, (M. R. Buchanan *et al.*, *A.A.S.*, *37*, 273-281, (1992)), montrent que la production intracellulaire des métabolites de la voie de la lipoxygénase, l'acide 13-hydroxyoctadécadiènoïque (13-HODE, métabolite de l'acide linoléique) et l'acide 15-hydroxy-eicosatétraènoïque (15-HETE, métabolite de l'acide arachidonique), régulent les propriétés adhésives de ces cellules.

On peut donc en conclure que le rapport 13-HODE/15-HETE est en étroite relation avec le pouvoir de cellules cancéreuses malignes d'adhérer aux cellules endothéliales, probablement en interférant avec l'expression du récepteur de la vitronectine. De la même manière, ces métabolites de la voie de la lipoxygénase semblent être impliqués dans l'expression de la glycoprotéine $II_B/III_A$ plaquettaire, récepteur du fibrinogène, qui est le chaînon final dans le processus d'agrégation plaquettaire. Les métabolites de la lipoxygénase interfèrent également au niveau du récepteur plaquettaire du thromboxane pour lequel ils présentent une activité de type antagoniste (M. Croset *et al.*, *Biochem. Pharm.*, (37) 7, 1275-1280, (1988)).

La demanderesse a découvert de nouveaux analogues de métabolites d'acides gras doués de propriétés très intéressantes lorsqu'ils sont utilisés dans les pathologies d'adhésion et en particulier dans les phénomènes d'agrégation plaquettaire et dans le cancer.

Plus spécifiquement, la présente invention a pour objet les nouveaux analogues de métabolites d'acides gras répondant à la formule (I) :

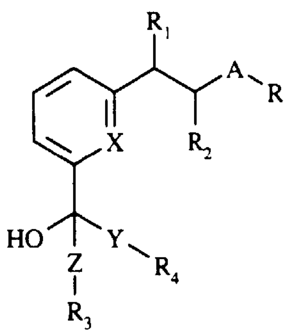

(I)

dans laquelle :

**A** représente un radical bivalent hydrocarboné, comprenant de 2 à 10 atomes de carbone, en chaîne linéaire ou ramifiée et comportant éventuellement une ou plusieurs insaturations sous forme de doubles et de triples liaisons,

**R** est choisi parmi un atome d'halogène, choisi parmi fluor, chlore, brome, iode, un radical -OR', -COOR', -COR', $P(O)(OR')_2$, -CH=N-OR', -CONHR', -CH=NR'', -CH=NAr, $-NHSO_2Ar$, -CON(OH)R', -NHR', -NHCOR', -CH=N-NHAr,

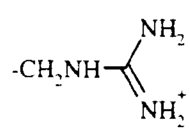

et un radical éventuellement substitué, imidazolyle, pyrazolyle ou tétrazolyle,

**R'** est choisi parmi un atome d'hydrogène et un radical alkyle linéaire ou ramifié contenant de 1 à 6 atomes de carbone,

**R''** représente un radical alkyle linéaire ou ramifié contenant de 1 à 6 atomes de carbone,

**Ar** représente un radical aryle éventuellement substitué, choisi parmi les radicaux phényle et naphtyle,

**$R_1$** et **$R_2$** représentent chacun un atome d'hydrogène ou forment ensemble une liaison,

**$R_3$** est choisi parmi un atome d'hydrogène ou un radical alkyle comprenant de 2 à 10 atomes de carbone en chaîne linéaire ou ramifiée,

**$R_4$** représente un radical hydrocarboné comprenant de 2 à 10 atomes de carbone en chaîne linéaire ou ramifiée et comportant éventuellement une ou plusieurs insaturations sous forme de doubles et de triples liaisons,

**X** représente le groupement CH ou un atome d'azote,

**Y** et **Z** représentent, indépendamment l'un de l'autre, un trait de valence ou un groupement paraphénylène,

étant entendu que le terme "éventuellement substitué" signifie que le radical considéré peut être éventuellement substitué par un ou plusieurs groupements choisis parmi chlore, fluor, brome, iode, amino, alkylamino, dialkylamino, nitro, cyano, alkyle, alkoxy, acyle, acyloxy, carboxy, alkoxycarbonyle, amido et carboxamido,
leurs stéréoisomères, leurs éventuels N-oxydes ainsi que leurs sels d'addition à un acide ou à une base, pharmaceutiquement acceptables.

La présente invention a également pour objet le procédé de préparation des dérivés de formule (I) caractérisé en ce que l'on protège les fonctions aldéhyde du composé de formule (II) :

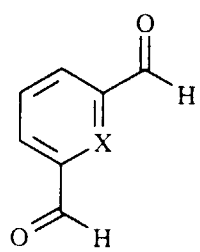

(II)

dans laquelle X est tel que défini dans la formule (I),
par l'orthoformiate de méthyle, à température ambiante, dans un solvant alcoolique anhydre, tel que le méthanol anhydre, en présence d'un sel d'ammonium, comme le nitrate d'ammonium, pour obtenir le bisacétal de formule (III) :

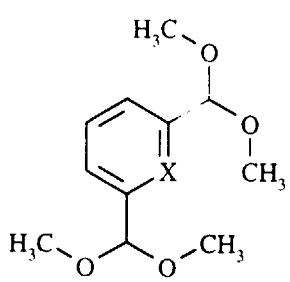

(III)

dans laquelle X est tel que décrit précédemment,
que l'on transforme en acétal aldéhyde de formule (IV) :

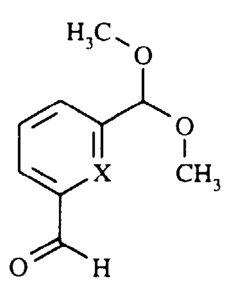

(IV)

dans laquelle X est tel que défini précédemment,
par action d'une solution aqueuse d'acide sulfurique en solvant organique, tel que le dichlorométhane, en présence de silice,
composé de formule (IV) que l'on fait réagir :

***soit*** : A) - lorsque le groupement Y défini dans la formule (I) représente une liaison **et** le radical $R_4$ du même dérivé de formule (I) souhaité contient une ou plusieurs insaturations (soit $R_{4A}$ ce radical),

avec du méthylsulfate de triméthylsulfonium, en présence d'une solution aqueuse d'hydroxyde de sodium, dans un solvant, tel que le dichlorométhane, à température ambiante, pour obtenir l'époxyde de formule ($V_A$) :

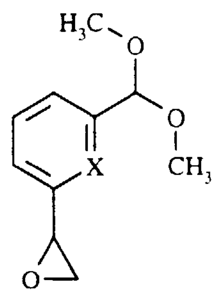

($V_A$)

dans laquelle X est tel que défini précédemment,
qui, mis au contact d'un composé de formule (VI) :

$$H\text{-}R_{5A} \qquad (VI)$$

dans laquelle $R_{5A}$ est tel que $-R_{4A}= -CH_2-R_{5A}$ où $R_{4A}$ est tel que défini précédemment,

ayant préalablement réagi avec une solution de butyllithium dans l'hexane en présence d'un chélatant des cations, tel que l'hexaméthylphosphotriamide (HMPA), dans un solvant polaire anhydre, tel que le tétrahydrofurane, à 0° C, conduit au composé de formule ($VI_A$) :

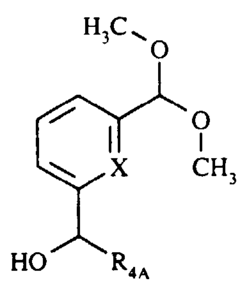

($VI_A$)

dans laquelle X et $R_{4A}$ sont tels que définis précédemment,

***soit*** : B) - lorsque le groupement Y du dérivé de formule (I) représente une liaison **et** le radical $R_4$ du même dérivé de formule (I) souhaité représente un groupement hydrocarboné saturé (soit $R_{4B}$ ce radical), avec le réactif de Grignard de formule ($V_B$) :

$$R_{5B}\text{-Mg-Hal} \qquad (V_B)$$

dans laquelle $R_{5B}$ est tel $R_{4B} = -CH_2-R_{5B}$ où $R_{4B}$ est tel que défini précédemment, et Hal est un atome d'halogène, tel que le brome,

dans le tétrahydrofurane anhydre, à une température de -50°C, pour conduire au dérivé de formule ($VI_B$) :

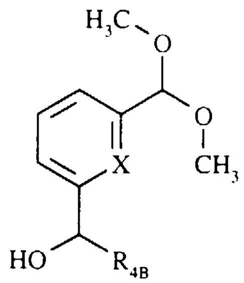

$(VI_B)$

dans laquelle X est tel que défini dans la formule (I) et $R_{4B}$ tel que défini dans la formule ($V_B$)

***soit*** : C) - lorsque le groupement Y du dérivé de formule (I) représente un groupement paraphénylène, avec le composé de formule ($V_C$) :

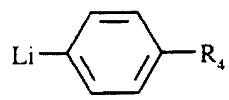

$(V_C)$

dans laquelle $R_4$ est tel que défini précédemment,

dans le tétrahydrofurane anhydre, à une température de -50°C, pour fournir le composé de formule ($VI_C$) :

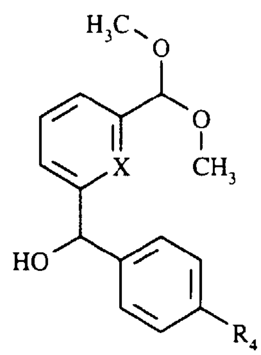

$(VI_C)$

dans laquelle X et $R_4$ sont tels que définis précédemment,

l'ensemble des composés de formules ($VI_A$), ($VI_B$) et ($VI_C$) formant l'ensemble des composés de formule (VI) :

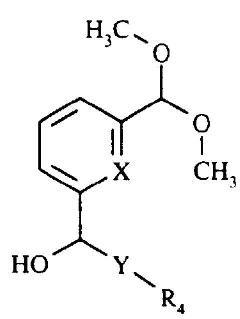

(VI)

dans laquelle X, Y et $R_4$ sont tels que définis précédemment,

que l'on peut, si on le souhaite, soumettre à une réaction d'oxydation, par un mélange de chlorure d'oxalyle et de diméthylsulfoxide, dans un solvant chloré, tel que le dichlorométhane, afin d'obtenir le composé de formule (VII) :

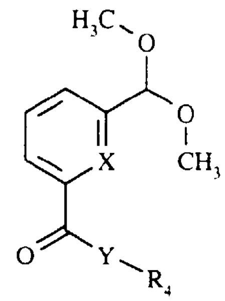

(VII)

dans laquelle X et $R_4$ sont tels que définis précédemment, qui est,

**soit** : A) traité par un réactif de formule ($VIII_A$) :

$$Li\text{-}R'_3 \qquad (VIII_A)$$

dans laquelle $R'_3$ représente un radical alkyle comprenant de 2 à 10 atomes de carbone en chaîne linéaire ou ramifiée,

dans les mêmes conditions que le composé de formule ($V_B$), pour conduire au composé de formule ($IX_A$) :

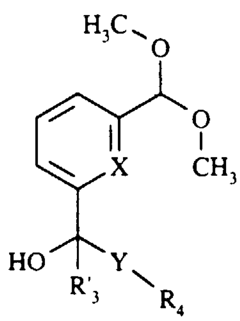

($IX_A$)

dans laquelle $R'_3$, $R_4$, X et Y sont tels que définis précédemment,

**soit** : B) traité par un réactif de formule ($VIII_B$) :

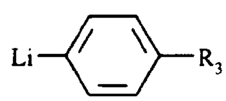

($VIII_B$)

dans laquelle $R_3$ est tel que défini précédemment,

dans les mêmes conditions que celle utilisées pour l'obtention du composé de formule ($VI_C$), pour conduire au composé de formule ($IX_B$) :

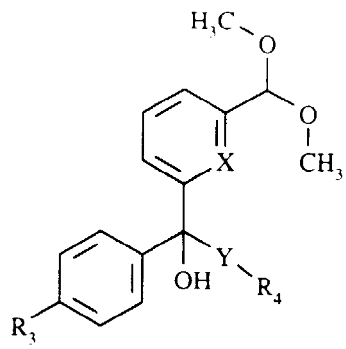

$(IX_B)$

dans laquelle $R_3$, $R_4$, X et Y sont tels que définis précédemment,

l'ensemble des composés de formules (VI), $(IX_A)$ et $(IX_B)$ formant l'ensemble des composés de formule (IX) :

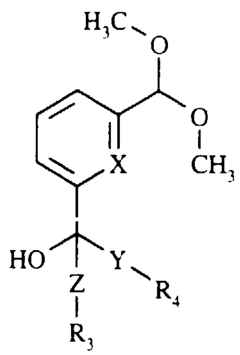

(IX)

dans laquelle $R_3$, $R_4$, X, Y et Z sont tels que définis précédemment,
composés de formule (IX) dont la fonction alcool est protégé par le chlorure de tertiobutyldiphénylsilyle, en présence d'imidazole dans un solvant, tel que le diméthylformamide, à température ambiante, pour donner le composé de formule (X) :

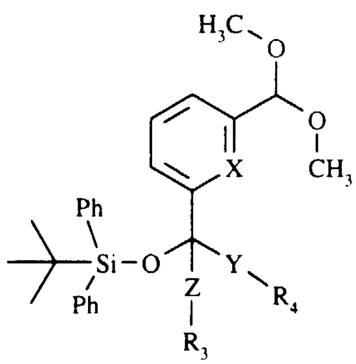

(X)

dans laquelle X, Y, Z, $R_3$ et $R_4$ sont tels que définis précédemment,
la fonction acétal du composé de formule (X) étant ensuite transformée de manière identique à celle utilisée pour le composé de formule (III), en aldéhyde de formule (XI) :

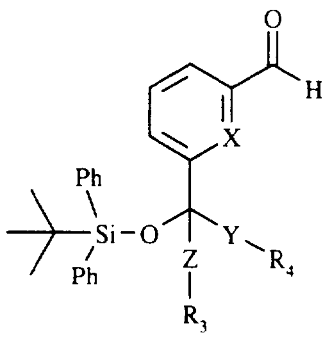

(XI)

dans laquelle X, Y, Z, $R_3$ et $R_4$ sont tels que définis précédemment, qui est ensuite engagé dans une réaction de Wittig avec un composé de formule (XII) :

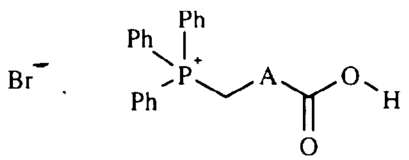

(XII)

dans laquelle A est tel que défini précédemment, préalablement mis en solution dans un solvant anhydre, tel que le tétrahydrofurane, en présence de bis(triméthylsilyl) amidure de lithium et de hexaméthylphosphotriamide (HMPA), à une température de -80°C, pour obtenir le composé de formule (XIIIa) :

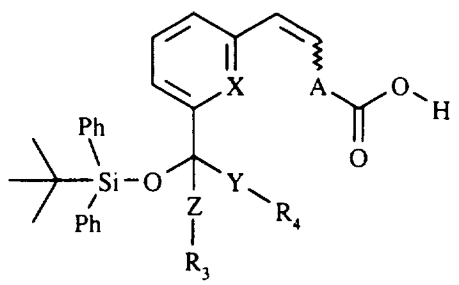

(XIIIa)

dans laquelle A, X, Y, Z, $R_3$ et $R_4$ sont tels que définis précédemment, qui peut être, si on le souhaite, transformé, selon une technique classique d'estérification, en composé de formule (XIIIb) :

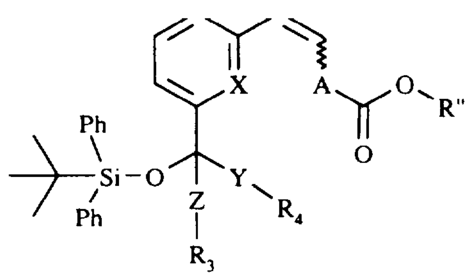

(XIIIb)

dans laquelle A, X, Y, Z, R'', $R_3$ et $R_4$ sont tels que définis précédemment, composés de formules (XIIIa) et (XIIIb), dont les isomères correspondants Z et E sont séparés sur colonne de silice, puis éventuellement engagés dans une réaction de déprotection de la fonction alcool, par action d'un fluorure de tétraalkylammonium, tel que le fluorure de tétrabutylammonium, dans un solvant polaire, tel que le tétrahydrofurane, à 0°C, pour obtenir respectivement les alcools de formules (XIVa) et (XIVb) :

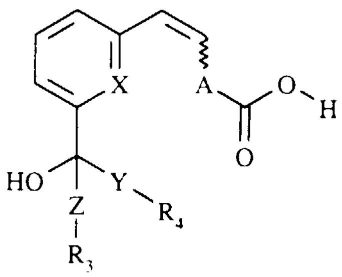 (XIVa) 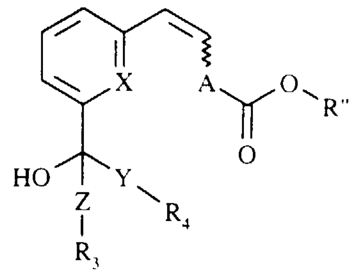 (XIVb)

dans lesquelles A, X, Y, Z, R", $R_3$ et $R_4$ sont tels que définis précédemment,
le composé de formule (XIVa) étant ensuite éventuellement transformé, après protection temporaire de la fonction alcool, en amide de formule (XIVc) :

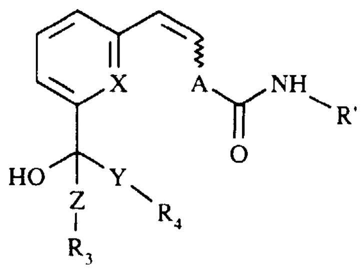 (XIVc)

dans laquelle A, X, Y, Z, R', $R_3$ et $R_4$ sont tels que définis précédemment,
l'ester de formule (XIIIb) pouvant également être directement saponifié selon la méthode décrite pour l'obtention de l'acide de formule (XIVa) puis éventuellement transformé en son chlorure d'acyle de formule (XIV) :

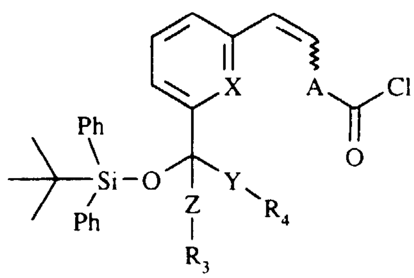 (XIV)

dans laquelle A, X, Y, Z, $R_3$ et $R_4$ sont tels que définis précédemment,
par action de chlorure d'oxalyle en présence de N,N-diméthylformamide dans le tétrahydrofurane, à O°C, chlorure d'acyle de formule (XIV) qui est immédiatement traité par un chlorhydrate de N-alkylhydroxylamine de formule (XV) :

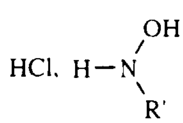 (XV)

dans laquelle R' est défini tel que précédemment,
en milieu basique, à 0°C, pour conduire, après libération de la fonction alcool, à l'acide hydroxamique de formule (XIVd) :

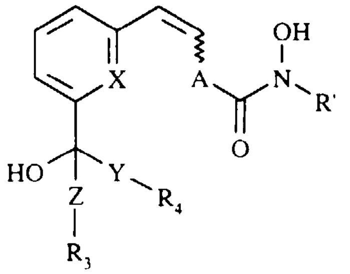

(XIVd)

dans laquelle A, X, Y, Z, R', $R_3$ et $R_4$ sont tels que définis précédemment, chlorure d'acyle de formule (XIV) qui peut également être immédiatement traité par une hydrazine de formule $H_2N$-NHAr dans laquelle Ar est tel que défini précédemment pour conduire, après libération de la fonction alcool, aux hydrazides correspondantes de formule (XIVe) :

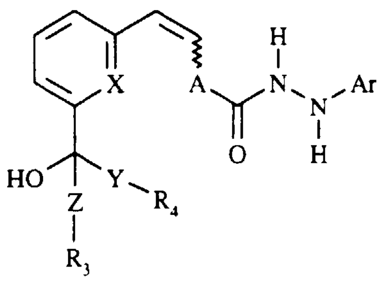

(XIVe)

dans laquelle A, X, Y, Z, Ar, $R_3$ et $R_4$ sont tels que définis précédemment, l'ester de formule (XIIIb) conduisant également, selon les techniques classiques de réduction et après libération de l'alcool protégé sous forme d'éther silylé, aux alcool et éther de formule (XIVf) :

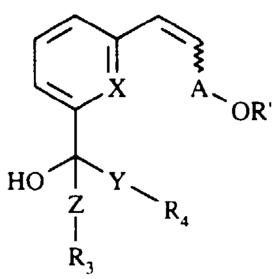

(XIVf)

dans laquelle A, X, Y, Z, R', $R_3$ et $R_4$ sont tels que définis précédemment, conduisant eux-mêmes, après protection temporaire de la fonction alcool α-aromatique, aux dérivés halogénés de formule (XIVg) :

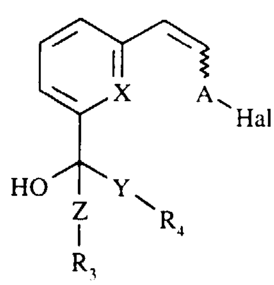

(XIVg)

dans laquelle A, X, Y, Z, $R_3$ et $R_4$ sont tels que définis précédemment,

et Hal représente un atome d'halogène choisi parmi fluor, chlore, brome et iode,

permettant la préparation des phosphonates de formule (XIVh) :

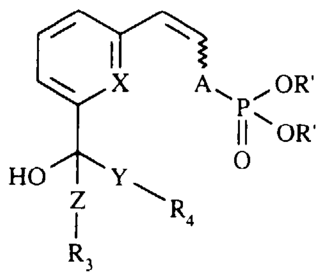

(XIVh)

dans laquelle A, X, Y, Z, R', $R_3$ et $R_4$ sont tels que définis précédemment, ou, par réaction sur un composé de formule (XVI) :

$$H\text{-}B_1 \qquad (XVI)$$

dans laquelle $B_1$ représente le groupement -NHR",

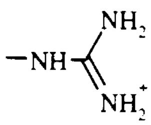

,

imidazolyle éventuelement substitué, pyrazolyle éventuellement substitué, ou le groupement tétrazolyle éventuellement substitué, et R" étant tel que précédemment défini,
dans des conditions opératoires classiques et appropriées, permettant la préparation des composés de formule (XVIa) :

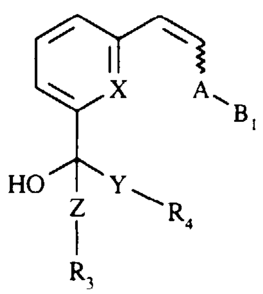

(XVIa)

dans laquelle A, $B_1$, X, Y, Z, $R_3$ et $R_4$ sont tels que définis précédemment,
les amines de formule (XVIb) :

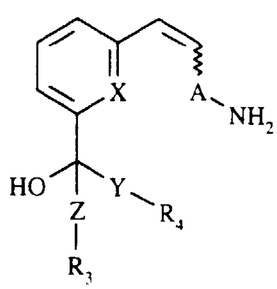

(XVIb)

dans laquelle A, X, Y, Z, $R_3$ et $R_4$ sont tels que définis précédemment,
étant alors obtenues par préparation de l'azide, par exemple avec l'azoture de sodium, à partir du composé halogéné précurseur de formule (XIVg), puis hydrogénation de cet azide, par exemple à l'aide de triphénylphosphine et d'eau, amines de formule (XVIb) qui peuvent être transformées, par action d'un composé de formule (XVI') :

Hal-$B_2$ (XVI')

dans laquelle $B_2$ représente -$SO_2$Ar ou -COR' et Hal, Ar et R' sont tels que définis précédemment, en composés de formule (XVIc) :

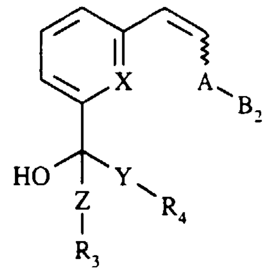

(XVIc)

dans laquelle A, $B_2$, X, Y, Z, $R_3$ et $R_4$ sont tels que définis précédemment,
l'ensemble des composés de formules (XVIa), (XVIb) et (XVIc) formant l'ensemble des composés de formule (XIVi) :

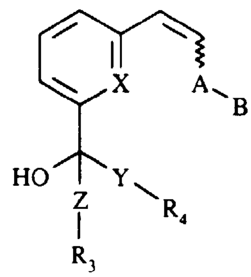

(XIVi)

dans laquelle A, X, Y, Z, $R_3$ et $R_4$ sont tels que définis précédemment, et B représente $B_1$, $B_2$ ou le groupement $NH_2$,
l'ester de formule (XIIIb) pouvant être également :

1/ - saponifié en acide correspondant puis traité par un composé de formule Li-R", dans laquelle R" est tel que décrit précédemment pour conduire, après libération de la fonction alcool jusqu'alors protégée sous forme d'éther silylé, aux cétones de formule (XIVj) :

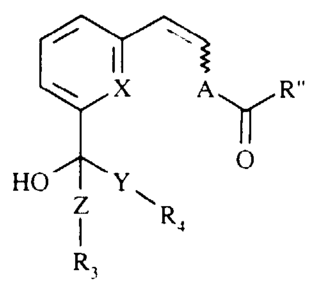

(XIVj)

dans laquelle A, X, Y, Z, R", $R_3$ et $R_4$ sont tels que définis précédemment,

2/ - traité par un équivalent d'hydrure de diisobutylaluminium (DIBAL) à basse température, pour conduire après libération de la fonction alcool, aux aldéhydes de formule (XIVk) :

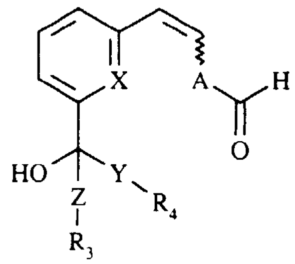

(XIVk)

dans laquelle A, X, Y, Z, $R_3$ et $R_4$ sont tels que définis précédemment,

ces aldéhydes de formule (XIVk) pouvant être :

***soit :*** α) traités par l'hydroxylamine pour conduire aux oximes de formule (XIVl) :

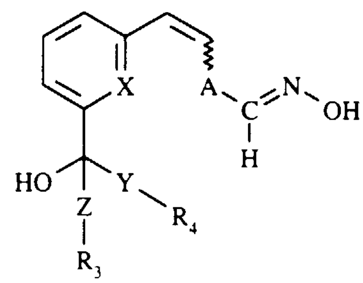

(XIVl)

dans laquelle A, X, Y, Z, $R_3$ et $R_4$ sont tels que définis précédemment,
les oximes de formule (XIVl) pouvant elles-mêmes être alkylées en composés de formule (XIVm) :

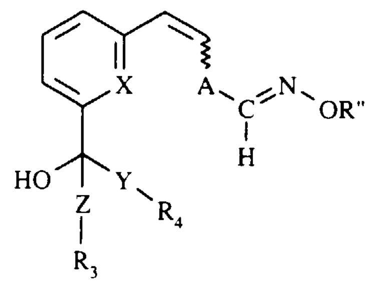

(XIVm)

dans laquelle A, X, Y, Z, R'', $R_3$ et $R_4$ sont tels que définis précédemment,
par un réactif de formule W-R'' dans laquelle W représente un groupement partant, tel qu'un halogène ou un groupement sulfate, et R'' est tel que défini précédemment,
***soit*** : β) traités par une amine de formule $H_2N$-R'', dans laquelle R'' est tel que défini précédemment, pour conduire aux imines de formule (XIVn) :

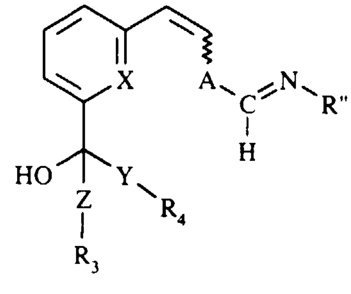

(XIVn)

dans laquelle A, X, Y, Z, R'', $R_3$ et $R_4$ sont tels que définis précédemment,

l'ensemble des dérivés de formules (XIVa) à (XIVn) formant l'ensemble des dérivés de formule (XVII) :

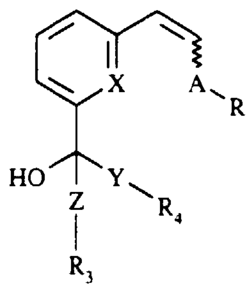

(XVII)

dans laquelle A, X, Y, Z, R, $R_3$ et $R_4$ sont tels que définis précédemment,
qui, éventuellement soumis à hydrogénation selon une technique classique, par exemple catalytique à l'aide de palladium, conduisent aux dérivés de formule (XVIII) :

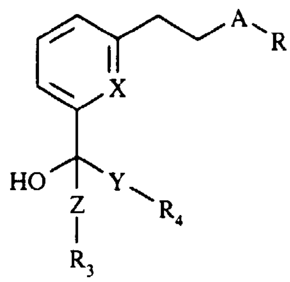

(XVIII)

dans laquelle A, X, Y, Z, R, $R_3$ et $R_4$ sont tels que définis précédemment,
l'ensemble des dérivés de formules (XVII) et (XVIII) formant l'ensemble des dérivés de formule (I) que l'on purifie le cas échéant selon une technique classique de purification et dont on sépare, si on le souhaite, les isomères par une technique classique de séparation et que l'on transforme, éventuellement en leurs N-oxydes et/ou en leurs sels d'addition à un acide ou à une base pharmaceutiquement acceptables.

Les composés de formule (I) dans laquelle X représente un atome d'azote peuvent également être préparés à partir de la 2,6-diméthanol-pyridine dont on protège une des fonctions alcool par le chlorure de tertiobutyldiphénylsilyle pour obtenir le composé de formule (XIX) :

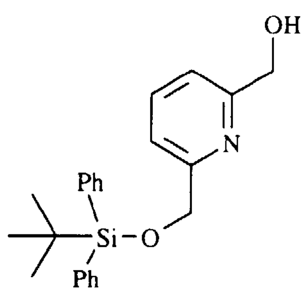

(XIX)

qui est ensuite oxydé, par exemple par un mélange de chlorure d'oxalyle et de diméthylsulfoxyde dans le dichlorométhane, en aldéhyde de formule (XX) :

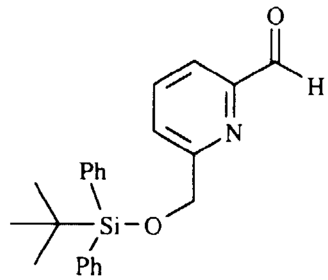

(XX)

qui est ensuite engagé dans la réaction de Wittig décrite pour l'obtention du composé de formule (XIIIb), pour conduire au composé de formule (XXI) :

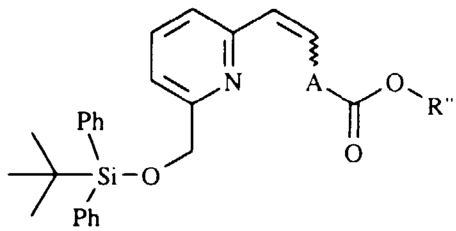

(XXI)

dans laquelle A et R" sont tels que définis précédemment,

dont la fonction alcool est déprotégée selon la méthode utilisée pour l'obtention du composé de formule (XIVa), puis oxydée en aldéhyde selon la méthode décrite pour le composé (XVIII), conduisant ainsi au composé de formule (XXII) :

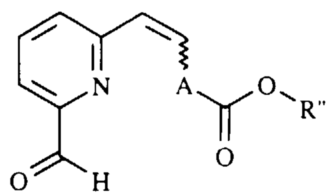

(XXII)

dans laquelle A et R" sont tels que définis précédemment,

qui est ensuite traité de manière analogue au composé de formule (IV), selon les valeurs de Y et de $R_4$, pour donner les composés de formule (XXIII) :

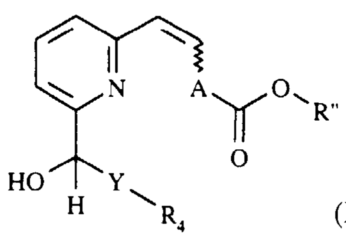

(XXIII)

dans laquelle A, Y, R" et $R_4$ sont tels que définis précédemment,

qui sont ensuite éventuellement traités de manière analogue aux composés de formule (VI) afin d'obtenir les composés de formule (XXIV) :

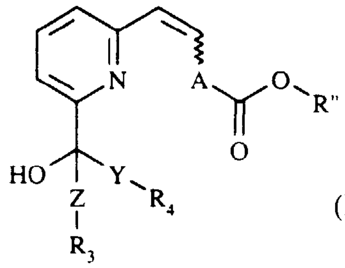

(XXIV)

dans laquelle A, Y, Z, R", $R_3$ et $R_4$ sont tels que définis précédemment, dont la fonction ester est éventuellement modifiée de façon identique aux méthodes employées pour l'obtention des composés (XIVa) à (XIVn) pour fournir les composés de formule (XXV) :

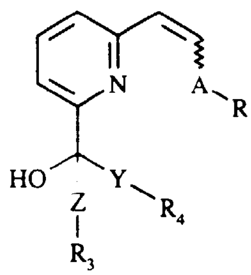

(XXV)

dans laquelle A, Y, Z, R, $R_3$ et $R_4$ sont tels que définis précédemment, qui sont éventuellement soumis à hydrogénation, selon la technique décrite pour l'hydrogénation des composés de formmule (XVII), afin d'obtenir les composés de formule (XXVI) :

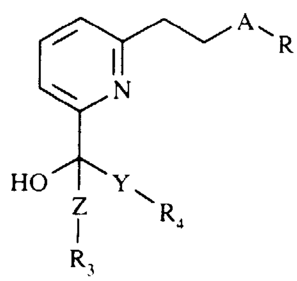

(XXVI)

dans laquelle A, Y, Z, R, $R_3$ et $R_4$ sont tels que définis précédemment, l'ensemble des composés de formule (XXV) et (XXVI) formant l'ensemble des composés de formule (XXVII) :

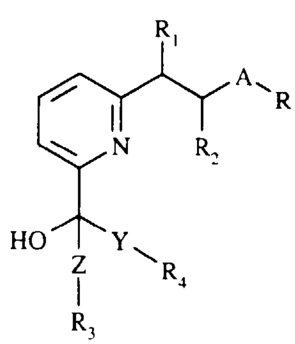

(XXVII)

dans laquelle A, R, $R_1$, $R_2$, $R_3$, $R_4$, Y, et Z sont tels que définis précédemment, cas particulier du composé de formule (I) dans laquelle X représente l'azote.

Les composés pour lesquels les radicaux A, $R_4$ ou $R_3$ contiennent une ou plusieurs doubles liaisons peuvent avantageusement être obtenus à partir de leurs précurseurs respectifs dans lesquels A, $R_4$ ou $R_3$ comportent des

triples liaisons. Ces composés sont obtenus par hydrogénation catalytique, par exemple à l'aide d'hydrogène et de catalyseur de Lindlar, à tout instant de la synthèse jugé opportun par l'homme de l'art.

Les aldéhydes de formule (XIVk) peuvent également être obtenus à partir des alcools de formule (XXVIII) :

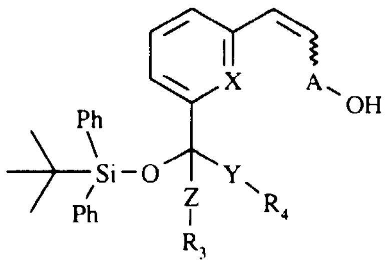

(XXVIII)

dans laquelle A, X, Y, Z, $R_3$ et $R_4$ sont tels que définis précédemment,

par action d'un agent oxydant, tel que le chlorochromate de pyridinium en présence d'acétate de sodium dans le chlorure de méthylène.

Les composés de l'invention possèdent de façon surprenante des propriétés d'antiagrégants plaquettaires très importantes. Ces composés présentent donc une action particulièrement bénéfique dans les affections où intervient un processus d'agrégation plaquettaire. Ainsi, ces nouveaux composés peuvent être utilisés dans le traitement des affections dues ou reliées à des pathologies d'adhésion plaquettaire et notamment dans les cas de thromboembolisme, par dissolution des caillots de sang, de l'embolie pulmonaire, de l'embolie artérielle des extrémités, de l'infarctus du myocarde, de l'athérosclérose, de cancer malin, ainsi que pour maintenir l'homéostasie sanguine, en particulier dans la circulation extracorporelle.

Ils peuvent également être utilisés en tant qu'agents préventifs de l'extension du processus thrombotique en les utilisant comme anticoagulants d'action directe et rapide.

La présente invention a également pour objet les compositions pharmaceutiques contenant un dérivé de formule (I), ou un de ses sels d'addition à un acide ou une base pharmaceutiquement acceptables, seul ou en combinaison avec un ou plusieurs excipients inertes et non toxiques. Parmi les compositions pharmaceutiques selon l'invention, on pourra citer plus particulièrement celles qui conviennent à l'administration orale, parentérale, nasale, rectale, perlinguale, oculaire ou pulmonaire et notamment les préparations injectables, les aérosols, les gouttes oculaires ou nasales, les comprimés simples, pelliculés ou dragéifiés, les gélules, les suppositoires, les capsules, les crèmes, pommades, gels dermiques,...

La posologie utile varie selon l'âge et le poids du patient, la voie d'administration, la nature de l'affection et des traitements éventuels associés et s'échelonne entre 0,5 mg et 2 grammes par 24 heures.

Les exemples suivants illustrent l'invention sans toutefois la limiter en aucune façon.

Les matières premières sont disponibles ou préparées à partir de modes opératoires connus.

PREPARATION A :

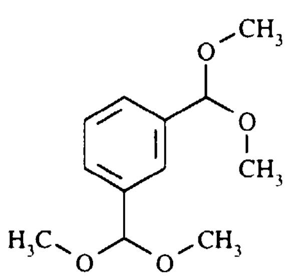

25 g d'isophtalaldéhyde (180 mmoles), 78 g d'orthoformiate de méthyle (4 équivalents), 250 ml de méthanol anhydre, puis 600 mg de nitrate d'ammonium sont introduits dans un ballon. Le mélange est porté à reflux pendant 2 heures, puis le méthanol est évaporé sous vide, les résidus sont dissous dans l'éther et le nitrate d'ammonium est éliminé par filtration, l'éther est à nouveau éliminé sous pression réduite et le bisacétal est purifié par distillation. On obtient 38 g (167,4 mmoles) d'une huile incolore correspondant au produit attendu.

Rendement : 93 %

Température d'ébullition (sous 3 mm de Hg) = 118°C

PREPARATION B :

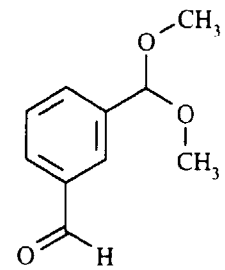

40 g de silice sont mis en suspension dans 140 ml de dichlorométhane auxquels sont ajoutés 15 gouttes d'une solution aqueuse d'acide sulfurique (1% en poids). 10 g de bisacétal de la préparation A sont ensuite ajoutés et le mélange est agité fortement à température ambiante pendant 45 minutes puis filtré. Les solvants sont alors évaporés sous pression réduite et le produit brut est purifié par chromatographie sur colonne de silice (éluant : éther/éther de pétrole, 20:80 avec 1% de triéthylamine). On obtient 7 g d'acétal aldéhyde attendu, sous forme d'huile incolore.
Rendement : 88 %

PREPARATION C :

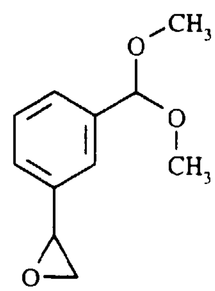

7 g (38,8 mmoles) de l'acétal aldéhyde, obtenu à la préparation B, sont mis en solution dans 70 ml de dichlorométhane. 16 g (85,0 mmoles) de méthylsulfate de triméthylsulfonium, puis 35 ml d'une solution aqueuse d'hydroxyde de sodium (50% en poids) sont ajoutés au mélange. Après forte agitation à température ambiante pendant 4 h, le milieu réactionnel est extrait au dichlorométhane. Après traitement habituel de la phase organique, le produit brut est purifié par chromatographie sur colonne de silice (éluant : éther/éther de pétrole, 20:80 avec 1 % de triéthylamine). 6,5 g d'une huile incolore correspondant à l'époxyde attendu sont obtenus.
Rendement : 85%

**Exemple 1: 1-[(1*Z*)-5-méthoxycarbonylpent-1-ényl]-3-(1-hydroxynon-3-ynyl)benzène**

Etape 1a : 1-diméthoxyméthyl-3-(1-hydroxynon-3-ynyl)benzène

8,6 ml de hept-1-yne (65.5 mmoles) en solution dans 30 ml de tétrahydrofurane anhydre, sont additionnés, à 0° C, goutte à goutte, sous azote et agitation magnétique, de 19 ml (47.5 mmoles) d'une solution 2.5 M de butyllithium dans l'hexane. Après 15 mn d'agitation à 0° C, 30 ml d'hexaméthylphosphotriamide (HMPA) sont ajoutés puis, lentement, 4.7 g (24.2 mmoles) d'epoxyde obtenu dans la préparation C, en solution dans 35 ml de tétrahydrofurane. L'ensemble est abandonné jusqu'à retour à température ambiante. Après traitement habituel de la phase organique, le produit brut est purifié par chromatographie sur colonne de silice (éluant : éther/éther de pétrole, 50:50 avec 1 % de triéthylamine). 6,8 g d'une huile incolore sont recueillis, correspondants à l'alcool attendu.
Rendement : 97 %

Etape 1b : Protection de la fonction alcool

A 6 g (20,7 mmoles) d'alcool obtenu à l'étape 1a en solution dans 100 ml de diméthylformamide anhydre sont

ajoutés ensuite, sous azote et sous agitation magnétique, 3,5 g (51,4 mmoles) d'imidazole puis 6,4 ml (24,6 mmoles) de chlorure de diphényltertiobutylsilyle. Après 24 h d'agitation à température ambiante, le milieu est hydrolysé avec une solution aqueuse de hydrogénocarbonate de sodium à 2 %. Le traitement des phases organiques fournit un produit brut qui est purifié par chromatographie sur colonne de silice (éluant : éther/éther de pétrole, 5:95 puis 10:90 avec 1 % de triéthylamine). On obtient 10,7 g d'éther silylé.
Rendement : 97 %

Etape 1c : Déprotection de la fonction acétal

A une suspension de 50 g de silice dans 100 ml de chlorure de méthylène est ajouté 1 ml d'une solution aqueuse d'acide sulfurique à 2.5 % en poids. Le mélange est agité fortement quelques minutes puis additionné de 10,7 g (20,2 mmoles) d'acétal préparé à l'étape 1b en solution dans 100 ml de dichlorométhane. Après forte agitation pendant 1 h 30, le mélange est filtré, le solvant évaporé sous vide et le produit brut est purifié par flash chromatographie (éluant : éther/éther de pétrole, 10:90). 9,6 g d'aldéhyde attendu sont obtenus sous forme d'huile incolore.
Rendement : 98 %

Etape 1d : 1-[(1*Z*)-5-méthoxycarbonylpent-1-ényl]-3-(1-hydroxynon-3-ynyl)benzène

6,3 g (13,95 mmoles) de bromure de (4-carboxybutyl) triphénylphosphonium sont séchés à la pompe (3-4 mmHg) dans un bain d'huile à 80°C, puis mis en suspension, sous azote, dans 200 ml de tétrahydrofurane anhydre. Le mélange est refroidi à -10°C et 4,5 g de bis(triméthylsilyl)amidure de lithium (27,9 mmoles) sont ajoutés par portions sous azote et sous agitation magnétique (la solution devient orange puis rouge foncé après 20-30 mn d'agitation) ; le mélange est alors refroidi à -35°C pour ajouter 19 ml d'hexaméthylphosphotriamide (HMPA) anhydre, puis à -80°C pour ajouter lentement 4,5 g (9,3 mmoles) d'aldéhyde obtenu à l'étape 1c dans 30 ml de tétrahydrofurane anhydre. La solution est agitée à -80°C pendant 1 h 30 puis à -35°C et 3 g de carbonate de sodium et 5,8 g de sulfate de diméthyle (4.4 ml) sont ajoutés ; le mélange est abandonné jusqu'à retour à température ambiante sous lente agitation pendant 12 heures. Après hydrolyse avec une solution aqueuse saturée de chlorure d'ammonium, et traitement habituel de la phase organique, 5,1 g d'un mélange des deux isomères (Z/E = 80/20) qui sont séparés par chromatographie sur colonne de silice (éluant : éther/éther de pétrole, 5:95) sont obtenus. 1,7 g (2,93 mmoles) de l'isomère Z est mis en solution dans 50 ml de tétrahydrofurane, auquel sont ajoutés, à 0°C, 3.5 ml (3,85 mmoles) d'une solution 1,1 M de fluorure de tétrabutylammonium dans le tétrahydrofurane. Après 1 h d'agitation à 0°C, le mélange est abandonné à température ambiante pendant 14 h puis hydrolysé, le milieu réactionnel est alors extrait à l'éther (3 fois) ; les phases organiques sont traités selon la méthode habituelle et 940 mg du produit titre sont obtenus sous forme d'huile incolore.
Rendement : 88 %
Caractéristiques spectrales de RMN du proton : (90 MHz, $CDCl_3$, δ ppm) :
7.42-7.08 (m, 4H aromatiques) ; 6.45 (dt, 1H, $ArC\underline{H} = CH-$, J = 11.7, 1.7 Hz) ; 5.63 (dt, 1H, $ArCH = C\underline{H}CH_2-$, J = 11.6, 7.3 Hz) ; 4.79 (t, 1H, $HOC\underline{H}CH_2-$, J = 6.3 Hz) ; 3.63 (s, 3H, $-CO_2C\underline{H}_3$) ; 2.71-2.50 (m, 2H, $HOCHC\underline{H}_2\ C\equiv C-$) ; 2.47-2.20 (m, 7H, $-C\underline{H}_2CO_2CH_3$ ; $-C\equiv CC\underline{H}_2CH_2-$, $ArCH = CHC\underline{H}_2-$ et $\underline{H}OCH-$) ; 1.95-1.61 (m, 2H, $-C\underline{H}_2CH_2CO_2CH_3$) ; 1.61-1.13 (m, 6H, $-CH_2(C\underline{H}_2)_3CH_3$) ; 0.89 (t, 3H, $-CH_2C\underline{H}_3$, J = 6.0 Hz).

**Exemple 2: 1-[(1*E*)-5-méthoxycarbonylpent-1-ényl]-3-(1-hydroxynon-3-ynyl)benzène**

Composé obtenu selon le mode opératoire décrit pour l'exemple 1 en isolant l'isomère E par chromatographie sur colonne de silice.

**Exemple 3: 1-[(1*Z*)-5-hydroxycarbonylpent-1-ényl]-3-(1-hydroxynon-3-ynyl)benzène**

880 mg (2,57 mmoles) du composé de l'exemple 1 sont mis en solution dans 30 ml de tétrahydrofurane auxquels sont additionnés 30 ml d'une solution aqueuse d'hydroxyde de lithium (2M). Le mélange est agité fortement à température ambiante pendant 15 h puis acidifié avec 5 ml d'acide acétique ; le milieu réactionnel est alors extrait à l'éther (3 fois), les phases organiques traitées de manière habituelle, et l'acide obtenu est purifié par chromatiographie sur colonne de silice (éluant : éther/éther de pétrole, 50:50 puis 70:30). 730 mg d'hydroxyacide attendu, sous forme d'huile incolore qui cristallise lentement, sont obtenus.
Rendement : 86 %
Point de fusion : 44°C

**<u>Exemple 4</u>: Sel de sodium de l'acide de l'exemple 3**

A 88,6 mg (2,21 mmoles) d'hydroxyde de sodium sont ajoutés 728 mg (2,21 mmoles) d'acide de l'exemple 3 en solution dans le méthanol. Le ballon est secoué à la main jusqu'à dissolution totale de l'hydroxyde de sodium, le méthanol est alors évaporé sous vide et le précipité blanc obtenu est lavé à l'hexane puis séché. On obtient quantitativement le sel de sodium attendu sous forme de poudre blanche.
<u>Point de fusion</u> : 130-135°C

**<u>Exemple 5</u>: 1-[(1*Z*)-5-méthoxycarbonylpent-1-ényl]-3-[(3*Z*)-1-hydroxynon-3-ényl]benzène**

<u>Etape 5a</u> : Hydrogénation catalytique du composé obtenu à l'étape 1c

4,5 g (9,32 mmoles) de l'aldéhyde obtenu à l'étape 1c sont mis en solution dans 100 ml de n-hexane ; 1 g de catalyseur de Lindlar puis 5 ml de pyridine sont ajoutés. Le mélange est agité à température ambiante sous atmosphère d'hydrogène pendant 1 heure 20 minutes, puis filtré, le filtrat lavé avec une solution diluée d'acide chloridrique puis séché (sulfate de magnésium), les solvants sont ensuite évaporés sous vide et le produit brut est purifié par chromatographie sur colonne de silice (éluant : éther/éther de pétrole, 10:90 puis 20:80). 4,4 g d'aldéhyde sous forme d'huile incolore sont obtenus.
<u>Rendement</u> : 97%

<u>Etape 5b</u> : 1-[(1*Z*)-5-méthoxycarbonylpent-1-ényl]-3-[(3*Z*)-1-hydroxynon-3-ényl]benzène

Ce produit est préparé selon un mode opératoire identique à celui décrit pour l'ester de l'étape 1d. A partir de 4,2 g d'aldéhyde obtenus à l'étape 5a, on obtient 4,4 g d'un mélange de deux isomères Z et E (Z/E : 90/10) qui sont séparés par chromatographie sur colonne de silice (éluant : éther/éther de pétrole, 5:95). L'isomère Z est engagé dans la réaction de déprotection de la fonction alcool pour obtenir l'hydroxyester attendu sous forme d'huile incolore.
<u>Rendement</u> : 77 %
<u>Caractéristiques spectrales de RMN du proton (90 MHz, $CDCl_3$, δ ppm)</u> :
7.37-7.07 (m, 4H aromatiques) ; 6.45 (d, 1H, $ArC\underline{H}{=}CH-$, J = 11.6 Hz) ; 5.77-5.22 (m, 3H, $ArCH{=}C\underline{H}-$ ; $-CH_2C\underline{H}{=}C\underline{H}CH_2-$) ; 4.66 (t, 1H, $HOC\underline{H}CH_2-$, J = 6.7 Hz) ; 3.61 (s, 3H, $-CO_2C\underline{H}_3$) ; 2.62-2.24 (7H : m, 4H, $-C\underline{H}_2CH{=}CHC\underline{H}_2-$ ; t, 2H, $-C\underline{H}_2CO_2CH_3$, J = 6.7 Hz et $\underline{H}OCH-$) ; 2.13-1.61 (m, 4H, $-C\underline{H}_2C\underline{H}_2CH_2CO_2CH_3$) ; 1.40-1.09 [m, 6H, $-CH_2\,(C\underline{H}_2)_3CH_3$] ; 0.87 (t, 3H, $-CH_2C\underline{H}_3$, J = 6.3 Hz).

**<u>Exemple 6</u>: 1-[(1*E*)-5-méthoxycarbonylpent-1-ényl]-3-[(3*Z*)-1-hydroxynon-3-ényl]benzène**

Composé obtenu de manière identique au composé de l'exemple 5, après isolation de l'isomère E dans l'étape 5b.

**<u>Exemple 7</u>: 1-[(1*Z*)-5-hydroxycarbonylpent-1-ényl]-3-[(3*Z*)-1-hydroxynon-3-ényl]benzène**

Même procédé que celui décrit pour le composé de l'exemple 3.
<u>Caractéristiques spectrales de RMN du proton (90 MHz, $CDCl_3$, δ ppm)</u> :
7.41-7.04 (m, 4H aromatiques) ; 6.47 (d, 1H, $ArC\underline{H}{=}CH-$, J = 11.6 Hz) ; 5.96 (large, 1H, $-CO_2\underline{H}$) ; 5.80-5.21 (m, 3H, $ArCH{=}C\underline{H}-$ $-CH_2C\underline{H}{=}C\underline{H}CH_2-$) ; 4.68 (t, 1H, $HOC\underline{H}CH_2-$, J = 6.7 Hz) ; 2.60-2.23 (m, 6H, $-C\underline{H}_2CO_2H$ ; $-C\underline{H}_2CH{=}CHC\underline{H}_2-$) ; 2.05-1.67 (m, 5H, $-C\underline{H}_2C\underline{H}_2CH_2CO_2H$ ; $\underline{H}OCH-$) ; 1.43-1.09 [m, 6H, $-CH_2(C\underline{H}_2)_3CH_3$] ; 0.87 (t, 3H, $-CH_2C\underline{H}_3$, J = 5.7 Hz).

**<u>Exemple 8</u>: Sel de sodium de l'exemple 7**

Procédé identique à celui décrit pour l'exemple 4, à partir de l'acide obtenu à l'exemple 7.
<u>Caractéristiques spectrales de RMN du proton (90 MHz, $D_2O$, δ ppm)</u> :
7.50-7.28 (m, 4H aromatiques) ; 6.59 (d, 1H, $ArC\underline{H}{=}CH-$ ; J = 11.2 Hz) ; 5.92 (dt, 1H, $ArCH{=}C\underline{H}CH_2$, J = 11.2 ; 7.3 Hz) ; 5.66-5.33 (m, 2H, $-CH_2C\underline{H}{=}C\underline{H}CH_2-$) ; 4.86 (t, 1H, $HOC\underline{H}-$, J = 6.7 Hz) ; 2.86-2.29 (m, 6H, $-C\underline{H}_2CO_2^-Na^+$ ; $-C\underline{H}_2CH{=}CHC\underline{H}_2-$) ; 2.14-1.78 (m, 4H, $-C\underline{H}_2C\underline{H}_2CH_2CO_2^-Na^+$ ) ; 1.48-1.03 [m, 6H, $-CH_2\,(C\underline{H}_2)_3CH_3$] ; 0.88 (t, 3H, $-CH_2C\underline{H}_3$, J = 5.8 Hz).

**Exemple 9: (5*Z*)-6-[3-(1-hydroxyhexyl)phényl]hex-5-énoate de méthyle**

Etape 9 a : 1-diméthoxyméthyl-3-(1-hydroxyhexyl) benzène

1,84 g (86,4 mmoles) de magnésium métallique, séché à la flamme puis refroidi sous azote est additionné de 54 ml de tétrahydrofurane anhydre et un cristal d'iode. Le mélange est chauffé à reflux, la couleur jaune de l'iode disparaît, 11 ml (86,4 mmoles) de 1-bromopentane en solution dans 50 ml de tétrahydrofurane anhydre sont additionnés . A la fin de l'addition, le chauffage est poursuivi pendant environ 20 minutes (consommation presque totale du magnésium), le mélange est refroidi sous azote, à température ambiante. A une solution de 8,9 g (49,4 mmoles) d'acétalaldéhyde (préparation B) dans 90 ml de tétrahydrofurane anhydre refroidie à -50° C, sont ajoutés, lentement, sous azote et agitation magnétique, la solution du magnésien préparée précédemment ; le mélange revenu à température ambiante, est hydrolysé avec une solution aqueuse saturée de chlorure d'ammonium, le milieu réactionnel est alors extrait à l'éther (3 fois). Le traitement des phases organiques fournit, après purification par chromatographie sur colonne de silice (éluant : éther/éther de pétrole, 50:50 avec 1% de triéthylamine), 9,8 g d'alcool attendu sous forme d'huile incolore.
Rendement : 78 %

Etape 9 b : Protection de la fonction alcool

Même procédé que celui décrit à l'étape 1b. A partir de 6,1 g d'alcool obtenu à l'étape 9a, sont isolés après chromatographie , 9,7 g d'éther silylé, sous forme d'huile incolore.
Rendement : 82%

Etape 9 c : Déprotection de la fonction acétal

Mode opératoire identique à celui décrit à l'étape 1c. A partir de 9,7 g d'acétal obtenu dans l'étape 9b, 8,4 g d'aldéhyde sous forme d'huile incolore sont obtenus.
Rendement : 96%

Etape 9d : (5Z)-6-[3-(1-hydroxyhexyl)phényl]hex-5-énoate de méthyle

Mode opératoire identique à celui décrit à l'étape 1d. A partir de 1,6 g de composé obtenu à l'étape 9c, on isole, après chromatographie, 700 mg d'hydroxyester de configuration Z.
Rendement : 80 %
Caractéristiques spectrales de RMN du proton (90 MHz, $CDCl_3$, δ ppm) :
7.47-7.07 (m, 4H aromatiques) ; 6.46 (d, 1H, ArC$\underline{H}$=CH-, J = 11.4 Hz) ; 5.63 (dt, 1H, ArCH=C$\underline{H}$$CH_2$-, J = 11.4 ; 7.3 Hz) ; 4.66 (t, 1H, HOC$\underline{H}$$CH_2$-; J = 6.7 Hz) ; 3.64 (s, 3H, $-CO_2C\underline{H}_3$) ; 2.49-2.20 [4H : 2H (t), $-C\underline{H}_2CO_2CH_3$, J = 6.8 Hz et 2H (m), $-CH{=}CHC\underline{H}_2$-] ; 2.13-1.57 (m, 5H, $\underline{H}OCHC\underline{H}_2$- ; $-C\underline{H}_2CH_2CO_2CH_3$) ; 1.42-1.15 [m, 6H, $-CH_2(C\underline{H}_2)_3CH_3$] ; 0.87 (t, 3H, $-CH_2C\underline{H}_3$ ; J = 6.0 Hz).

**Exemple 10: (5*E*)-6-[3-(1-hydroxyhexyl)phényl]hex-5-énoatede méthyle**

Dérivé obtenu selon le mode opératoire décrit pour l'exemple 9 en isolant l'isomère E par chromatographie sur colonne de silice.

**Exemple 11: 1-[(1*Z*)-5-hydroxycarbonylpent-1-ényl]-3-(1-hydroxyhexyl)benzène**

Mode opératoire identique à celui décrit dans l'exemple 3.
Rendement : 86 %
Caractéristiques spectrales de RMN du proton (90 MHz, $CDCl_3$, δ ppm) :
7.44-7.02 (m, 4H aromatiques) ; 6.47 (d, 1H, ArC$\underline{H}$=CH-, J = 11.4 Hz) ; 6.24 (s élargi, 2H, $-CO_2\underline{H}$ ; -O$\underline{H}$) ; 5.63 (dt, 1H, ArCH=C$\underline{H}$-, J = 11.4 ; 7.4 Hz) ; 4.64 (t, 1H, HOC$\underline{H}$$CH_2$-, J = 6.9 Hz) ; 2.59-2.17 (m, 4H, $-C\underline{H}_2CH_2C\underline{H}_2CO_2H$) ; 2.03-1.56 (m, 4H, $HOCHC\underline{H}_2$- ; $-C\underline{H}_2CH_2CO_2H$) ; 1.10-0.68 [m, 6H, $-CH_2(C\underline{H}_2)_3CH_3$] ; 0.86 (t, 3H, $-CH_2C\underline{H}_3$, J = 6.0 Hz).

**Exemple 12: (5*Z*)-6-[3-(1-hydroxyhex-1-yl)phényl]hex-5-énoate de sodium**

Ce sel est obtenu quantitativement à partir de l'acide obtenu à l'exemple 11 et selon un mode opératoire identique à celui décrit pour l'exemple 4. Le sel obtenu est une poudre blanche.
Point de fusion = 140°C

**Exemple 13: 6-[3-(1-hydroxyhexyl)phényl]hexanoate de méthyle**

3,1 g (5,7 mmoles) d'ester du composé de l'exemple 10, dont la fonction alcool est protégée, sont solubilisés dans 100 ml d'acétate d'éthyle puis additionnés de 300 mg de palladium sur charbon. Le ballon est alors équipé d'un appareil d'hydrogénation catalytique et le mélange est agité sous atmosphère d'hydrogène pendant 1 heure, puis filtré. Le solvant est ensuite évaporé sous vide et le produit brut est purifié par chromatographie (éluant : éther/éther de pétrole, 10:90). 3,1 g d'ester sont obtenus, puis la fonction alcool est déprotégée selon la méthode décrite à l'étape 1d. L'hydroxyester attendu se présente sous forme d'huile incolore.
Rendement : 90%
Caractéristiques spectrales de RMN du proton (90 MHz, $CDCl_3$, δ ppm) :
7.32-6.91 (m, 4H aromatiques) ; 4.62 (t, 1H, HOC$\underline{H}$$CH_2$-, J = 6.7 Hz) ; 3.64 (s, 3H, -$CO_2C\underline{H}_3$) ; 2.61 (t, 2H, ArC$\underline{H}_2$-, J = 7.0 Hz) ; 2.29 (t, 2H, -$C\underline{H}_2CO_2CH_3$, J = 7.0 Hz) ; 2.11 (s large, 1H, $\underline{H}$OCH-) ; 1.95-1.06 [m, 14H, $HOCH(C\underline{H}_2)_4CH_3$ ; -$CH_2$-$(C\underline{H}_2)_3$-$CH_2$-$CO_2CH_3$] ; 0.87 (t, 3H, -$CH_2C\underline{H}_3$, J = 6.7 Hz).

**Exemple 14: 1-(5-hydroxycarbonylpentyl)-3-(1-hydroxyhexyl)benzène**

Mode opératoire identique à celui décrit pour l'exemple 3. A partir de 1,3 g d'hydroxyester obtenu dans l'exemple 13 sont isolés, après chromatographie sur colonne de silice, 1,07 g d'hydroxyacide sous forme d'huile incolore.
Rendement : 87 %
Caractéristiques spectrales de RMN du proton (90 MHz, $(CD_3)_2CO$, δ ppm) :
7.55-6.90 (m, 4H aromatiques) ; 4.61 (t, 1H, HOC$\underline{H}$$CH_2$-, J = 6.6 Hz) ; 2.61 (t, 2H, ArC$\underline{H}_2$$CH_2$-, J = 7.0 Hz) ; 2.28 (t, 2H, -$C\underline{H}_2CO_2H$, J = 7.0 Hz) ; 1.91-1.07 [m, 14H, $HOCH(C\underline{H}_2)_4CH_3$ ; -$CH_2(C\underline{H}_2)_3CH_2CO_2H$] ; 0.86 (t, 3H, -$CH_2C\underline{H}_3$, J = 7.0 Hz).

**Exemple 15: 6-[3-(1-hydroxyhexyl)phényl]hexanoate de sodium**

Mode opératoire identique à celui décrit pour l'exemple 4. A partir de 1,7 g d'hydroxyacide de l'exemple 14 sont obtenus, après lavage, 1,1 g de sel attendu sous forme de poudre blanche.
Rendement : 95 %
Point de fusion : 165°C

**Exemple 16: 2-[(1*Z*)-5-méthoxycarbonylpent-1-ényl]-6-(1-hydroxyhexyl)pyridine**

Etape 16a : 2-hydroxyméthyl-6-(tertiobutyldiphénylsilyloxyméthyl) pyridine

2,87 g (72 mmoles) d'hydrure de sodium à 60% dans l'huile minérale sont lavés à l'éther de pétrole (3 fois) puis mis en suspension dans 150 ml de tétrahydrofurane anhydre pour ajouter 10 g (72 mmoles) de 2,6-diméthanolpyridine. Le mélange est alors agité à 50°C pendant 6 heures (un précipité blanc, le monoalcoolate, apparaît en suspension) puis 18,5 ml (72 mmoles) de chlorure de tertiobutyldiphénylsilyle sont ajoutés, goutte à goutte, à température ambiante, et la solution est agitée pendant 2 heures. Après hydrolyse, le milieu réactionnel est extrait à l'éther (3 fois), les phases organiques traitées de manière habituelle, et le résidu huileux est chromatographié sur colonne de silice (éluant : éther/éther de pétrole, 50:50). 16,5 g de produit attendu sont obtenus sous forme d'huile incolore.
Rendement : 61 %

Etape 16b : 2-formyl-6-(tertiobutyldiphénylsilylméthyl)pyridine

4,3 ml (48 mmoles) de chlorure d'oxalyle en solution dans 6 ml de dichlorométhane sont refroidis à -60°C pour ajouter, goutte à goutte, sous azote et agitation magnétique, une solution de 7,6 ml de diméthylsulfoxide dans 24 ml de dichlorométhane. Après 20 mn d'agitation à -60°C, 16 g (44 mmoles) de composé obtenu à l'étape 16a, dans 60 ml de dichlorométhane sont ajoutés goutte à goutte. L'ensemble est agité à -60°C pendant 30 mn pour additionner lentement 32 ml de triéthylamine. Le milieu réactionnel est agité jusqu'à retour à température ambiante, puis hydrolysé et extrait à l'éther (3 fois). Après traitement de la phase organique et purification sur colonne de silice (éluant : éther/éther de pétrole, 20:80), 14,5 g d'aldéhyde attendu sont obtenus.
Rendement : 91 %

Etape 16c : 2-[(1*Z*)-5-méthoxycarbonylpent-1-ényl]-6-(1-hydroxyhexyl)pyridine

Mode opératoire identique à celui décrit à l'étape 1d de l'exemple 1.

Rendement : 76 %
Point de fusion : 38-40°C

Etape 16d : 2-[(1*Z*)-5-méthoxycarbonylpent-1-ényl]-6-formylpyridine

Mode opératoire identique à celui décrit pour l'étape 16b.
Rendement : 98%

Etape 16e : 2-[(1*Z*)-5-méthoxycarbonylpent-1-ényl]-6-(1-hydroxyhexyl)pyridine

A une solution de 3 g (12,8 mmoles) d'aldéhyde obtenu à l'étape 16d dans 24 ml de toluène anhydre, refroidie à -60°C est ajoutée une solution de bromure de n-hexylmagnésium dans le tétrahydrofurane (préparé fraîchement à partir de 486 mg (20mmoles) de magnésium métallique et 2,5 ml (20 mmoles) de bromure de n-hexyle). Après 1 heure d'agitation à basse température, puis retour à température ambiante, le milieu est hydrolisé avec une solution aqueuse saturée de chlorure d'ammonium, puis extrait à l'éther (3 fois), les phases organiques traitées de manière habituelle et le résidu huileux est chromatographié sur colonne de silice (éluant : éther/éther de pétrole, 50:50) pour donner 1,8 g d'hydroxyester attendu sous forme d'huile incolore.
Rendement : 76 %
Caractéristiques spectrales de RMN du proton (90 MHz, $CDCl_3$, δ ppm) :
7.63 (t, 1H aromatique, $H_4$, J = 7.7 Hz) ; 7.12-7.01 (dd,2H, $H_3$ et $H_5$, J = 7.7 ; 2.5 Hz) ; 6.45 (dt, 1H, ArC<u>H</u>=CH-, J = 11.7 ; 1.5 Hz) ; 5.85 (dt, 1H, ArCH=C<u>H</u>$CH_2$-, J = 11.7 ; 7.2 Hz) ; 4.73 (t, 1H, HOC<u>H</u>$CH_2$-, J = 6.7 Hz) ; 4.40 (large, 1H, <u>H</u>OCH-) ; 3.65 (s, 3H, $-CO_2C\underline{H}_3$) ; 2.71 (q, 2H, ArCH=CH$C\underline{H}_2$-, J = 7.2 Hz) ; 2.38 (t, 2H, $-C\underline{H}_2CO_2CH_3$, J = 7.3 Hz) ; 1.83 (p, 4H, $-C\underline{H}_2CH_2CO_2CH_3$, J = 7.0 ; HOCH$C\underline{H}_2$-masqués par le pentuplet) ; 1.56-1.16 [m, 6H, $-CH_2(C\underline{H}_2)_3CH_3$] ; 0.87 (t, 3H, $-CH_2C\underline{H}_3$, J = 6.8 Hz).

**Exemple 17: 2-[(1*Z*)-5-hydroxycarbonylpent-1-ényl]-6-(1-hydroxyhexyl)pyridine**

Mode opératoire identique à celui décrit pour le composé de l'exemple 3.
Rendement : 87 %
Caractéristiques spectrales de RMN du proton (90 MHz, $CDCl_3$, δ ppm) :
7.63 (t, 1H aromatique, $H_4$, J = 7.7 Hz) ; 7.32-6.95 (m, 3H, 2H aromatiques, $-CO_2\underline{H}$) ; 6.46 (d, 1H, ArC<u>H</u>=CH-, J = 11.6 Hz) ; 5.86 (dt, 1H, ArCH=C<u>H</u>$CH_2$-, J = 11.7 ; 7.2 Hz) ; 4.73 (t, 1H, HOC<u>H</u>$CH_2$- ; J = 5.8 Hz) ; 2.70 (pseudo q, 2H,-CH=CH$C\underline{H}_2$-, J= 7.5 Hz) ; 2.40 (t, 2H, $-C\underline{H}_2CO_2H$, J = 7.2 Hz) ; 2.00-1.56 (m, 4H, $-C\underline{H}_2CH_2CO_2H$ ; HOCH$C\underline{H}_2$-) ; 1.50-1.03 [m, 7H, $-CH_2(C\underline{H}_2)_3CH_3$ ; <u>H</u>OCH-] ; 0.86 (t, 3H, $-CH_2C\underline{H}_3$, J = 5.8 Hz).

**Exemple 18: (5*Z*)-6-[6-(1-hydroxyhex-1-yl)pyrid-2-yl]hex-5-énoate de sodium**

Mode opératoire identique à celui décrit à l'exemple 4. Composé obtenu à partir du composé de l'exemple 17.
Rendement quantitatif
Point de fusion : 130°C

**Exemple 19: acide (5*Z*)-N-méthyl-6-[3-(1-hydroxyhexyl)phényl]hex-5-én-1-hydroxamique**

320 mg (0,6 mmoles) d'acide obtenu à l'exemple 11, dont la fonction alcool a été préalablement protégée par le chlorure de diphényltertiobutylsilyle, en solution dans 5 ml de tétrahydrofurane anhydre, sont additionnés, sous azote, de 44 µl (≈ 0,6 mmoles) de N,N-diméthylformamide. Le mélange est refroidi à 0°C pour additionner 105 µl (1,2 mmoles, 2 équivalents) de chlorure d'oxalyle. L'agitation est maintenue à 0°C sous azote pendant 1 heure 30 minutes pour obtenir le chlorure d'acyle correspondant. 75 mg (0,9 mmoles) de chlorhydrate de N-méthylhydroxylamine dans 2 ml d'eau, sont additionnés de 600 µl (1,8 mmoles) d'une solution d'hydroxyde de sodium 3N. Le mélange est agité pendant 30 minutes puis refroidi à 0°C pour additionner, rapidement à l'aide d'une seringue, la solution brute de chlorure d'acyle formé précédemment. Après 2 heures d'agitation, le milieu réactionnel est extrait à l'éther (3 fois), les phases organiques lavées puis séchées (sulfate de magnésium), les solvants sont évaporés sous vide et le produit brut obtenu est engagé dans la réaction de déprotection de la fonction alcool puis purifié par chromatographie sur colonnne de silice (éluant : éther/éther de pétrole, 20:80 ; 50:50, puis éther seul). On obtient 90 mg d'acide hydroxamique du titre sous forme d'huile incolore.
Rendement : 47 %
Caractéristiques spectrales de RMN du proton (90 MHz, $CDCl_3$, δ ppm) :
7.41-6.99 (m, 4H aromatiques) ; 6.43 (d, 1H, ArC<u>H</u>=CH-, J = 11.3 Hz) ; 5.62 (dt, 1H, ArCH=C<u>H</u>$CH_2$-, J = 11.4 ; 7.2 Hz) ;

4.60 (t, 1H, HOC<u>H</u>$CH_2$-, J = 6.2 Hz) ; 3.30-2.98 (m, 3H, =N-C$\underline{H}_3$) ; 2.54-2.08 (m, 4H, -C$\underline{H}_2$$CH_2$C$\underline{H}_2$CON) ; 1.95-1.52 (m, 4H, -C$\underline{H}_2$$CH_2$CON ; HOCHC$\underline{H}_2$-) ; 1.44-1.10 [m, 6H, -$CH_2$(C$\underline{H}_2$)$_3$$CH_3$] ; 0.86 (t, -3H, $CH_2$C$\underline{H}_3$, J = 6.2 Hz).

**<u>Exemple 20</u>: (1*Z*)-6-[3-(1-hydroxyhexyl)phényl]hex-5-én-1-ol**

A une solution de 1,80 g (3,31 mmoles) d'ester obtenu à l'exemple 9 dont la fonction alcool a été préalablement protégée par le chlorure de diphényltertiobutylsilyle, dans 65 ml d'éther anhydre refroidie à -40°C, sont additionnés, goutte à goutte, sous azote et agitation magnétique, 7 ml (7 mmoles) d'une solution d'hydrure de diisobutylaluminium 1M dans le toluène. Après 10 minutes de la fin d'addition, le milieu réactionnel est hydrolysé à l'eau, un précipité blanc gélatineux se forme, il est dissout par addition d'une solution aqueuse saturée de tartrate double de potassium et de sodium. Le mélange est extrait à l'éther (3 fois), les phases organiques séchées (sulfate de magnésium) puis filtrées, les solvants sont ensuite évaporés sous vide et le résidu obtenu est engagé dans la réaction de déprotection de la fonction alcool puis chromatographié sur colonne de silice (éluant : éther/éther de pétrole, 50:50). On obtient l'alcool du titre sous forme d'huile incolore.
<u>Rendement</u> : 70 %
<u>Caractéristiques spectrales de RMN du proton (90 MHz, $CDCl_3$, δ ppm)</u> :
7.39-7.01 (m, 4H aromatiques) ; 6.43 (dt, 1H, ArC<u>H</u>=CH-, J = 11.4 ; 1.6 Hz) ; 5.66 (dt, 1H, ArCH=C<u>H</u>-, J = 11.4 ; 7.3 Hz) ; 4.64 (t, 1H, HOC<u>H</u>$CH_2$-, J = 6.2 Hz) ; 3.60 (t, 2H, -C$\underline{H}_2$OH, J = 6.8 Hz) ; 2.50 - 2.12 (q élargi, 2H, -CH=CHC$\underline{H}_2$-) ; 1.90 - 1.08 [m, 14H, -C$\underline{H}_2$C$\underline{H}_2$$CH_2$O<u>H</u> ; <u>H</u>OCH (C$\underline{H}_2$)$_4$$CH_3$] ; 0.87 (t, 3H, -$CH_2$C$\underline{H}_3$, J = 6.0 Hz).

**<u>Exemple 21</u>: 1-[6-(1*Z*)-bromohex-1-èn-1-yl]-3-(1-hydroxyhexyl)benzène**

<u>Etape 21 a</u> : 1-[6-(1*Z*)-bromohex-1-èn-1-yl]-3-[1-(diphényltertiobutylsilyloxy)hexyl]benzène

A une solution de 1,0 g (2,0 mmoles) d'alcool obtenu à l'exemple 20, isolé avant la libération de l'alcool secondaire, et de 840 mg (2,5 mmoles) de tétrabromométhane dans 16 ml de dichlorométhane refroidie à 0°C est ajoutée goutte à goutte, sous azote et agitation magnétique, une solution de 714 mg (2,7 mmoles) de triphénylphosphine dans 8 ml de dichlorométhane. Après 15 minutes d'agitation à 0°C, de l'éther de pétrole est ajouté à froid et le milieu réactionnel est filtré sur silice, les solvants sont évaporés sous vide et l'huile obtenue est purifiée par flash chromatographie (éluant : éther/éther de pétrole, 5:95). On obtient 1,15 g du dérivé bromé du titre avec un rendement quantitatif sous forme d'huile incolore.
<u>Caractéristiques spectrales de RMN du proton (90 MHz, $CDCl_3$, δ ppm)</u> :
7.78-7.58 (m, 2H aromatiques) ; 7.54-6.97 (m, 12H aromatiques) ; 6.40 (dt, 1H ArC<u>H</u>=CH-, J = 11.5 ; 1.6 Hz) ; 5.58 (dt, 1H, -CH=C<u>H</u>$CH_2$-, J = 11.6 ; 7.2 Hz) ; 4.64 (t, 1H, SiOC<u>H</u>$CH_2$-, J = 6.0 Hz) ; 3.33 (t, 2H, -C$\underline{H}_2$Br, J = 6.5 Hz) ; 2.35 (q élargi, 2H, CH = CHC$\underline{H}_2$-, J = 7.2 Hz) ; 2.00-1.45 (m, 6H, SiOCHC$\underline{H}_2$- ; -C$\underline{H}_2$C$\underline{H}_2$$CH_2$Br) ; 1.19-0.94 [m, 15 H, -$CH_2$ (C$\underline{H}_2$)$_3$$CH_3$, -C(C$\underline{H}_3$)$_3$] ; 0.76 (t, 3H, -$CH_2$C$\underline{H}_3$, J = 6.0 Hz).

<u>Etape 21 b</u> : 1-[6-(1*Z*)-bromohex-1-èn-1-yl]-3-(1-hydroxyhexyl)benzène

Le bromure obtenu à l'étape précédente est traité par du fluorure de tétra-n-butylammonium afin de libérer la fonction alcool.

**<u>Exemple 22</u>: 1-[(1*Z*)-6-diéthoxyphosphinylhex-1-ényl]-3-(1-hydroxyhexyl)benzène**

420 mg (0,72 mmoles) du dérivé bromé obtenu à l'étape 21a sont ajoutés à 7 ml de phosphite de triéthyle. Le mélange est chauffé à 130-140° sous azote et agitation magnétique pendant 7 heures, puis l'excès de phosphite de triéthyle est distillé sous vide. Le produit brut est engagé dans la réaction de libération de la fonction alcool secondaire puis purifié par chromatographie sur colonne de silice (éluant : éther). On obtient 235 mg de phosphonate attendu sous forme d'huile incolore.
<u>Rendement</u> : 82 %
<u>Caractéristiques spectrales de RMN du proton (90 MHz, $CDCl_3$, δ ppm)</u> :
7.40-7.06 (m, 4H aromatiques) ; 6.44 (dt, 1H ArC<u>H</u>=CH-, J = 11.4 ; 1.3 Hz) ; 5.62 (dt, 1H, ArCH= C<u>H</u> -, J = 11.4 ; 7.3 Hz) ; 4.63 (t, 1H, HOC<u>H</u>$CH_2$- ; J = 6.2 Hz) ; 4.04 [p, 4H, -PO(OC$\underline{H}_2$$CH_3$)$_2$, J = 7.2 Hz) ; 2.70 (large, 1H, <u>H</u>OCH-) ; 2.33 (q élargi, 2H, -CH = CHC$\underline{H}_2$-) ; 1.74-1.53 [m, 8H, -$CH_2$(C$\underline{H}_2$)$_3$PO (OEt)$_2$ ; HOCHC$\underline{H}_2$-] ; 1.28 [t, 12H, -PO (O$CH_2$C$\underline{H}_3$)$_2$, J = 7.2 Hz ; -$CH_2$(C$\underline{H}_2$)$_3$$CH_3$ masqués par le triplet) ; 0.87 (t, 3H, -$CH_2$C$\underline{H}_3$, J = 6.3 Hz).

**Exemple 23: 1-[(1*Z*)-6-aminohex-1-ényl]-3-(1-hydroxyhexyl)benzène**

Etape 23 a : 1-[(1*Z*)-6-azidohex-1-ényl]-3-[1-(diphényltertiobutylsilyloxy)hexyl]benzène

A une solution de 590 mg (1.02 mmoles) du dérivé bromé obtenu à l'étape 21a dans 10 ml de diméthylsulfoxyde, sont ajoutés 107 mg (1.6 mmoles) d'azoture de sodium ($NaN_3$). Le mélange est agité à température ambiante, sous azote, pendant 3 heures, puis de l'eau est ajoutée et le milieu réactionnel est extrait à l'éther (3 fois), les phases organiques lavées à l'eau puis séchées (sulfate de magnésium) et filtrées, les solvants sont ensuite évaporés sous vide et le produit brut est purifié par flash chromatographie (éluant : éther/éther de pétrole, 5:95). On obtient 530 mg d'azide attendu sous forme d'huile incolore.
Rendement : 96 %
Caractéristiques spectrales de RMN du proton (90 MHz, $CDCl_3$, δ ppm) :
7.75-7.62 (m, 2H aromatiques) ; 7.55-6.95 (m, 12H aromatiques) ; 6.40 (d, 1H, ArC<u>H</u>=CH-, J = 11.6 Hz) ; 5.58 (dt, 1H, ArCH=C<u>H</u>$CH_2$-, J = 11.3 ; 7.0 Hz) ; 4.63 (t, 1H, SiOC<u>H</u>$CH_2$-, J = 5.9 Hz) ; 3.20 (t, 2H, -$C\underline{H}_2N_3$, J = 6.3 Hz) ; 2.31 (q élargi, 2H, -CH=CH$C\underline{H}_2$-) ; 1.76 - 1.38 [m, 6H, -$CH_2(C\underline{H}_2)_2CH_2N_3$ ; SiOCH$C\underline{H}_2$-] ; 1.19 - 0.90 [m, 15H, -$CH_2(C\underline{H}_2)_3CH_3$, -$C(C\underline{H}_3)_3$] ; 0.75 (t, 3H, -$CH_2C\underline{H}_3$, J = 6.0 Hz).

Etape 23 b : 1-[(1*Z*)-6-azidohex-1-ényl]-3-(1-hydroxyhexyl)benzène

500 mg d'azide obtenus à l'étape précédente sont engagés dans la réaction de déprotection de la fonction alcool. Après chromatographie, 250 mg d'azide attendu sont obtenus sous forme d'huile incolore.
Rendement : 89 %

Etape 23 c : 1-[(1*Z*)-6-aminohex-1-ényl]-3-(1-hydroxyhexyl)benzène

A une solution de 160 mg (0,53 mmoles) d'azide obtenu à l'étape précédente dans 0.5 ml de tétrahydrofurane, sont ajoutés 140 mg (0,53 mmoles) de triphénylphosphine puis 14 µl d'eau et une pierre ponce (un dégagement d'azote est observé). Le mélange est abandonné à température ambiante pendant 8 heures, puis le tétrahydrofurane est évaporé sous vide et le résidu est chromatographié sur colonne de silice en utilisant le méthanol comme éluant. On obtient 100 mg d'amine attendue sous forme d'huile incolore.
Rendement : 68 %
Caractéristiques spectrales de RMN du proton (90 MHz, $CDCl_3$, δ ppm) :
7.37-7.05 (m, 4H aromatiques) ; 6.41 (d, 1H, ArC<u>H</u>=CH-, J = 11.6 Hz) ; 5.62 (dt, 1H, ArCH=C<u>H</u>$CH_2$-, J = 11.6 ; Hz) 4.49 (t, 1H, HOC<u>H</u>$CH_2$-, J = 6.3 Hz) ; 2.72 - 2.45 (m, 2H, -$C\underline{H}_2NH_2$) ; 2.45-2.08 (m, 5H dont 3 échangeables au $D_2O$, -CH=CH$C\underline{H}_2$- ; <u>H</u>OCH- ; $\underline{H}_2NCH_2$-) ; 1.83-1.55 (m, 2H, HOCH$C\underline{H}_2$-) ; 1.55-1.10 [m, 10H, -$CH_2(C\underline{H}_2)_3CH_3$ ; -$CH_2(C\underline{H}_2)_2CH_2NH_2$] ; 0.86 (t, 3H, -$CH_2C\underline{H}_3$, J = 6.1 Hz).

**Exemple 24: 1-[(1*Z*)-6-(p-toluènesulfonylamino)hex-1-ényl]-3-(1-hydroxyhexyl)benzène**

200 mg (0,39 mmoles) d'amine obtenue dans l'exemple 23 dont la fonction alcool a été au préalable protégée par le chlorure de diphényltertiobutylsilyle, dans 1 ml de dichlorométhane refroidis à 0°C, sont additionnés, sous azote et agitation magnétique, de 120 µl (0,84 mmoles) de triéthylamine anhydre, puis de 120 mg (0,62 mmoles) de chlorure de p-toluènesulfonyle. Après 15 minutes d'agitation à 0°C, de l'eau est ajoutée, le milieu réactionnel est alors extrait à l'éther (3 fois), les phases organiques lavées avec une solution saturée de chlorure de sodium puis séchées (sulfate de magnésium) et filtrées, les solvants sont ensuite évaporés sous vide et l'huile obtenue est engagée dans la réaction de déprotection de la fonction alcool puis purifiée par chromatographie sur colonne de silice (éluant : éther/éther de pétrole, 50:50). 120 mg de sulfamide attendue sont obtenues sous forme d'huile incolore.
Rendement : 72 %
Caractéristiques spectrales de RMN du proton (90 MHz, $CDCl_3$, δ ppm) :
7.70 (dt, 2H aromatiques en α du -$SO_2NH$-, J = 8.3 ; 1.3 Hz) ; 7.40-7.05 (m, 6H aromatiques) ; 6.39 (d, 1H, ArC<u>H</u>=CH-, J = 11.8 Hz) ; 5.54 (dt, 1H, ArCH=C<u>H</u>-, J = 11.8 ; 7.4 Hz) ; 5.10 (t, 1H échangeable au $D_2O$, -N<u>H</u>$SO_2$- ; J = 6.2 Hz) ; 4.63 (t, 1H, HOC<u>H</u>$CH_2$-, J = 6.3 Hz) ; 2.83 (q élargi, 2H, -$C\underline{H}_2NH$-) ; 2.50 (s, 1H échangeable au $D_2O$, <u>H</u>OCH-) ; 2.39 (s, 3H, P-$C\underline{H}_3C_6H_4$-) ; 2.24 (q élargi, 2H, -CH=CH$C\underline{H}_2$-) ; 1.85-1.58 (m, 2H, HOCH$C\underline{H}_2$-) ; 1.50-1.18 [m, 10H, -$CH_2(C\underline{H}_2)_3CH_3$ ; -$CH_2(C\underline{H}_2)_2CH_2NH$-] ; 0.85 (t, 3H, -$CH_2C\underline{H}_3$, J = 6.0 Hz).

**Exemple 25: (5*Z*)-6-[3-(1-hydroxyhexyl)phényl]hex-5-én-1-aloxime**

L'alcool obtenu dans l'exemple 20, dont la fonction alcool secondaire est protégée au préalable par le chlorure de

diphényltertiobutylsilyle, est oxydé par du chlorochromate de pyridinium en présence d'acétate de sodium dans le chlorure de méthylène. L'aldéhyde ainsi obtenu est traité par du chlorhydrate d'hydroxylamine pour donner, après déprotection de la fonction alcool, le produit attendu sous forme racémique.
Caractéristiques spectrales de RMN du proton (90 MHz, $CDCl_3$,δ) :
7.55-7.07 (m, 5H, arom+CH=N), 6.45 (d, 1H, J = 11.4) ; 5.63 (dt, 1H, J = 11.4, J = 7.3) ; 4.65 (t, 1H, J = 6.6) ; 2.76-2.05 (m, 4H) ; 2.05-1.00 (m, 11H) ; 1.00-0.71 (m, 3H)

**Exemple 26: (5*Z*)-6-{3-[(3*Z*)-(1-hydroxynon-3-én-1-yl)]phényl}hex-5-énoate de sodium**

Composé préparé à partir de l'acide décrit à l'exemple 7 selon la méthode décrite pour l'obtention du composé de l'exemple 4.
Caractéristiques spectrales de RMN du proton (90 MHz, $D_2O$, δ ppm) :
7.50-7.28 (m, 4H aromatiques) ; 6.59 (d, 1H, ArCH=CH-, J = 11.2 Hz) ; 5.92 (dt, 1H ; ArCH=CHCH$_2$- ; J = 7.3 ; 11.2 Hz) ; 5.52 (massif, 2H ; -CH$_2$CH=CHCH$_2$-) ; 4.86 (t, 1H ; HOCHCH$_2$- ; J = 6.7 Hz) ; 2.86-2.29 (m, 6H ; -CH$_2$CO$_2^-$Na$_2^+$ ; -CH$_2$CH=CHCH$_2$-) ; 2.14-1.78 (massif, 4H, ArCH=CHCH$_2$CH$_2$-) ; 1.22 (massif ; 6H, -CH$_2$(CH$_2$)$_3$CH$_3$) ; 0.88 (t, 3H, -CH$_2$CH$_3$, J = 5.8 Hz)

**Exemple 27: (5*Z*)-6-[3-(1-hydroxy-1-n-butylhexyl)phényl]hex-5-énoate de méthyle**

Etape 27a : (5*Z*)-6-[3-(1-oxohexyl)phényl]hex-5-énoate de méthyle

220 µl (2,5 mmoles) de phosgène en solution dans 6 ml de dichlorométhane sont refroidis à -60° pour ajouter, goutte à goutte, sous azote et agitation magnétique, une solution de 390 µl de diméthylsulfoxyde dans 1,2 ml de dichlorométhane. Après 20 mn d'agitation à -60°, sont ajoutés (goutte à goutte) 680 mg (2.23 mmoles) d'hydroxyester obtenu à l'exemple 9 en solution dans 3 ml de dichlorométhane. La solution est agitée (à -60°) pendant 30 mn pour additionner lentement 1,6 ml de triéthylamine et on laisse remonter la température, sous agitation, pendant 30 mn. Le milieu réactionnel est alors additionné d'eau puis extrait à l'éther (3 fois), les phases organiques lavées à l'eau puis séchées (sulfate de magnésium) et filtrées, les solvants sont ensuite évaporés sous vide et le produit brut est purifié par chromatographie sur colonne de silice (éluant : éther/éther de pétrole, 20:80). On obtient 630 mg de cétoester attendu sous forme d'huile incolore.
Rendement : 93 %

Etape 27b : (5*Z*)-6-[3-(1-hydroxy-1-n-butylhexyl)phényl]hex-5-énoate de méthyle

Le composé obtenu à l'étape précédente est placé dans du tétrahydrofurane anhydre refroidi à -60°C puis est additionné de butyllithium. Le produit titre est obtenu après purification par chromatographie sur colonne de silice.

**Exemple 28: (5*Z*)-6-[(1*R*)-3-(1-hydroxyhexyl)phényl]hex-5-énoate de méthyle**

470 mg (1,85 mmoles) de S-(-)-diphénylprolinol, en solution dans 9 ml de tétrahydrofurane anhydre, sont additionnés de 5,5 ml (5,5 mmoles) d'une solution 1M d'hydrure de bore dans le tétrahydrofurane. L'ensemble est chauffé à reflux pendant deux heures. Après refroidissement, le solvant est éliminé sous vide et le résidu séché, également sous vide. Celui-ci est repris par 5 ml de tétrahydrofurane anhydre et refroidi à 0°C. 2 ml (2,0 mmoles) d'hydrure de bore 1M dans le tétrahydrofurane sont ajoutés, puis après quelques minutes d'agitation, l'ensemble est additionné de 560 mg (1,85 mmoles) de composé obtenu à l'Exemple 27, Etape 27a. Le milieu réactionnel est ensuite neutralisé par quelques millilitres de méthanol, les solvants évaporés sous vide et le résidu repris à l'éther. Le traitement habituel de la phase organique fournit, après purification sur colonne de silice, (éluant : éther/éther de pétrole, 50:50), 370 mg de composé attendu.
Rendement : 66 %
Pouvoir rotatoire : $[\alpha]_D^{20}$ = +9,4 (concentration : $3,7.10^{-3}$ M dans le méthanol)

**Exemple 29: acide (5*Z*)-6-[(1*R*)-3-(1-hydroxyhexyl)phényl]hex-5-énoïque**

Composé obtenu selon un mode opératoire analogue à celui décrit dans l'exemple 3, à partir de l'ester obtenu à l'exemple 28.

**Exemple 30:(5*Z*)-6-[(1*R*)-3-(1-hydroxyhexyl)phényl]hex-5-énoate de sodium**

Composé obtenu selon un mode opératoire analogue à celui décrit dans l'exemple 4, à partir de l'acide obtenu à l'exemple 29.
Pouvoir rotatoire : $[\alpha]_D^{20}$ = +12,8 (concentration : $4{,}4.10^{-3}$ M dans le méthanol)
Microanalyse élémentaire : $C_{18}H_{25}O_3Na$ (masse molaire : 312,29)

| | C | H |
|---|---|---|
| % Calculé | 69,21 | 8,07 |
| % Trouvé | 69,49 | 8,09 |

**Exemple 31: (5*Z*)-6-[(1*S*)-3-(1-hydroxyhexyl)phényl]hex-5-énoate de méthyle**

Composé obtenu selon un mode opératoire analogue à celui décrit dans l'exemple 28, en remplaçant le *S*-(-)-diphénylprolinol par le *R*-(+)-diphénylprolinol.
Rendement : 63%
Pouvoir rotatoire : $[\alpha]_D^{20}$ = -9,3 (concentration : $4{,}3.10^{-3}$ M dans le méthanol)

**Exemple 32: acide (5*Z*)-6-[(1*S*)-3-(1-hydroxyhexyl)phényl]hex-5-énoïque**

Composé obtenu selon un mode opératoire analogue à celui décrit dans l'exemple 3, à partir de l'ester obtenu à l'exemple 31.

**Exemple 33: (5*Z*)-6-[(1*S*)-3-(1-hydroxyhexyl)phényl]hex-5-énoate de sodium**

Composé obtenu selon un mode opératoire analogue à celui décrit dans l'exemple 4, à partir de l'acide obtenu à l'exemple 32.
Pouvoir rotatoire : $[\alpha]_D^{20}$ = -12,9 (concentration : $3{,}2.10^{-3}$ M dans le méthanol)
Microanalyse élémentaire : $C_{18}H_{25}O_3Na$ (masse molaire : 312,29)

| | C | H |
|---|---|---|
| % Calculé | 69,21 | 8,07 |
| % Trouvé | 68,97 | 8,02 |

**Exemple 34: (5*Z*)-6-[3-(6-hydroxy-6-undécanyl)phényl]hex-5-énoate de méthyle**

Composé obtenu selon le mode opératoire décrit pour l'exemple 9, à partir du composé obtenu à l'exemple 27, Etape 27a.
Rendement : 64%
Caractéristiques spectrales de RMN du proton (90 MHz, $CDCl_3$, δ (ppm)) :
7.41-7.01 (m, 4H aromatiques) ; 6.48 (d, 1H, ArC$\underline{H}$=CH-, J = 11.4Hz) ; 5.62 (dt, 1H, ArCH=C$\underline{H}$-, J = 11.9 ; 7.3 Hz) ; 3.63 (s, 3H, $-CO_2C\underline{H}_3$) ; 2.45-2.20 (4H : t, 2H, $-C\underline{H}_2CO_2CH_3$, J = 7.0 Hz et 2H : $-CH{=}CHC\underline{H}_2-$ masqués par le triplet) ; 1.95-1.55 [m, 7H, $-C\underline{H}_2CH_2CO_2CH_3$ ; $(-C\underline{H}_2C_4H_9)_2$ et $\underline{H}$OC-] ; 1.40-0.99 [m, 12H, HOC- $(CH_2C\underline{H}_2C\underline{H}_2\,C\underline{H}_2CH_3)_2$] ; 0.82 [pseudo triplet, 6H, $(-CH_2C\underline{H}_3)_2$].

**Exemple 35: (5*Z*)-6-{3-[hydroxy-(1*Z*)-(4-pent-1-énylphényl)méthyl]phényl}hex-5-énoate de méthyle**

Etape 35a : 1-(4-bromophényl)pent-1-ène

A une suspension de 34 g (85,1 mmoles) de bromure de n-butyltriphénylphosphonium, dans 150 ml de tétrahydrofurane, refroidie à -10°C, sont ajoutés 52 ml (83,2 mmoles) d'une solution 1,6 M de n-butyllithium dans l'hexane. Après 20 minutes de réaction, l'ensemble est refroidi à -80°C et 50 ml d'hexaméthylphosphotriamide sont ajoutés, ainsi que 10 g (54 mmoles) de 4-bromobenzaldéhyde en solution dans 40 ml de tétrahydrofurane. Après retour à température ambiante et hydrolyse par une solution saturée de chlorure d'ammonium, le milieu réactionnel est extrait à l'éther. Le traitement habituel de la phase organique fournit, après purification par chromatographie sur colonne de silice (éluant : éther de pétrole), 10 g du composé attendu.

Rendement : 82%

Etape 35b : (3-diméthoxyméthylphényl)-(4-pent-1-énylphényl)méthanol

A une solution de 4,6 g (20,4 mmoles) du composé obtenu à l'étape précédente, dans 80 ml de tétrahydrofurane à -80°C, sont ajoutés 17 ml (23,8 mmoles) d'une solution 1,4 M de *sec*-butyllithium dans le cyclohexane. Après 10 minutes d'agitation, 3,7 g (20,5 mmoles) de composé obtenu à la préparation B, dans 15 ml de tétrahydrofurane anhydre sont ajoutés. Après retour à température ambiante et hydrolyse par une solution saturée de chlorure d'ammonium, le milieu réactionnel est extrait à l'éther. Le traitement habituel de la phase organique fournit, après purification par chromatographie sur colonne de silice (éluant : éther/éther de pétrole, 40:60 puis 50:50 avec 1% de triéthylamine), 5,3 g du composé attendu.
Rendement : 79%

Etape 35c :

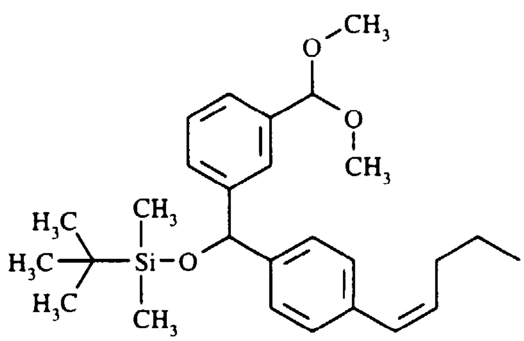

A une solution de 2,1 g (6,4 mmoles) de composé obtenu dans l'Etape 35b dans 25 ml de diméthylformamide, sont ajoutés 1,1 g (16,1 mmoles) d'imidazole puis 1,2 g (7,9 mmoles) de chlorure de diméthyltertiobutylsilyle. Le mélange est abandonnée à température ambiante pendant 15 heures puis hydrolysé à l'eau. Le milieu réactionnel est extrait à l'éther. Le traitement habituel de la phase organique fournit, après purification sur colonne de silice (éluant : éther/éther de pétrole, 10:90 avec 1% de triéthylamine), 2,6 g d'éther silylé attendu.
Rendement : 92%

Etape 35d :

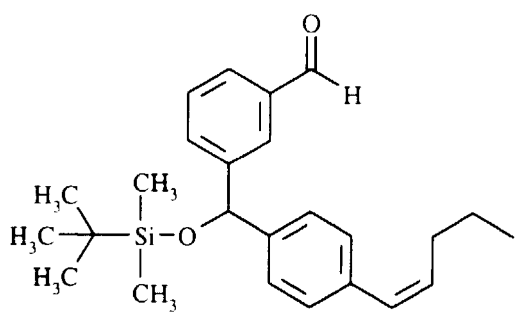

Composé obtenu, avec un rendement quantitatif, selon le mode opératoire décrit dans l'Exemple 1, Etape 1c, à partir de l'éther silylé obtenu dans l'étape précédente.

Etape 35e : (5*Z*)-6-{3-[hydroxy-(1*Z*)-(4-pent-1-énylphényl)méthyl]phényl}hex-5-énoate de méthyle

Composé obtenu selon le mode opératoire décrit dans l'exemple 1, Etape 1d, à partir du composé obtenu dans l'étape précédente.
Rendement : 81 %
Caractéristiques spectrales de RMN du proton (300 MHz, $CDCl_3$, δ (ppm)) :

7.38-7.12 (m, 8H aromatiques) ; 6.44 (d, 1H, ArCH=CH-, J = 11.6 Hz) ; 6.38 (dt, 1H, ArCH=CH-, J = 11.7 ; 1.8 Hz) ; 5.84 (s, 1H, HOCH-) ; 5.66 (dt, 1H, -CH=CHCH$_2$-, J = 11.7 ; 7.3 Hz) ; 5.63 (dt, 1H, -CH=CHCH$_2$-, J = 11.5 ; 7.5 Hz) ; 3.62 (s, 3H, -CO$_2$CH$_3$) ; 2.5 (large, 1H, HOCH-) ; 2.37-2.24 (m, 6H, -CH$_2$CH$_2$CH$_2$CO$_2$CH$_3$ ; Ar'CH=CHCH$_2$-) ; 1.75 (tt, 2H, -CH$_2$CH$_2$CO$_2$CH$_3$, J = 7.5 Hz) ; 1.46 (tq, 2H, -CH$_2$CH$_3$, J = 7.4 Hz) ; 0.92 (t, 3H, -CH$_2$CH$_3$ J = 7.3 Hz).
Microanalyse élémentaire : $C_{25}H_{30}O_3$ (masse molaire : 378,52)

| | C | H |
|---|---|---|
| % Calculé | 79.33 | 7.99 |
| % Trouvé | 79.44 | 8.16 |

**Exemple 36: (5*Z*)-6-{3-[hydroxy-(4-pentylphényl)méthyl]phényl}hex-5-énoate de méthyle**

2,8 g (6,3 mmoles) de composé obtenu à l'Exemple 35, Etape 35c dans 120 ml d'acétate d'éthyle sont additionné de 250 mg de palladium sur charbon. L'ensemble est maintenu sous agitation et sous atmosphère d'hydrogène pendant 30 minutes. Le produit d'hydrogénation ainsi obtenu est soumis, après purification par chromatographie, aux traitements décrits dans les Etapes 35d et 35e pour fournir le produit attendu.
Rendement global : 71%
Caractéristiques spectrales de RMN du proton (90 MHz, $CDCl_3$, δ (ppm)) :
7.38-7.02 (m, 8H aromatiques) ; 6.43 (d, 1H, CH=CHCH$_2$-, J = 11.3 Hz) ; 5.77 (d, 1H, HOCH-, J = 2.9 Hz) ; 5.60 (dt, 1H, -CH=CHCH$_2$, J = 11.6 ; 7.3 Hz) ; 3.60 (s, 3H, -CO$_2$CH$_3$) ; 2.73-2.14 (m, 7H, -CH$_2$CH$_2$CH$_2$CO$_2$CH$_3$ ; ArCH$_2$- ; HOCH-) ; 1.94-1.18 [m, 8H, -CH$_2$(CH$_2$)$_3$CH$_3$ ; -CH$_2$CH$_2$CO$_2$CH$_3$] ; 0.88 (t, 3H, -CH$_2$CH$_3$, J = 5.8 Hz).
Microanalyse élémentaire : $C_{25}H_{32}O_3$ (masse molaire : 380,53)

| | C | H |
|---|---|---|
| % Calculé | 78.91 | 8.48 |
| % Trouvé | 78.90 | 8.40 |

Les acides décrits dans les Exemples 37, 38 et 39 suivants sont obtenus selon le mode opératoire décrit dans l'Exemple 29, à partir des Exemples 34, 35 et 36 respectivement.

**Exemple 37: acide (5*Z*)-6-[3-(6-hydroxy-6-undécanyl)phényl]hex-5-énoïque**

**Exemple 38: acide (5*Z*)-6-{3-[hydroxy-(1*Z*)-(4-pent-1-énylphényl)méthyl]phényl} hex-5-énoïque**

**Exemple 39: acide (5*Z*)-6-{3-[hydroxy-(4-pentylphényl)méthyl]phényl}hex-5-énoïque**

Les sels de sodium décrits dans les Exemples 40, 41 et 42 suivants sont obtenus selon le mode opératoire décrit dans l'Exemple 30, à partir des Exemples 37, 38 et 39 respectivement.

**Exemple 40: (5*Z*)-6-[3-(6-hydroxy-6-undécanyl)phényl]hex-5-énoate de sodium**

Caractéristiques spectrales de RMN du proton (90 MHz, $CD_3OD$, δ (ppm)) :
7.40-7.03 (m, 4H aromatiques) ; 6.44 (d, 1H, ArCH=CH-, J = 12.2 Hz) ; 5.72 (dt, 1H, ArCH=CH-, J = 12.2 ; 7.1 Hz) ; 2.42-2.14 (m, 4H, -CH$_2$CH$_2$CH$_2$CO$_2^-$Na$^+$) ; 1.94-1.40 [m, 6H, -CH$_2$CH$_2$CO$_2^-$Na$^+$, (-CH$_2$C$_4$H$_9$)$_2$] ; 1.39-0.97 (m, 12H, HOC(CH$_2$CH$_2$CH$_2$CH$_2$ CH$_3$)$_2$] ; 0.83 [t, 6H, (-CH$_2$CH$_3$)$_2$ ; J = 5.0 Hz]
Microanalyse élémentaire : $C_{23}H_{35}O_3Na$ (masse molaire : 382,52)

| | C | H |
|---|---|---|
| % Calculé | 72.22 | 9.22 |
| % Trouvé | 72.14 | 9.17 |

**Exemple 41: (5*Z*)-6-{3-[hydroxy-(1*Z*)-(4-pent-1-énylphényl)méthyl]phényl}hex-5-énoate de sodium**

Caractéristiques spectrales de RMN du proton (300 MHz, $CD_3OD$, δ (ppm)) :
7.35-7.13 (m, 8H aromatiques) ; 6.40 (d, 1H, ArCH=CH-, J = 11.6 Hz) ; 6.39 (d, 1H, ArCH=CH-, J = 11.6Hz) ; 5.76 (s,

1H, HOC$\underline{H}$-) ; 5.67 (dt, 1H, -CH=C$\underline{H}$CH$_2$-, J = 11.3 ; 7.4 Hz); 5.63 (dt, 1H, -CH=C$\underline{H}$CH$_2$-, J = 11.5 ; 7.3 Hz) ; 2.30 (dt, 2H, -CH=CHC$\underline{H}_2$-, J = 7.2 Hz) 2.29 (dt, 2H, -CH=CHC$\underline{H}_2$-, J = 7.2Hz) ; 2.18 (t, 2H, -C$\underline{H}_2$CO$_2^-$Na$^+$, J = 7.6 Hz) ; 1.72 (tt, 2H, -C$\underline{H}_2$CH$_2$CO$_2^-$Na$^+$, J = 7.6 Hz) ; 1.46 (tq, 2H, -C$\underline{H}_2$CH$_3$, J = 7.4 Hz) ; 0.92 (t, 3H, -CH$_2$C$\underline{H}_3$, J = 7.3 Hz).
<u>Microanalyse élémentaire</u> : $C_{24}H_{27}O_3Na$ (masse molaire : 386,47)

| | C | H |
|---|---|---|
| **% Calculé** | 74.59 | 7.04 |
| **% Trouvé** | 74.21 | 7.22 |

**<u>Exemple 42</u>: (5*Z*)-6-{3-[hydroxy-(4-pentylphényl)méthyl]phényl}hex-5-énoate de sodium**

<u>Caractéristiques spectrales de RMN du proton (90 MHz, CD$_3$OD, δ (ppm))</u> :
7.64-6.96 (m, 8H aromatiques) ; 6.38 (d, 1H, -CH=CHCH$_2$, J = 11.7 Hz) ; 5.88-5.46 (m, 2H, HOC$\underline{H}$- ; -CH=C$\underline{H}$CH$_2$-) ; 2.67-2.03 (m, 6H, ArC$\underline{H}_2$- ; -C$\underline{H}_2$CH$_2$C$\underline{H}_2$CO$_2^-$Na+) ; 1.94-1.14 [m, 8H, -CH$_2$(C$\underline{H}_2$)$_3$CH$_3$ ; -C$\underline{H}_2$CH$_2$CO$_2^-$Na$^+$] ; 0.87 (t, 3H, -CH$_2$C$\underline{H}_3$).
<u>Microanalyse élémentaire</u> : $C_{24}H_{29}O_3Na$ (masse molaire : 388,49)

| | C | H |
|---|---|---|
| **% Calculé** | 74.20 | 7.52 |
| **% Trouvé** | 74.33 | 7.78 |

**ETUDE PHARMACOLOGIQUE**

**<u>Exemple A</u> : Etude de l'agrégation plaquettaire**

L'étude est réalisée sur des plaquettes de lapin. Après anesthésie de l'animal par le sodium pentobarbital (30 mg/kg i.v.), le sang artériel est prélevé sur citrate de sodium (0.109M) (11 vol. de citrate pour 9 vol. de sang). Le plasma riche en plaquettes (PRP) est obtenu après centrifugation (20°C) à 250 g pendant 20 minutes. Le PRP est de nouveau centrifugé (1000 g) pendant 15 minutes et le culot plaquettaire à nouveau suspendu dans une solution saline tamponnée avec du tris (hydroxyméthyl)aminométhane, contenant de la gélatine (0.2 %). Après une nouvelle centrifugation (1000 g ; 15 min), le culot est à nouveau mis en suspension dans une solution physiologique et conservé à température ambiante.

L'effet anti-agrégant des produits est examiné sur des plaquettes activées avec du collagène (2 µl/ml). L'agrégation plaquettaire est réalisée à 37°C dans des tubes en verre siliconé, à l'aide d'un agrégomètre (Coultronics). Les plaquettes sont agitées à 1000 rpm. Pour tester l'effet de l'antagoniste, le PRP est incubé avec la substance à tester pendant 3 minutes à 37°C. Ensuite, le collagène (2µl/ml) est ajouté.

L'effet des antagonistes est mesuré et l'$IC_{50}$ est déterminée comme la concentration de l'antagoniste nécessaire pour produire 50 % d'inhibition des réponses agrégantes au collagène.

Les résultats sont reproduits dans le tableau suivant :

| **COMPOSE** | **$IC_{50}$ (µM)** |
|---|---|
| Exemple 4 | 2.2 |
| Exemple 12 | 20 |
| Exemple 15 | 21 |
| Exemple 18 | 30 |
| Exemple 26 | 1.8 |

**<u>Exemple B</u> : Composition pharmaceutique : comprimés**

Formule de préparation pour 1000 comprimés dosés à 50 mg.

| | |
|---|---|
| Composé de l'exemple 26 | 50 g |
| Amidon de blé | 15 g |

(suite)

| | |
|---|---|
| Amidon de maïs | 15 g |
| Lactose | 65 g |
| Stéarate de magnésium | 2 g |
| Silice | 1 g |
| Hydroxypropylcellulose | 2 g |

## Revendications

1. Composés de formule (I) :

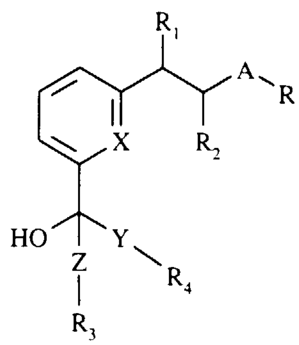

(I)

dans laquelle :

**A** représente un radical bivalent hydrocarboné, comprenant de 2 à 10 atomes de carbone, en chaîne linéaire ou ramifiée et comportant éventuellement une ou plusieurs insaturations sous forme de doubles et de triples liaisons,

**R** est choisi parmi un atome d'halogène, choisi parmi fluor, chlore, brome, iode, un radical -OR', -COOR', -COR', P(O)(OR')$_2$, -CH=N-OR', -CONHR', -CH=NR'', -CH=NAr, -NHSO$_2$Ar, -CON(OH)R', -NHR', -NHCOR', -CH=N-NHAr,

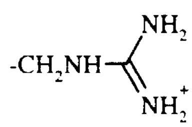

et un radical éventuellement substitué, imidazolyle, pyrazolyle ou tétrazolyle,

**R'** est choisi parmi un atome d'hydrogène et un radical alkyle linéaire ou ramifié contenant de 1 à 6 atomes de carbone,

**R''** représente un radical alkyle linéaire ou ramifié contenant de 1 à 6 atomes de carbone,

**Ar** représente un radical aryle éventuellement substitué, choisi parmi les radicaux phényle et naphtyle,

**R$_1$** et **R$_2$** représentent chacun un atome d'hydrogène ou forment ensemble une liaison,

**R$_3$** est choisi parmi un atome d'hydrogène et un radical alkyle comprenant de 2 à 10 atomes de carbone en chaîne linéaire ou ramifiée,

**R$_4$** représente un radical hydrocarboné comprenant de 2 à 10 atomes de carbone en chaîne linéaire ou ramifiée et comportant éventuellement une ou plusieurs insaturations sous forme de doubles et de triples liaisons,

**X** représente le groupement CH ou un atome d'azote,

**Y** et **Z** représentent, indépendamment l'un de l'autre, un trait de valence ou un groupement paraphénylène,

étant entendu que le terme "éventuellement substitué" signifie que le radical considéré peut être éventuellement substitué par un ou plusieurs groupements choisis parmi chlore, fluor, brome, iode, amino, alkylamino, dialkylamino, nitro, cyano, alkyle, alkoxy, acyle, acyloxy, carboxy, alkoxycarbonyle, amido et carboxamido,

leurs stéréoisomères, leurs éventuels N-oxydes ainsi que leurs sels d'addition à un acide ou à une base, pharmaceutiquement acceptables.

**2.** Composés selon la revendication 1, dans laquelle X représente le groupement CH, leurs éventuels stéréoisomères, N-oxydes et sels d'addition à un acide ou à une base, pharmaceutiquement acceptables.

**3.** Composés selon la revendication 1, dans laquelle X représente l'azote, leurs éventuels stéréoisomères, N-oxydes et sels d'addition à un acide ou à une base, pharmaceutiquement acceptables.

**4.** Composés selon la revendication 1, dans laquelle Z représente un trait de valence, leurs éventuels stéréoisomères, N-oxydes et sels d'addition à un acide ou à une base, pharmaceutiquement acceptables.

**5.** Composés selon la revendication 1, dans laquelle Y représente un trait de valence, leurs éventuels stéréoisomères, N-oxydes et sels d'addition à un acide ou à une base, pharmaceutiquement acceptables.

**6.** Composés selon la revendication 1, dans laquelle $R_3$ représente l'hydrogène, leurs éventuels stéréoisomères, N-oxydes et sels d'addition à un acide ou à une base, pharmaceutiquement acceptables.

**7.** Composé selon l'une quelconque des revendications 1, 2, 4, 5, ou 6 qui est le (5Z)-6-[(3Z)-(1-hydroxynon-3-én-1-yl)phényl]hex-5-énoate de sodium, et ses stéréoisomères.

**8.** Procédé de préparation des dérivés de formule (I) selon la revendication 1, caractérisé en ce que l'on protège les fonctions aldéhyde du composé de formule (II) :

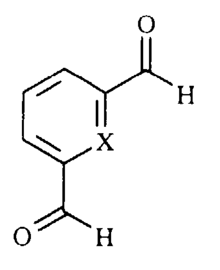

(II)

dans laquelle X est tel que défini dans la formule (I),
par l'orthoformiate de méthyle, à température ambiante, dans un solvant alcoolique anhydre, en présence d'un sel d'ammonium, pour obtenir le bisacétal de formule (III) :

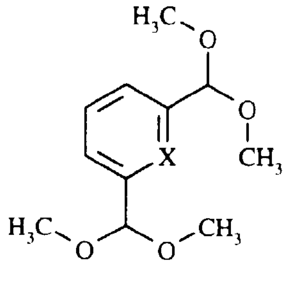

(III)

dans laquelle X est tel que décrit précédemment,
que l'on transforme en acétal aldéhyde de formule (IV) :

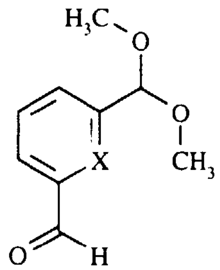

(IV)

dans laquelle X est tel que défini précédemment, par action d'une solution aqueuse d'acide sulfurique en solvant organique, en présence de silice, composé de formule (IV) que l'on fait réagir :

***soit*** : A) - lorsque le groupement Y défini dans la formule (I) représente une liaison et le radical $R_4$ du même dérivé de formule (I) souhaité contient une ou plusieurs insaturations (soit $R_{4A}$ ce radical), avec du méthylsulfate de triméthylsulfonium, en présence d'une solution aqueuse d'hydroxyde de sodium, dans un solvant, à température ambiante, pour obtenir l'époxyde de formule ($V_A$) :

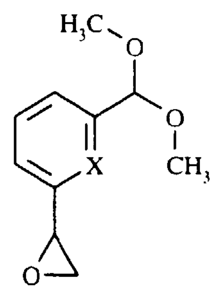

($V_A$)

dans laquelle X est tel que défini précédemment, qui, mis au contact d'un composé de formule (VI) :

$$H\text{-}R_{5A} \qquad (VI)$$

dans laquelle $R_{5A}$ est tel que $-R_{4A} = -CH_2-R_{5A}$ où $R_{4A}$ est tel que défini précédemment, ayant préalablement réagi avec une solution de butyllithium dans l'hexane en présence d'un chélatant des cations, dans un solvant polaire anhydre, à 0°C, conduit au composé de formule ($VI_A$) :

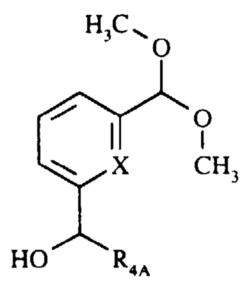

($VI_A$)

dans laquelle X et $R_{4A}$ sont tels que définis précédemment,

***soit*** : B) - lorsque le groupement Y du dérivé de formule (I) représente une liaison **et** le radical $R_4$ du même dérivé de formule (I) souhaité représente un groupement hydrocarboné saturé (soit $R_{4B}$ ce radical), avec le réactif de Grignard de formule ($V_B$) :

$R_{5B}$-Mg-Hal $(V_B)$

dans laquelle $R_{5B}$ est tel $R_{4B}$ = $-CH_2-R_{5B}$ où $R_{4B}$ est tel que défini précédemment, et Hal est un atome d'halogène,
dans le THF anhydre, à une température de -50°C, pour conduire au dérivé de formule $(VI_B)$ :

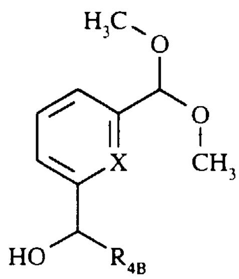

$(VI_B)$

dans laquelle X est tel que défini dans la formule (I) et $R_{4B}$ tel que défini dans la formule $(V_B)$
***soit*** : C) - lorsque le groupement Y du dérivé de formule (I) représente un groupement paraphénylène, avec le composé de formule $(V_C)$ :

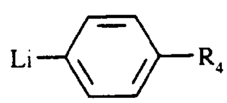

$(V_C)$

dans laquelle $R_4$ est tel que défini précédemment,
dans le THF anhydre, à une température de -50°C, pour fournir le composé de formule $(VI_C)$ :

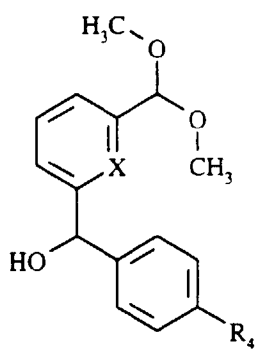

$(VI_C)$

dans laquelle X et $R_4$ sont tels que définis précédemment,

l'ensemble des composés de formules $(VI_A)$, $(VI_B)$ et $(VI_C)$ formant l'ensemble des composés de formule (VI) :

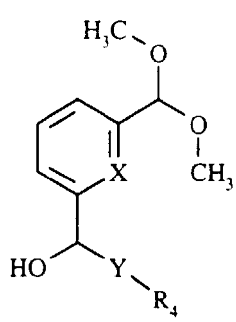

(VI)

dans laquelle X, Y et $R_4$ sont tels que définis précédemment, que l'on peut, si on le souhaite, soumettre à une réaction d'oxydation, par un mélange de chlorure d'oxalyle et de diméthylsulfoxide, dans un solvant chloré, afin d'obtenir le composé de formule (VII) :

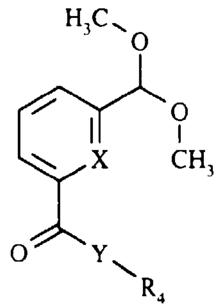

(VII)

dans laquelle X et $R_4$ sont tels que définis précédemment, qui est,

**soit** : A) traité par un réactif de formule ($VIII_A$) :

$$Li\text{-}R'_3 \qquad (VIII_A)$$

dans laquelle $R'_3$ représente un radical alkyle comprenant de 2 à 10 atomes de carbone en chaîne linéaire ou ramifiée,
dans les mêmes conditions que le composé de formule ($V_B$), pour conduire au composé de formule ($IX_A$) :

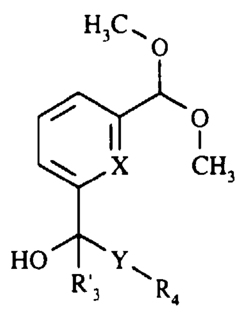

($IX_A$)

dans laquelle $R'_3$, $R_4$, X et Y sont tels que définis précédemment,
**soit** : B) traité par un réactif de formule ($VIII_B$) :

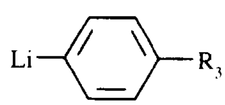

($VIII_B$)

dans laquelle $R_3$ est tel que défini précédemment,
dans les mêmes conditions que celle utilisées pour l'obtention du composé de formule ($VI_C$), pour conduire au composé de formule ($IX_B$) :

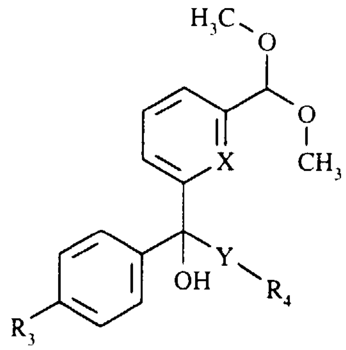

(IXB)

dans laquelle $R_3$, $R_4$, X et Y sont tels que définis précédemment,

l'ensemble des composés de formules (VI), ($IX_A$) et ($IX_B$) formant l'ensemble des composés de formule (IX) :

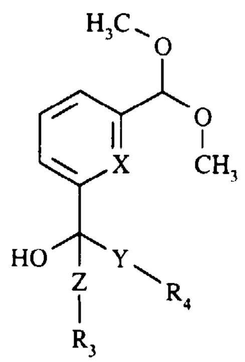

(IX)

dans laquelle $R_3$, $R_4$, X, Y et Z sont tels que définis précédemment,
composés de formule (IX) dont la fonction alcool est protégé par le chlorure de tertiobutyldiphénylsilyle, en présence d'imidazole dans un solvant, à température ambiante, pour donner le composé de formule (X) :

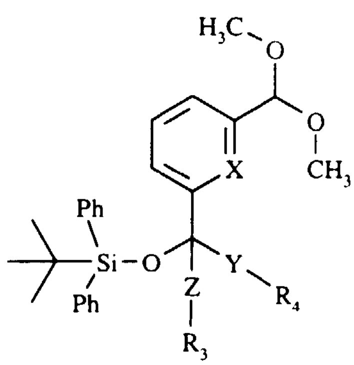

(X)

dans laquelle X, Y, Z, $R_3$ et $R_4$ sont tels que définis précédemment,
la fonction acétal du composé de formule (X) étant ensuite transformée de manière identique à celle utilisée pour le composé de formule (III), un aldéhyde de formule (XI) :

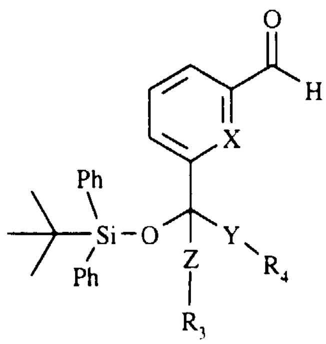

(XI)

dans laquelle X, Y, Z, $R_3$ et $R_4$ sont tels que définis précédemment,
qui est ensuite engagé dans une réaction de Wittig avec un composé de formule (XII) :

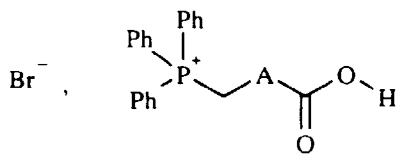

(XII)

dans laquelle A est tel que défini précédemment,
préalablement mis en solution dans un solvant anhydre, en présence de bis(triméthylsilyl)amidure de lithium et de hexaméthylphosphotriamide (HMPA), à une température de -80°C, pour obtenir le composé de formule (XIIIa) :

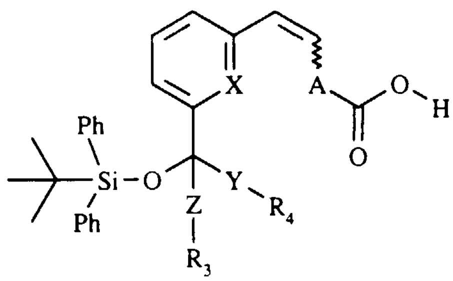

(XIIIa)

dans laquelle A, X, Y, Z, $R_3$ et $R_4$ sont tels que définis précédemment,
qui peut être, si on le souhaite, transformé, selon une technique classique d'estérification, en composé de formule (XIIIb) :

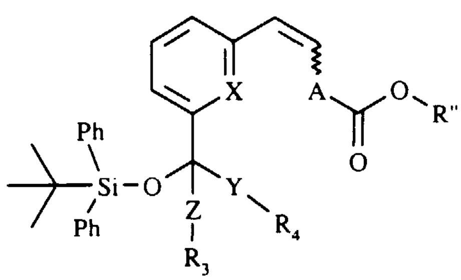

(XIIIb)

dans laquelle A, X, Y, Z, R", $R_3$ et $R_4$ sont tels que définis précédemment,
composés de formules (XIIIa) et (XIIIb), dont les isomères correspondants Z et E sont séparés sur colonne de silice, puis éventuellement engagés dans une réaction de déprotection de la fonction alcool, par action d'un fluorure de tétraalkylammonium, dans un solvant polaire, à 0°C, pour obtenir respectivement les alcools de formules (XIVa) et (XIVb) :

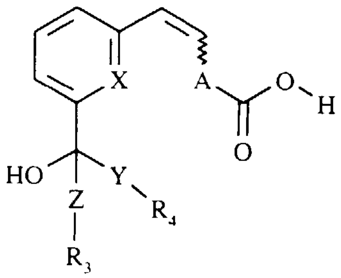 (XIVa) 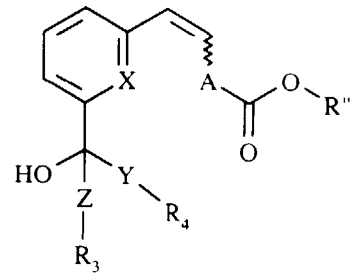 (XIVb)

dans lesquelles A, X, Y, Z, R'', $R_3$ et $R_4$ sont tels que définis précédemment,
le composé de formule (XIVa) étant ensuite éventuellement transformé, après protection temporaire de la fonction alcool, en amide de formule (XIVc) :

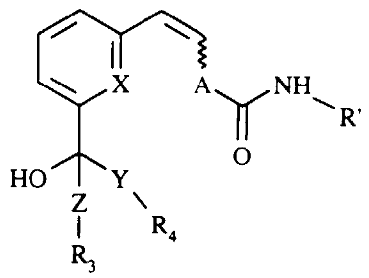 (XIVc)

dans laquelle A, X, Y, Z, R', $R_3$ et $R_4$ sont tels que définis précédemment,
l'ester de formule (XIIIb) pouvant également être directement saponifié selon la méthode décrite pour l'obtention de l'acide de formule (XIVa) puis éventuellement transformé en son chlorure d'acyle de formule (XIV) :

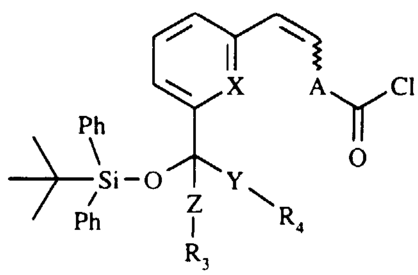 (XIV)

dans laquelle A, X, Y, Z, $R_3$ et $R_4$ sont tels que définis précédemment,
par action de chlorure d'oxalyle en présence de N,N-diméthylformamide dans le THF, à O°C, chlorure d'acyle de formule (XIV) qui est immédiatement traité par un chlorhydrate de N-alkylhydroxylamine de formule (XV) :

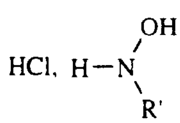 (XV)

dans laquelle R' est défini tel que précédemment,
en milieu basique, à O°C, pour conduire, après libération de la fonction alcool, à l'acide hydroxamique de formule (XIVd) :

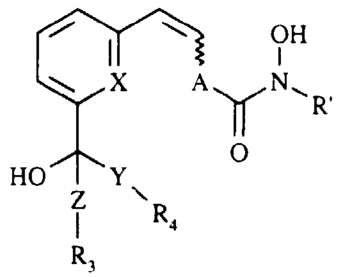

(XIVd)

dans laquelle A, X, Y, Z, R', $R_3$ et $R_4$ sont tels que définis précédemment,

chlorure d'acyle de formule (XIV) qui peut également être immédiatement traité par une hydrazine de formule $H_2N$-NHAr dans laquelle Ar est tel que défini précédemment pour conduire, après libération de la fonction alcool, aux hydrazides correspondantes de formule (XIVe) :

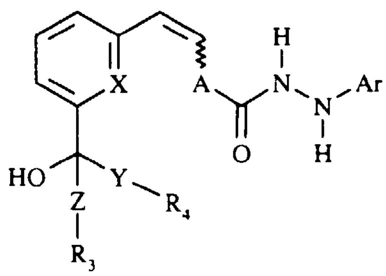

(XIVe)

dans laquelle A, X, Y, Z, Ar, $R_3$ et $R_4$ sont tels que définis précédemment,

l'ester de formule (XIIIb) conduisant également, selon les techniques classiques de réduction et après libération de l'alcool protégé sous forme d'éther silylé, aux alcool et éther de formule (XIVf) :

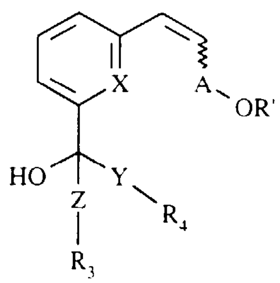

(XIVf)

dans laquelle A, X, Y, Z, R', $R_3$ et $R_4$ sont tels que définis précédemment,

conduisant eux-mêmes, après protection temporaire de la fonction alcool α-aromatique, aux dérivés halogénés de formule (XIVg) :

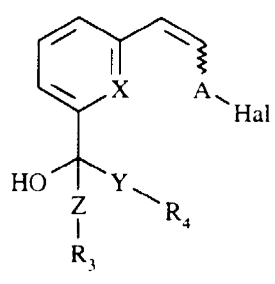

(XIVg)

dans laquelle A, X, Y, Z, $R_3$ et $R_4$ sont tels que définis précédemment,et Hal représente un atome d'halogène choisi parmi fluor, chlore, brome et iode,

permettant la préparation des phosphonates de formule (XIVh) :

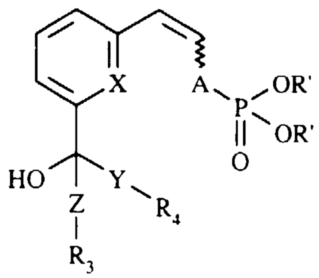

(XIVh)

dans laquelle A, X, Y, Z, R', $R_3$ et $R_4$ sont tels que définis précédemment,
ou, par réaction sur un composé de formule (XVI) :

$$H\text{-}B_1 \qquad \text{(XVI)}$$

dans laquelle $B_1$ représente le groupement -NHR",

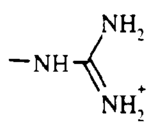

,

imidazolyle éventuelement substitué, pyrazolyle éventuellement substitué, ou le groupement tétrazolyle éventuellement substitué, et R" étant tel que précédemment défini,
dans des conditions opératoires classiques et appropriées, permettant la préparation des composés de formule (XVIa) :

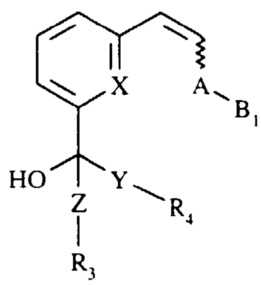

(XVIa)

dans laquelle A, $B_1$, X, Y, Z, $R_3$ et $R_4$ sont tels que définis précédemment,
les amines de formule (XVIb) :

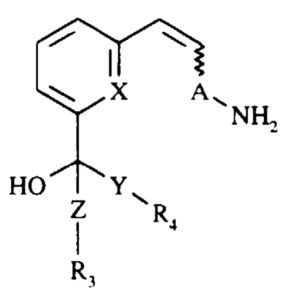

(XVIb)

dans laquelle A, X, Y, Z, $R_3$ et $R_4$ sont tels que définis précédemment,

étant alors obtenues par préparation de l'azide, à partir du composé halogéné précurseur de formule (XIVg), puis hydrogénation de cet azide,
amines de formule (XVIb) qui peuvent être transformées, par action d'un composé de formule (XVI') :

Hal-$B_2$ (XVI')

dans laquelle $B_2$ représente $-SO_2Ar$ ou -COR' et Hal, Ar et R' sont tels que définis précédemment,
en composés de formule (XVIc) :

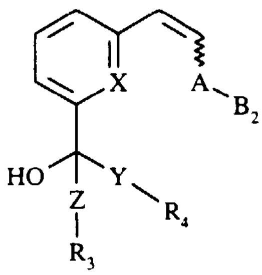
(XVIc)

dans laquelle A, $B_2$, X, Y, Z, $R_3$ et $R_4$ sont tels que définis précédemment,
l'ensemble des composés de formules (XVIa), (XVIb) et (XVIc) formant l'ensemble des composés de formule (XIVi) :

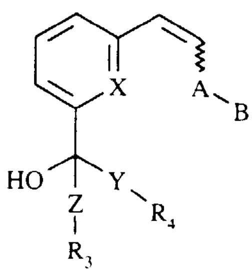
(XIVi)

dans laquelle A, X, Y, Z, $R_3$ et $R_4$ sont tels que définis précédemment, et B représente $B_1$, $B_2$ ou le groupement $NH_2$,
l'ester de formule (XIIIb) pouvant être également :

1/ - saponifié en acide correspondant puis traité par un composé de formule Li-R", dans laquelle R" est tel que décrit précédemment pour conduire, après libération de la fonction alcool jusqu'alors protégée sous forme d'éther silylé, aux cétones de formule (XIVj) :

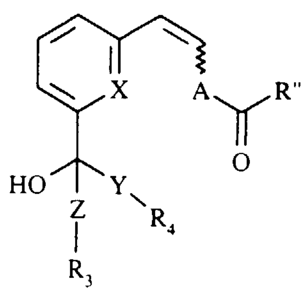
(XIVj)

dans laquelle A, X, Y, Z, R", $R_3$ et $R_4$ sont tels que définis précédemment,
2/ - traité par un équivalent d'hydrure de diisobutylaluminium (DIBAL) à basse température, pour conduire après libération de la fonction alcool, aux aldéhydes de formule (XIVk) :

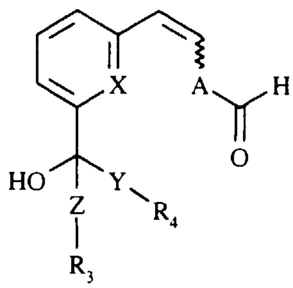

(XIVk)

dans laquelle A, X, Y, Z, $R_3$ et $R_4$ sont tels que définis précédemment,

ces aldéhydes de formule (XIVk) pouvant être :

***soit*** : α) traités par l'hydroxylamine pour conduire aux oximes de formule (XIVl) :

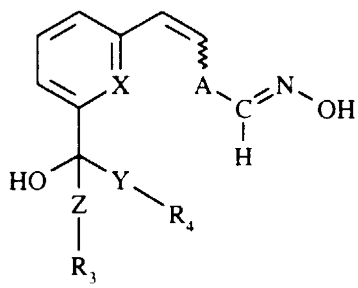

(XIVl)

dans laquelle A, X, Y, Z, $R_3$ et $R_4$ sont tels que définis précédemment,
les oximes de formule (XIVl) pouvant elles-mêmes être alkylées en composés de formule (XIVm) :

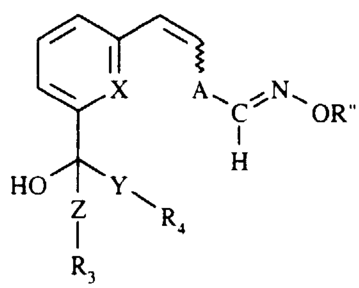

(XIVm)

dans laquelle A, X, Y, Z, R'', $R_3$ et $R_4$ sont tels que définis précédemment,
par un réactif de formule W-R'' dans laquelle W représente un groupement partant, et R'' est tel que défini précédemment,
***soit*** : β) traités par une amine de formule $H_2N$-R'', dans laquelle R'' est tel que défini précédemment, pour conduire aux imines de formule (XIVn) :

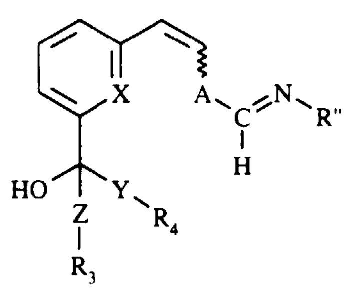

(XIVn)

dans laquelle A, X, Y, Z, R'', $R_3$ et $R_4$ sont tels que définis précédemment,

l'ensemble des dérivés de formules (XIVa) à (XIVn) formant l'ensemble des dérivés de formule (XVII) :

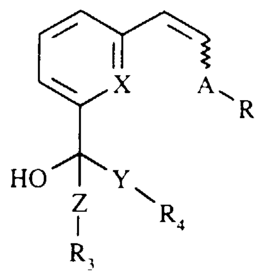

(XVII)

dans laquelle A, X, Y, Z, R, $R_3$ et $R_4$ sont tels que définis précédemment,
qui, éventuellement soumis à hydrogénation selon une technique classique, conduisent aux dérivés de formule (XVIII) :

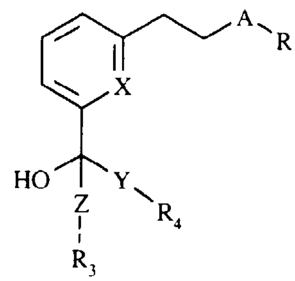

(XVIII)

dans laquelle A, X, Y, Z, R, $R_3$ et $R_4$ sont tels que définis précédemment,
l'ensemble des dérivés de formules (XVII) et (XVIII) formant l'ensemble des dérivés de formule (I) que l'on purifie le cas échéant selon une technique classique de purification et dont on sépare, si on le souhaite, les isomères par une technique classique de séparation et que l'on transforme, éventuellement en leurs N-oxydes et/ou en leurs sels d'addition à un acide ou à une base pharmaceutiquement acceptables.

**9.** Procédé de préparation des composés de formule I selon la revendication 3 dans laquelle X représente un atome d'azote caractérisé en ce que le produit de départ est la 2,6-diméthanol-pyridine dont on protège une des fonctions alcool par le chlorure de tertiobutyldiphénylsilyle pour obtenir le composé de formule (XIX) :

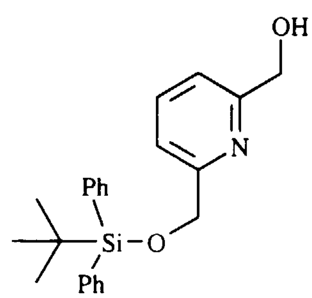

(XIX)

qui est ensuite oxydé en aldéhyde de formule (XX) :

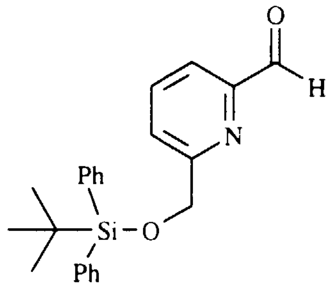

(XX)

qui est ensuite engagé dans la réaction de Wittig décrite pour l'obtention du composé de formule (XIIIb), pour conduire au composé de formule (XXI) :

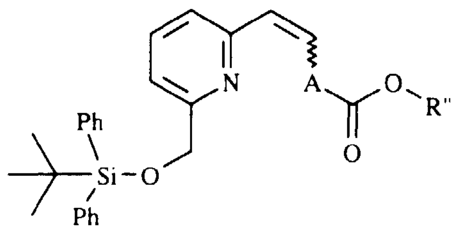

(XXI)

dans laquelle A et R'' sont tels que définis précédemment,
dont la fonction alcool est déprotégée selon la méthode utilisée pour l'obtention du composé de formule (XIVa), puis oxydée en aldéhyde selon la méthode décrite pour le composé (XVIII), conduisant ainsi au composé de formule (XXII) :

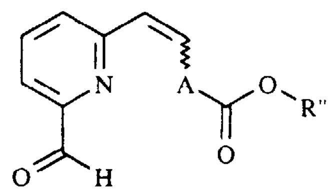

(XXII)

dans laquelle A et R'' sont tels que définis précédemment,
qui est ensuite traité de manière analogue au composé de formule (IV), selon les valeurs de Y et de $R_4$, pour donner les composés de formule (XXIII) :

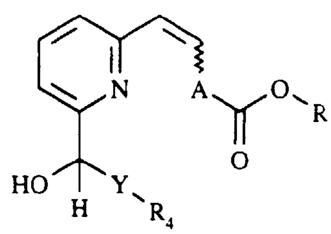

(XXIII)

dans laquelle A, Y, R'' et $R_4$ sont tels que définis précédemment,
qui sont ensuite éventuellement traités de manière analogue aux composés de formule (VI) afin d'obtenir les composés de formule (XXIV) :

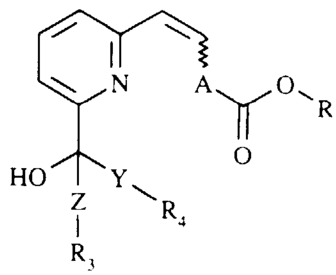

(XXIV)

dans laquelle A, Y, Z, R'', $R_3$ et $R_4$ sont tels que définis précédemment,
dont la fonction ester est éventuellement modifiée de façon identique aux méthodes employées pour l'obtention des composés (XIVa) à (XIVn) pour fournir les composés de formule (XXV) :

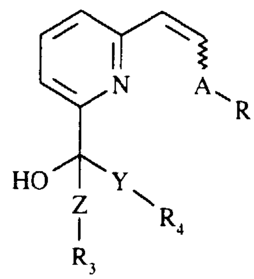

(XXV)

dans laquelle A, Y, Z, R, $R_3$ et $R_4$ sont tels que définis précédemment,
qui sont éventuellement soumis à hydrogénation, selon la technique décrite pour l'hydrogénation des composés de formmule (XVII), afin d'obtenir les composés de formule (XXVI) :

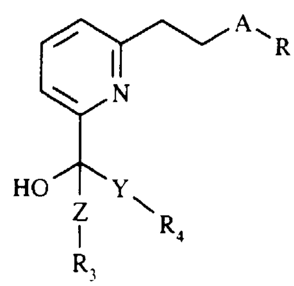

(XXVI)

dans laquelle A, Y, Z, R, $R_3$ et $R_4$ sont tels que définis précédemment,
l'ensemble des composés de formule (XXV) et (XXVI) formant l'ensemble des composés de formule (XXVII) :

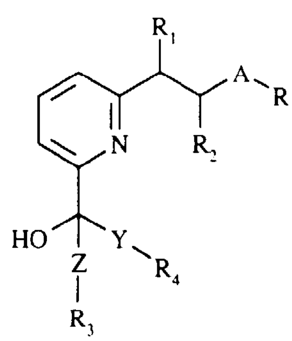

(XXVII)

dans laquelle A, R, $R_1$, $R_2$, $R_3$, $R_4$, Y, et Z sont tels que définis précédemment,
cas particulier du composé de formule (I) dans laquelle X représente l'azote que l'on purifie le cas échéant selon une technique classique de purification et dont on sépare, si on le souhaite, les isomères par une technique classique de séparation et que l'on transforme, si nécessaire, en leurs sels d'addition à un acide ou à une base pharmaceutiquement acceptables.

**10.** Compositions pharmaceutiques contenant comme principe actif au moins un composé selon l'une quelconque des revendications 1 à 7, seul ou en combinaison avec un ou plusieurs véhicules inertes, non toxiques et pharmaceutiquement acceptables.

**11.** Compositions pharmaceutiques selon la revendication 10 exerçant une activité anti-agrégante des plaquettes et utiles dans le traitement des affections dues ou reliées à des pathologies d'adhésion plaquettaire et notamment dans les cas de thromboembolisme, par dissolution des caillots de sang, de l'embolie pulmonaire, de l'embolie artérielle des extrémités, de l'infarctus du myocarde, de l'athérosclérose, de cancer malin, ainsi que pour maintenir l'homéostasie sanguine, en particulier dans la circulation extracorporelle.

## Patentansprüche

**1.** Verbindungen der Formel (I):

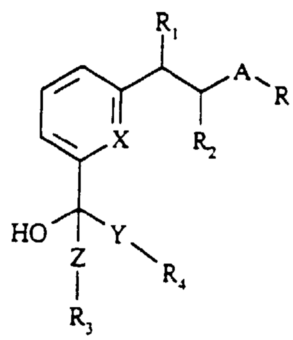

(I)

in der

**A** eine zweiwertige Kohlenwasserstoffgruppe mit 2 bis 10 Kohlenstoffatomen in gerader oder verzweigter Kette, die gegebenenfalls eine oder mehrere Unsättigungen in Form von Doppel- und Dreifachbindungen aufweist.
**R** ausgewählt aus Halogenatomen, wie Fluor-, Chlor-, Brom- oder Iodatomen, eine Gruppe der Formeln -OR', -COOR', -COR', P(O)(OR')$_2$, -CH=N-OR', -CONHR', -CH=NR". -CH=NAr, -NHSO$_2$Ar, -CON(OH)R', -NHR', -NHCOR', -CH=N-NHAr,

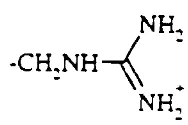

und einem gegebenenfalls substituierten Imidazolyl-, Pyrazolyl- oder Tetrazolyl-rest,
**R'** ausgewählt aus Wasserstoff und einer geradkettigen oder verzweigten Alkylgruppe mit 1 bis 6 Kohlenstoffatomen,
**R"** eine geradkettige oder verzweigte Alkylgruppe mit 1 bis 6 Kohlenstoffatomen,
**Ar** eine gegebenenfalls substituierte Arylgruppe ausgewählt aus Phenyl- und Naphthylgruppen,
**$R_1$** und **$R_2$** jeweils ein Wasserstoffatom oder gemeinsam eine Bindung,
**$R_3$** ausgewählt ist aus Wasserstoff und Alkylgruppen mit 2 bis 10 Kohlenstoffatomen in gerader oder verzweigter Kette,
**$R_4$** eine Kohlenwasserstoffgruppe mit 2 bis 10 Kohlenstoffatomen in gerader oder verzweigter Kette, die gegebenenfalls eine oder mehrere Unsättigungen in Form von Doppel- und Dreifachbindungen aufweist,
**X** eine Gruppe CH oder ein Stickstoffatom und
**Y** und **Z** unabhängig voneinander einen Bindungsstrich oder eine p-Phenylengruppe bedeuten,

wobei der Begriff "gegebenenfalls substituiert" bedeutet, daß die in Rede stehende Gruppe gegebenenfalls durch eine oder mehrere Gruppen ausgewählt aus Chlor, Fluor. Brom, Iod, Amino, Alkylamino, Dialkylamino, Nitro, Cyano, Alkyl, Alkoxy, Acyl, Acyloxy, Carboxy, Alkoxycarbonyl, Amido und Carboxamido substituiert sein kann,
deren Stereoisomeren, deren eventuelle N-Oxide sowie deren Additionssalze mit einer pharmazeutisch annehmbaren Säure oder Base.

**2.** Verbindungen nach Anspruch 1, worin X die Gruppe CH bedeutet, deren eventuelle Stereoisomere, N-Oxide und Additionssalze mit einer pharmazeutisch annehmbaren Säure oder Base.

**3.** Verbindungen nach Anspruch 1, worin X Stickstoff bedeutet, deren eventuelle Stereoisomere, N-Oxide und Additionssalze mit einer pharmazeutisch annehmbaren Säure oder Base.

**4.** Verbindungen nach Anspruch 1, worin Z einen Bindungsstrich bedeutet, deren eventuelle Stereoisomere, N-Oxide und Additionssalze mit einer pharmazeutisch annehmbaren Säure oder Base.

**5.** Verbindungen nach Anspruch 1, worin Y einen Bindungsstrich bedeutet, deren eventuelle Stereoisomere, N-Oxide und Additionssalze mit einer pharmazeutisch annehmbaren Säure oder Base.

**6.** Verbindungen nach Anspruch 1, worin $R_3$ Wasserstoff bedeutet, deren eventuelle Stereoisomere, N-Oxide und Additionssalze mit einer pharmazeutisch annehmbaren Säure oder Base.

**7.** Verbindungen nach irgendeinem der Ansprüche 1, 2, 3, 4, 5 oder 6, nämlich (5Z)-6-[(3Z)-(1-Hydroxynon-3-en-1-yl)-phenyl]-hex-5-ensäure-Natriumsalz und dessen Stereoisomere.

**8.** Verfahren zur Herstellung der Derivate der Formel (I) nach Anspruch 1, **dadurch gekennzeichnet,** daß man die Aldehydfunktionen der Verbindung der Formel (II):

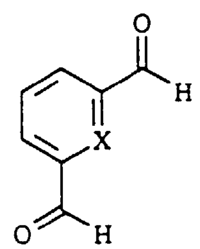

(II)

in der X die bezüglich der Formel (I) angegebenen Bedeutungen besitzt, mit Orthoameisensäuremethylester bei Raumtemperatur in einem wasserfreien alkoholischen Lösungsmittel und in Gegenwart eines Ammoniumsalzes schützt zur Bildung des Bisacetals der Formel (III):

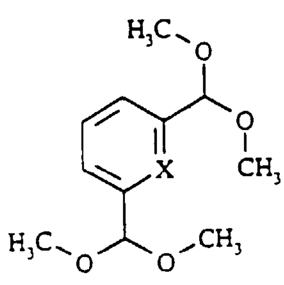

(III)

in der X die oben angegebenen Bedeutungen besitzt,
welches man durch Einwirkung einer wäßrigen Lösung von Schwefelsäure in organischem Lösungsmittel und in Gegenwart von Siliciumdioxid in den Acetalaldehyd der Formel (IV) umwandelt:

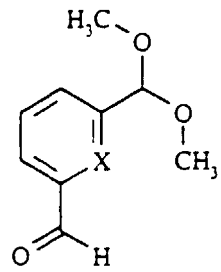

(IV)

in der X die oben angegebenen Bedeutungen besitzt,
welche Verbindung der Formel (IV) man:

***entweder:*** A) - wenn Y, wie es bezüglich der Formel (I) definiert worden ist, eine Bindung darstellt, und die Gruppe $R_4$ des gleichen angestrebten Derivats der Formel (I) eine oder mehrere Unsättigungen enthält (d. h. die Gruppe $R_{4A}$ dieser Gruppe),
mit Trimethylsulfoniummethylsulfat in Gegenwart einer wäßrigen Natriumhydroxidlösung in einem Lösungsmittel bei Umgebungstemperatur umsetzt zur Bildung des Epoxids der Formel ($V_A$):

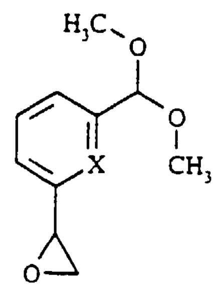

($V_A$)

in der X die oben angegebenen Bedeutungen besitzt, welche im Kontakt mit einer Verbindung der Formel (VI):

$$H - R_{5A} \qquad (VI)$$

in der $R_{5A}$ die Bedeutung besitzt von $-R_{4A} = -CH_2-R_{5A}$, worin
$R_{4A}$ die oben angegebenen Bedeutungen besitzt, welche man zuvor mit einer Lösung von Butyllithium in Hexan in Gegenwart eines Kationenchelatbildners in einem polaren wasserfreien Lösungsmittel bei 0°C umgesetzt hat,
zu der Verbindung der Formel ($VI_A$) führt:

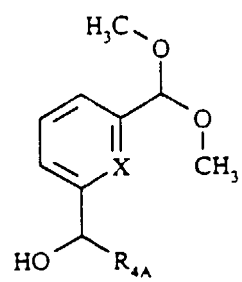

($VI_A$)

in der X und $R_{4A}$ die oben angegebenen Bedeutungen besitzen,
***oder***: B) - wenn die Gruppe Y des Derivats der Formel (I) eine Bindung **und** die Gruppe $R_4$ des gleichen

angestrebten Derivats der Formel (I) eine gesättigte Kohlenwasserstoffgruppe darstellt (d. h. die Gruppe $R_{4B}$ dieses Rests), mit dem Grignard-Reagens der Formel ($V_B$);

$$R_{5B} - Mg - Hal \qquad (V_B)$$

in der $R_{5B}$ die Bedeutungen besitzt von $R_{4B} = -CH_2-R_{5B}$, worin $R_{4B}$ die oben angegebenen Bedeutungen besitzt, und Hal ein Halogenatom darstellt,
in wasserfreiem THF bei einer Temperatur von -50°C umsetzt zur Bildung des Derivats der Formel ($VI_B$):

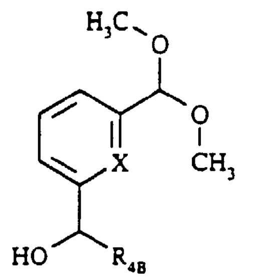

($VI_B$)

in der X die bezüglich der Formel (I) angegebenen Bedeutungen besitzt und $R_{4B}$ die bezüglich der Formel ($V_B$) angegebenen Bedeutungen besitzt,
***oder:*** C) - wenn die Gruppe Y des Derivats der Formel (I) eine p-Phenylengruppe darstellt,
mit einer Verbindung der Formel ($V_C$):

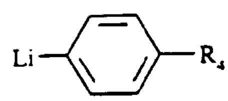

($V_C$)

in der $R_4$ die oben angegebenen Bedeutungen besitzt,
in wasserfreiem THF bei einer Temperatur von -50°C umsetzt zur Bildung der Verbindung der Formel ($VI_C$):

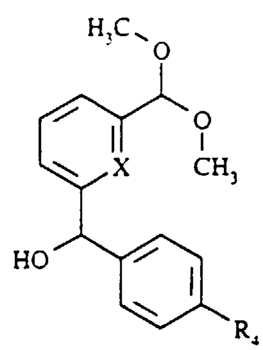

($VI_C$)

in der X und $R_4$ die oben angegebenen Bedeutungen besitzen,

wobei die Gesamtheit der Verbindungen der Formeln ($VI_A$), ($VI_B$) und ($VI_C$) gemeinsam die Verbindungen der Formel (VI) darstellen:

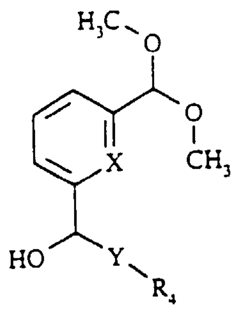

(VI)

in der X, Y und $R_4$ die oben angegebenen Bedeutungen besitzen,
welche man gewünschtenfalls einer Oxidationsreaktion mit einer Mischung aus Oxalylchlorid und Dimethylsulfoxid in einem chlorierten Lösungsmittel unterwerfen kann zur Bildung der Verbindung der Formel (VII):

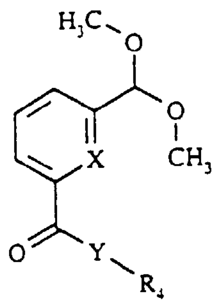

(VII)

in der X und $R_4$ die oben angegebenen Bedeutungen besitzen,
welche

***entweder:*** A) mit einem Reagens der Formel ($VIII_A$):

$$Li - R'_3 \qquad (VIII_A)$$

in der $R'_3$ eine Alkylgruppe mit 2 bis 10 Kohlenstoffatomen in gerader oder verzweigter Kette darstellt, unter den gleichen Bedingungen wie die Verbindung der Formel ($V_B$) behandelt zur Bildung der Verbindung der Formel ($IX_A$):

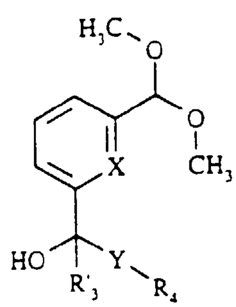

($IX_A$)

in der $R'_3$, $R_4$, X und Y die oben angegebenen Bedeutungen besitzen,
***oder:*** B) mit einem Reagens der Formel ($VIII_B$):

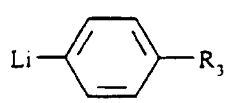

($VIII_B$)

in der $R_3$ die oben angegebenen Bedeutungen besitzt, unter den gleichen Bedingungen wie sie für die Bildung der Verbindung der Formel ($VI_C$) angewandt worden sind, behandelt zur Bildung der Verbindung der Formel ($IX_B$):

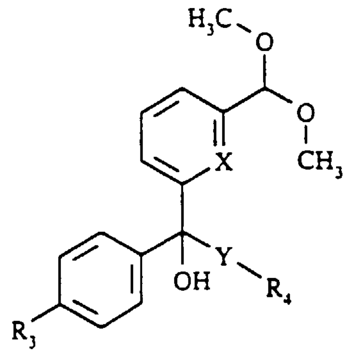

($IX_B$)

in der $R_3$, $R_4$, X und Y die oben angegebenen Bedeutungen besitzen, wobei die Gesamtheit derVerbindungen der Formeln (VI), ($IX_A$) und ($IX_B$) gemeinsam die Verbindungen der Formel (IX) bilden:

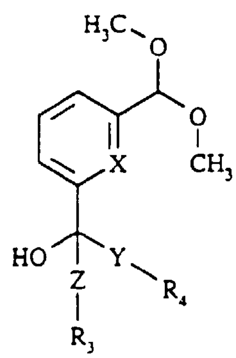

(IX)

in der $R_3$, $R_4$, X, Y und Z die oben angegebenen Bedeutungen besitzen, bei welchen Verbindungen der Formel (IX) man die Alkoholfunktion mit tert.-Butyldiphenylsilylchlorid in Gegenwart von Imidazol in einem Lösungsmittel bei Umgebungstemperatur schützt zur Bildung der Verbindung der Formel (X):

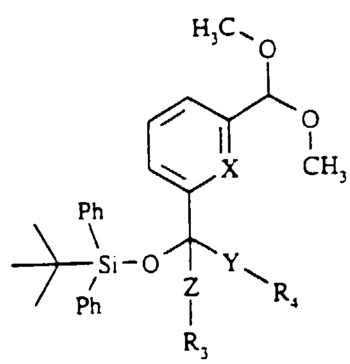

(X)

in der X, Y, Z, $R_3$ und $R_4$ die oben angegebenen Bedeutungen besitzen, wonach die Acetalfunktion der Verbindung der Formel (X) anschließend in identischer Weise wie der für die Verbindung der Formel (III) verwendeten umgewandelt wird zur Bildung des Aldehyds der Formel (XI):

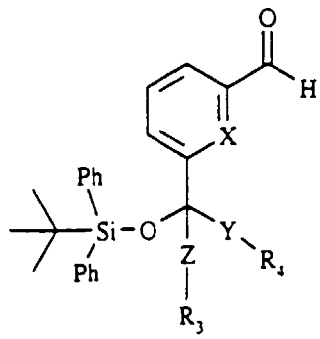

(XI)

in der X, Y, Z, $R_3$ und $R_4$ die oben angegebenen Bedeutungen besitzen, welcher anschließend einer Wittig-Reaktion mit einer Verbindung der Formel (XII):

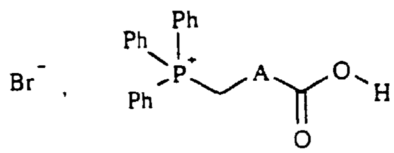

(XII)

in der A die oben angegebenen Bedeutungen besitzt,
die man zuvor in einem wasserfreien Lösungsmittel in Lösung gebracht hat, in Gegenwart von Lithium-bis(trimethylsilyl)-amid und Hexamethylphosphotriamid (HMPA) bei einer Temperatur von -80°C unterworfen wird zur Bildung der Verbindung der Formel (XIIIa):

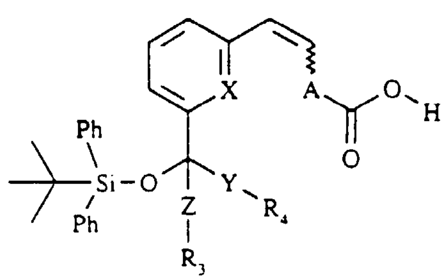

(XIIIa)

in der A, X, Y, Z, $R_3$ und $R_4$ die oben angegebenen Bedeutungen besitzen, welche gewünschtenfalls mit Hilfe einer klassischen Veresterungsmethode in die Verbindung der Formel (XIIIb) umgewandelt werden kann:

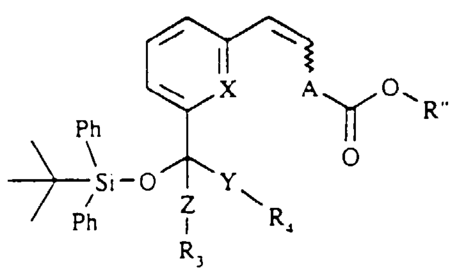

(XIIIb)

in der A, X, Y, Z, R", $R_3$ und $R_4$ die oben angegebenen Bedeutungen besitzen, welche Verbindungen der Formeln (XIIIa) und (XIIIb), deren entsprechende Z- und E-Isomeren auf einer mit Siliciumdioxid beschickten Säule getrennt werden und anschließend einer Reaktion zur Abspaltung der Schutzgruppe der Alkoholfunktion unterworfen werden durch Einwirkung eines Tetraalkylammoniumfluorids in einem polaren Lösungsmittel bei 0°C, so daß man die Alkohole der Formeln (XIVa) bzw. (XIVb) erhält:

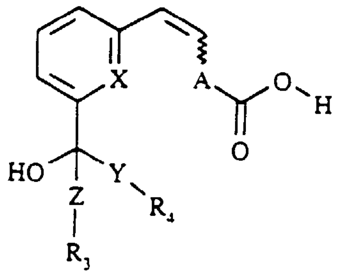

(XIVa)

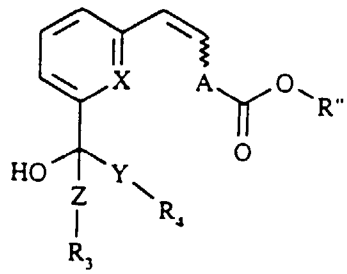

(XIVb)

in denen A, X, Y, Z, R", $R_3$ und $R_4$ die oben angegebenen Bedeutungen besitzen,
welche Verbindung der Formel (XIVa) anschließend gegebenenfalls, nach dem zeitweiligen Schutz der Alkoholfunktion, in das Amid der Formel (XIVc) umgewandelt wird:

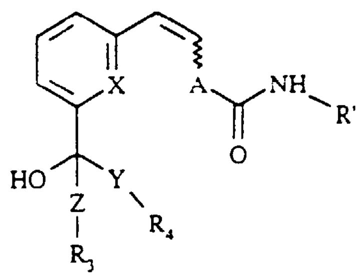

(XIVc)

in der A, X, Y, Z, R', $R_3$ und $R_4$ die oben angegebenen Bedeutungen besitzen,
während der Ester der Formel (XIIIb) ebenfalls direkt nach der zur Bildung der Säure der Formel(XIVa) beschriebenen Methode verseift und gegebenenfalls durch Einwirkung von Oxalylchlorid in Gegenwart von N,N-Dimethylformamid in THF bei 0°C in sein Acylchlorid der Formel (XIV) umgewandelt werden kann:

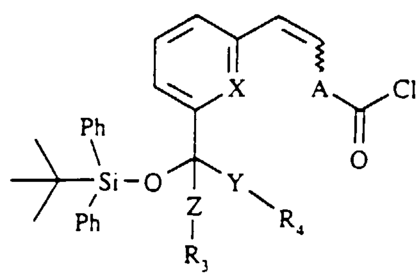

(XIV)

in der A, X, Y, Z, $R_3$ und $R_4$ die oben angegebenen Bedeutungen besitzen, welches Acylchlorid der Formel (XIV) sofort mit einem N-Alkylhydroxylamin-Hydrochlorid der Formel (XV):

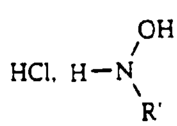

(XV)

in der R' die oben angegebenen Bedeutungen besitzt,
in basischem Medium bei 0°C behandelt wird, so daß man nach der Freisetzung der Alkoholfunktion die Hydroxamsäure der Formel (XIVd) erhält:

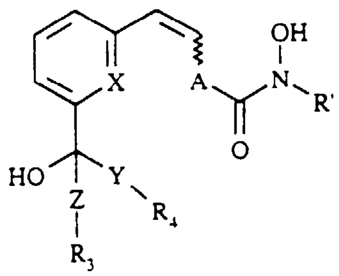

(XIVd)

in denen A, X, Y, Z, R', $R_3$ und $R_4$ die oben angegebenen Bedeutungen besitzen,
wobei das Acylchlorid der Formel (XIV) ebenfalls direkt mit einem Hydrazin der Formel $H_2N$-NHAr, in derAr die oben angegebenen Bedeutungen besitzt, behandelt werden kann, so daß man nach der Freisetzung der Alkoholfunktion die entsprechenden Hydrazide der Formel (XIVe) erhält:

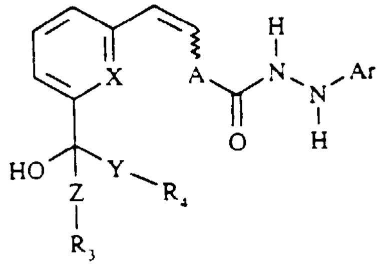

(XIVe)

in der A, X, Y, Z, Ar, $R_3$ und $R_4$ die oben angegebenen Bedeutungen besitzen,
welcher Ester der Formel (XIIIb) ebenfalls mit Hilfe klassischer Reduktionsmethoden und nach der Freisetzung des in Form des Silylethers geschützten Alkohols zu dem Alkohol und Ether der Formel (XIVf) führt:

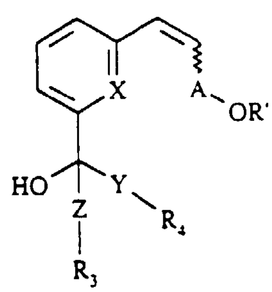

(XIVf)

in der A, X, Y, Z, R', $R_3$ und $R_4$ die oben angegebenen Bedeutungen besitzen,
welche ihrerseits nach der zeitweiligen Abspaltung der Schutzgruppe der α-aromatischen Alkoholfunktion zu den Halogenderivaten der Formel (XIVg) führt:

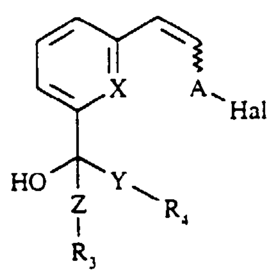

(XIVg)

in der A, X, Y, Z, $R_3$ und $R_4$ die oben angegebenen Bedeutungen besitzen und Hal ein Halogenatom ausgewählt aus Fluor, Chlor, Brom und Iod darstellt,
welche die Herstellung der Phosphonate der Formel (XIVh):

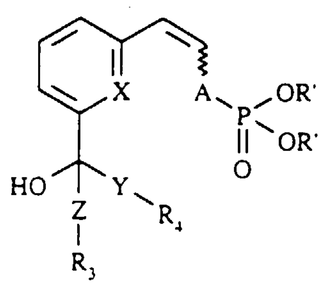

(XIVh)

in der A, X, Y, Z, R', $R_3$ und $R_4$ die oben angegebenen Bedeutungen besitzen,
ermöglichen,
oder, durch Reaktion mit einer Verbindung der Formel (XVI):

$$H - B_1 \qquad (XVI)$$

in der $B_1$ die Gruppe -NHR",

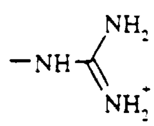

,

eine gegebenenfalls substituierte Imidazolylgruppe, eine gegebenenfalls substituierte Pyrazolylgruppe oder eine gegebenenfalls substituierte Tetrazolylgruppe darstellt und R" die oben angegebenen Bedeutungen besitzt.
unter klassischen und geeigneten Verfahrensbedingungen die Herstellung der Verbindungen der Formel (XVIa) ermöglichen:

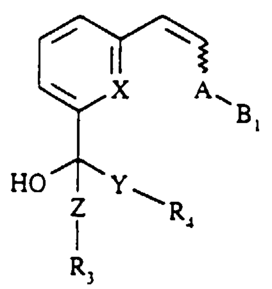

(XVIa)

in der A, $B_1$, X, Y, Z, $R_3$ und $R_4$ die oben angegebenen Bedeutungen besitzen,
wobei man die Amine der Formel (XVIb):

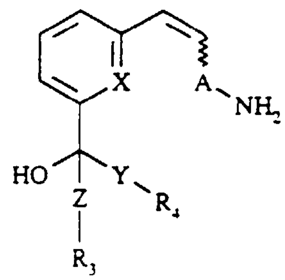

(XVIb)

in der A, X, Y, Z, $R_3$ und $R_4$ die oben angegebenen Bedeutungen besitzen, erhält durch Herstellung des Azids ausgehend von der Vorläufer-Halogenverbindung der Formel (XIVg) und Hydrieren dieses Azids, welche Amine der Formel (XVIb) durch Einwirkung einer Verbindung der Formel (XVI'):

Hal - $B_2$ (XVI')

in der $B_2$ -$SO_2$Ar oder -COR' darstellt, worin Hal, Ar und R' die oben angegebenen Bedeutungen besitzen, in die Verbindungen der Formel (XVIc) umgewandelt werden können:

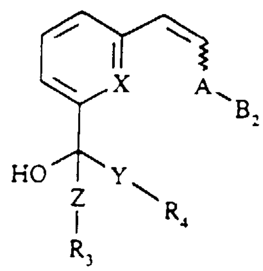

(XVIc)

in der A, $B_2$, X, Y, Z, $R_3$ und $R_4$ die oben angegebenen Bedeutungen besitzen, wobei die Gesamtheit derVerbindungen der Formeln (XVIa), (XVIb) und (XVIc) gemeinsam die Verbindungen der Formel (XIVi) bilden:

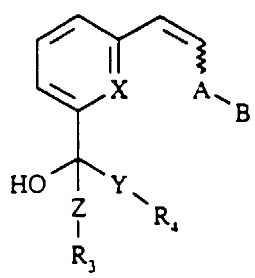

(XIVi)

in der A, X, Y, Z, $R_3$ und $R_4$ die oben angegebenen Bedeutungen besitzen und B $B_1$, $B_2$ oder die Gruppe $NH_2$ darstellt,
welcher Ester der Formel (XIIb) ebenfalls:

1/ - die entsprechende Säure verseift und dann mit einer Verbindung der Formel Li-R", in der R" die oben angegebenen Bedeutungen besitzt, behandelt werden kann, so daß man nach der Freisetzung der Alkoholfunktion, die bislang in Form des Silylethers geschützt ist, die Ketone der Formel (XIVj) erhält:

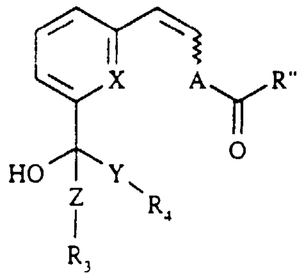

(XIVj)

in der A, X, Y, Z, R", $R_3$ und $R_4$ die oben angegebenen Bedeutungen besitzen,
2/ - mit einem Äquivalent Diisobutylaluminiumhydrid (DIBAL) bei niedriger Temperatur behandelt werden kann. so daß man nach der Freisetzung der Alkoholfunktion die Aldehyde der Formel (XIVk) erhält:

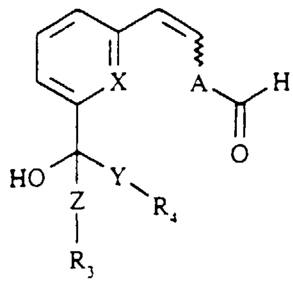

(XIVk)

in der A, X, Y, Z, $R_3$ und $R_4$ die oben angegebenen Bedeutungen besitzen, welche Aldehyde der Formel (XIVk):

***entweder:*** α) mit Hydroxylamin behandelt werden können zur Bildung der Oxime der Formel (XIVl):

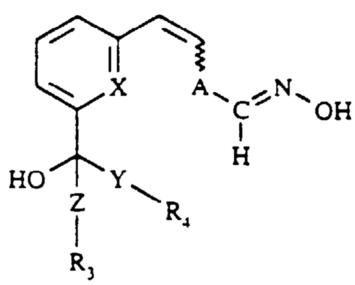

(XIVl)

in der A, X, Y, Z, $R_3$ und $R_4$ die oben angegebenen Bedeutungen besitzen,
wobei die Oxime der Formel (XIVl) ihrerseits mit einem Reagens der Formel W-R", in der W eine austretende Gruppe darstellt und R" die oben angegebenen Bedeutungen besitzt, zu den Verbindungen der Formel (XIVm) alkyliert werden können:

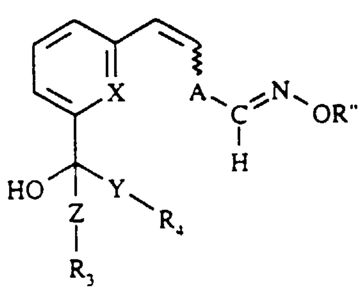

(XIVm)

in der A, X, Y, Z, R", $R_3$ und $R_4$ die oben angegebenen Bedeutungen besitzen,

***oder:*** β) mit einem Amin der Formel $H_2N\text{-}R''$, in der R" die oben angegebenen Bedeutungen besitzt, behandelt werden können zur Bildung der Imine der Formel (XIVn):

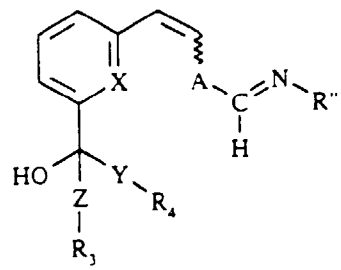

(XIVn)

in der A, X, Y, Z, R", $R_3$ und $R_4$ die oben angegebenen Bedeutungen besitzen,

wobei die Gesamtheit der Derivate der Formeln (XIva) bis (XIVn) gemeinsam die Derivate der Formel (XVII) bilden:

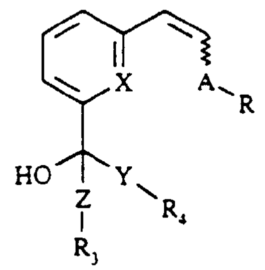

(XVII)

in der A, X, Y, Z, R, $R_3$ und $R_4$ die oben angegebenen Bedeutungen besitzen, welche gegebenenfalls einer Hydrierung nach einer klassischen Methode unterworfen werden können und zu den Derivaten der Formel (XVIII) führen:

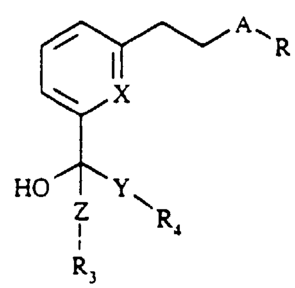

(XVIII)

in der A, X, Y, Z, R, $R_3$ und $R_4$ die oben angegebenen Bedeutungen besitzen, wobei die Gesamtheit der Derivate der Formeln (XVII) und (XVIII) die Gesamtheit der Derivate der Formel (I) bilden, welche man gegebenenfalls mit Hilfe einer klassischen Reinigungsmethode reinigt und gewünschtenfalls mit Hilfe einer klassischen Trennungsmethode in die Isomeren auftrennt und gegebenenfalls in ihre N-Oxide und/oder ihre Additionssalze mit einer pharmazeutisch annehmbaren Säure oder Base umwandelt.

**9.** Verfahren zur Herstellung der Verbindungen der Formel (I) nach Anspruch 3, in der X ein Stickstoffatom darstellt, **dadurch gekennzeichnet,** daß das Ausgangsmaterial 2,6-Dimethanol-pyridin ist, dessen Alkoholfunktionen man mit tert.-Butyldiphenylsilylchlorid schützt, so daß man die Verbindung der Formel (XIX) erhält:

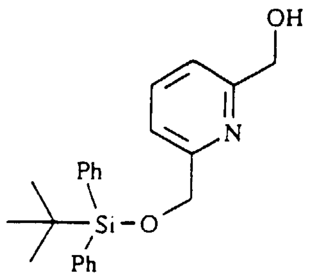

(XIX)

welche anschließend zu dem Aldehyd der Formel (XX) oxidiert wird:

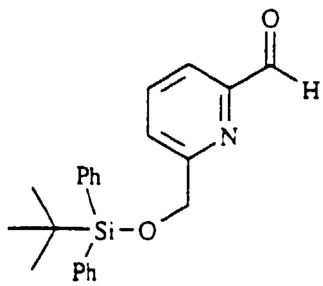

(XX)

der anschließend der bezüglich der Herstellung der Verbindung der Formel (XIIIb) beschriebenen Wittig-Reaktion unterworfen wird zur Bildung der Verbindung der Formel (XXI):

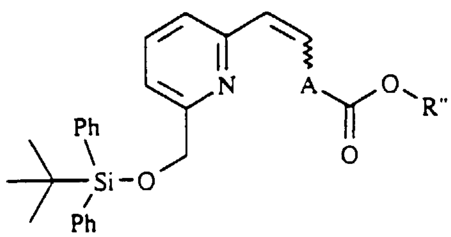

(XXI)

in der A und R" die oben angegebenen Bedeutungen besitzen,
deren Alkoholfunktion nach der für die Bildung der Verbindung der Formel (XIVa) beschriebenen Methode von der Schutzgruppe befreit und dann nach der für die Verbindung der Formel (XVIII) beschriebenen Methode in die Aldehydfunktion oxidiert wird und in dieser Weise zu der Verbindung der Formel (XXII) führt:

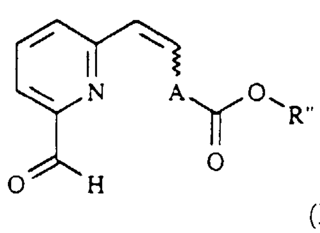

(XXII)

in der A und R" die oben angegebenen Bedeutungen besitzen,
welche anschließend in Abhängigkeit von den Bedeutungen von Y und $R_4$ in analoger Weise zu der Verbindung der Formel (IV) behandelt wird zur Bildung der Verbindungen der Formel (XXIII):

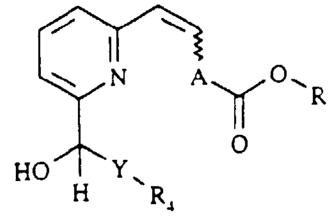

(XXIII)

in der A, Y, R" und $R_4$ die oben angegebenen Bedeutungen besitzen, welche anschließend gegebenenfalls in analoger Weise zu den Verbindungen der Formel (VI) behandelt werden, so daß man die Verbindungen der Formel (XXIV) erhält:

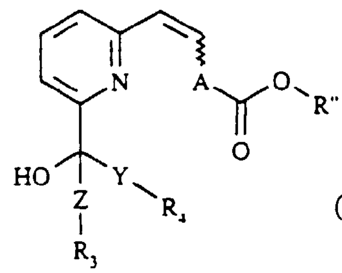

(XXIV)

in der A, Y, Z, R", $R_3$ und $R_4$ die oben angegebenen Bedeutungen besitzen, deren Esterfunktion gegebenenfalls modifiziert wird in identischer Weise zu den Methoden, die zur Bildung der Verbindungen (XIVa) bis (XIVn) angewandt worden sind zur Bildung der Verbindungen der Formel (XXV):

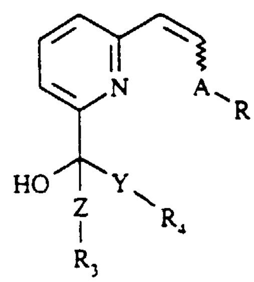

(XXV)

in der A, Y, Z, R, $R_3$ und $R_4$ die oben angegebenen Bedeutungen besitzen, welche gegebenenfalls einer Hydrierung unterzogen werden nach der für die Hydrierung der Verbindungen der Formel (XVII) beschriebenen Technik unterworfen werden zur Bildung der Verbindungen der Formel (XXVI):

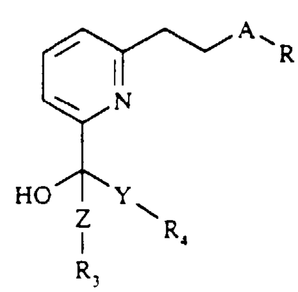

(XXVI)

in der A, Y, Z, R, $R_3$ und $R_4$ die oben angegebenen Bedeutungen besitzen, wobei die Gesamtheit der Verbindungen der Formeln (XXV) und (XXVI) gemeinsam die Verbindungen der Formel (XXVII) bilden:

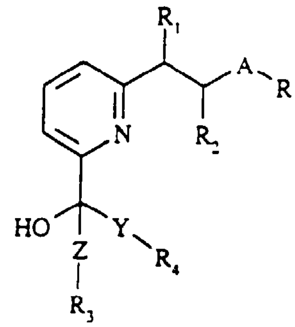

(XXVII)

in der A, R, $R_1$, $R_2$, $R_3$, $R_4$, Y und Z die oben angegebenen Bedeutungen besitzen,
einem Sonderfall der Verbindungen der Formel (I), in der X Stickstoff bedeutet, welche Verbindungen man gegebenenfalls mit Hilfe einer klassischen Reinigungsmethode reinigt, die man gewünschtenfalls mit Hilfe einer klassischen Trennungsmethode in ihre Isomeren auftrennt und erforderlichenfalls mit Hilfe einer pharmazeutisch annehmbaren Säure oder Base in ihre Additionssalze umwandelt.

**10.** Pharmazeutische Zubereitungen enthaltend als Wirkstoff mindestens eine Verbindung nach einem der Ansprüche 1 bis 7 allein oder in Kombination mit einem oder mehreren inerten, nichttoxischen und pharmazeutisch annehmbaren Trägermaterialien.

**11.** Pharmazeutische Zubereitungen nach Anspruch 10 mit einer anti-plättchenaggregierenden Wirkung nützlich zur Behandlung von Erkrankungen, die eine Folge sind von oder verknüpft sind mit pathologischen Zuständen der Plättchenaggregation und insbesondere Fällen von Thromboembolie, durch Auflösen von Blutpfropfen, von Lungenembolie, Arterienembolie der Extremitäten, Myokardinfarkt, Atherosklerose, bösartigem Krebs sowie zur Aufrechterhaltung einer Bluthämostasie und insbesondere der extrakorporalen Zirkulation.

## Claims

**1.** Compounds of formula (I)

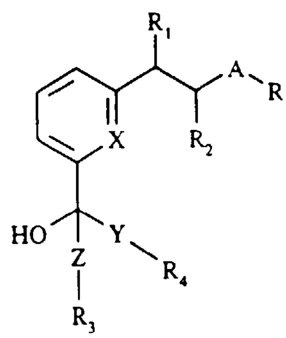

(I)

wherein:

**A** represents a divalent hydrocarbon radical containing from 2 to 10 carbon atoms in linear or branched chain and optionally containing one or more unsaturations in the form of double and triple bonds,

**R** is selected from a halogen atom, selected from fluorine, chlorine, bromine and iodine, a radical -OR', -COOR', -COR', $P(O)(OR')_2$, -CH=N-OR', -CONHR', -CH=NR", -CH=NAr, $-NHSO_2Ar$, -CON(OH)R', -NHR', -NHCOR', -CH=N-NHAr,

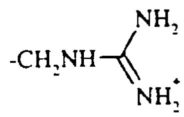

and an optionally substituted imidazolyl, pyrazolyl or tetrazolyl radical,

**R'** is selected from a hydrogen atom and a linear or branched alkyl radical containing from 1 to 6 carbon atoms,

**R"** represents a linear or branched alkyl radical containing from 1 to 6 carbon atoms,

**Ar** represents an optionally substituted aryl radical selected from the radicals phenyl and naphthyl,

$\mathbf{R_1}$ and $\mathbf{R_2}$ each represents a hydrogen atom or together form a bond,

$\mathbf{R_3}$ is selected from a hydrogen atom and an alkyl radical containing from 2 to 10 carbon atoms in linear or branched chain,

$\mathbf{R_4}$ represents a hydrocarbon radical containing from 2 to 10 carbon atoms in linear or branched chain and optionally containing one or more unsaturations in the form of double and triple bonds,

**X** represents a CH group or a nitrogen atom,

**Y** and **Z** each represents, independently of the other, a valency bond or a para-phenylene group,

it being understood that the expression "optionally substituted" denotes that the radical in question may optionally be substituted by one or more groups selected from chlorine, fluorine, bromine, iodine, amino, alkylamino, dialkylamino, nitro, cyano, alkyl, alkoxy, acyl, acyloxy, carboxy, alkoxycarbonyl, amido and carboxamido,

their stereoisomers, their possible N-oxides and also the pharmaceutically acceptable addition salts thereof with an acid or base.

**2.** Compounds according to claim 1, wherein X represents a CH group, their possible stereoisomers, N-oxides and pharmaceutically acceptable addition salts with an acid or base.

**3.** Compounds according to claim 1, wherein X represents nitrogen, their possible stereoisomers, N-oxides and pharmaceutically acceptable addition salts with an acid or base.

**4.** Compounds according to claim 1, wherein Z represents a valency bond, their possible stereoisomers, N-oxides and pharmaceutically acceptable addition salts with an acid or base.

**5.** Compounds according to claim 1, wherein Y represents a valency bond, their possible stereoisomers, N-oxides and pharmaceutically acceptable addition salts with an acid or base.

**6.** Compounds according to claim 1, wherein $R_3$ represents hydrogen, their possible stereoisomers, N-oxides and pharmaceutically acceptable addition salts with an acid or base.

**7.** Compound according to any one of claims 1, 2, 4, 5 and 6, which is sodium (5Z)-6-[(3Z)-(1-hydroxynon-3-en-1-yl) phenyl]hex-5-enoate, and its stereoisomers.

**8.** Process for the preparation of compounds of formula (I) according to claim 1, characterised in that the aldehyde functions of the compound of formula (II) :

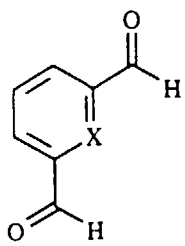

(II)

wherein X is as defined for formula (I)
are protected with methyl orthoformate, at room temperature, in an anhydrous alcoholic solvent, in the presence of an ammonium salt, to obtain the bis-acetal of formula (III) :

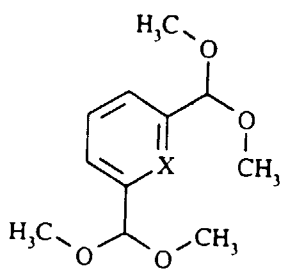

(III)

wherein X is as defined above,
which is converted into an acetal aldehyde of formula (IV) :

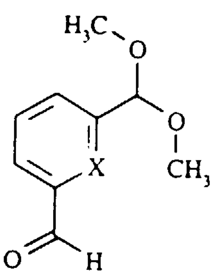

, (IV)

wherein X is as defined above,
by the action of an aqueous solution of sulphuric acid in an organic solvent in the presence of silica,
which compound of formula (IV) is reacted :

A) - when the group Y defined in formula (I) represents a bond and the radical $R_4$ of the same desired compound of formula (I) contains one or more unsaturations (designated the radical $R_{4A}$),
with trimethylsulphonium methyl sulphate, in the presence of an aqueous sodium hydroxide solution, in a solvent, at room temperature, to obtain the epoxide of formula ($V_A$):

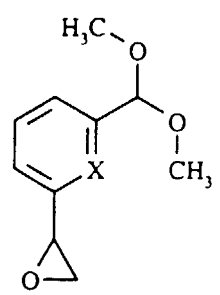

($V_A$)

wherein X is as defined above,

which, contacted in an anhydrous polar solvent, at 0°C, with a compound of formula (VI) :

$$H\text{-}R_{5A} \qquad (VI)$$

wherein $R_{5A}$ is such that $-R_{4A} = -CH_2-R_{5A}$ where $R_{4A}$ is as defined above,
that has previously been reacted with a solution of butyllithium in hexane in the presence of a cation chelator, yields the compound of formula ($VI_A$) :

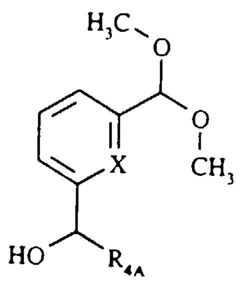

($VI_A$)

wherein X and $R_{4A}$ are as defined above,

B) when the group Y of the compound of formula (I) represents a bond and the radical R4 of the same desired compound of formula (I) represents a saturated hydrocarbon group (designated the radical $R_{4B}$),
with the Grignard reagent of formula ($V_B$) :

$$R_{5B}\text{-Mg-Hal} \qquad (V_B)$$

wherein $R_{5B}$ is such that $R_{4B} = -CH_2-R_{5B}$ where $R_{4B}$ is as defined above and Hal is a halogen atom,
in anhydrous THF, at a temperature of -50°C, to yield the compound of formula ($VI_B$) :

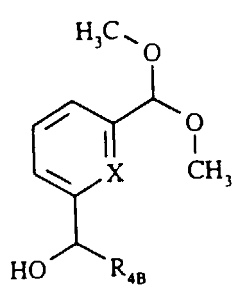

($VI_B$)

wherein X is as defined for formula (I) and $R_{4B}$ is as defined for formula ($V_B$)

C) - when the group Y of the compound of formula (I) represents a para-phenylene group, with the compound of formula of formula ($V_C$) :

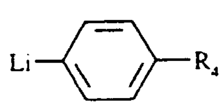

($V_C$)

wherein $R_4$ is as defined above,
in anhydrous THF, at a temperature of -50°C, to yield the compound of formula ($VI_C$) :

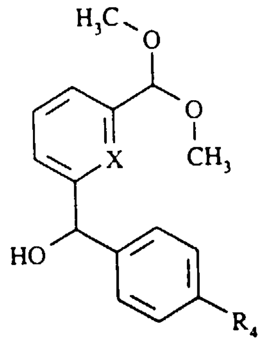

(VI$_C$)

wherein X and $R_4$ are as defined above,

the totality of the compounds of formulae (VI$_A$), (VI$_B$) and (VI$_C$) forming the totality of the compounds of formula (VI) :

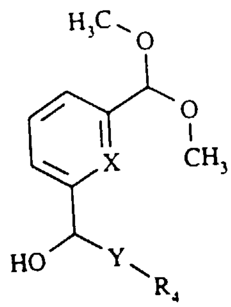

(VI)

wherein X, Y and $R_4$ are as defined above, which may, if desired, be subjected to oxidation with a mixture of oxalyl chloride and dimethyl sulphoxide, in a chlorinated solvent, to obtain the compound of formula (VII) :

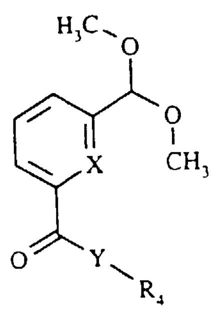

(VII)

wherein X and $R_4$ are as defined above, which is

**either** A) treated with a reagent of formula (VIII$_A$) :

$$Li\text{-}R'_3 \qquad (VIII_A)$$

wherein $R'_3$ represents an alkyl radical containing from 2 to 10 carbon atoms in linear or branched chain, under the same conditions as the compound of formula (V$_B$), to yield the compound of formula (IX$_A$) :

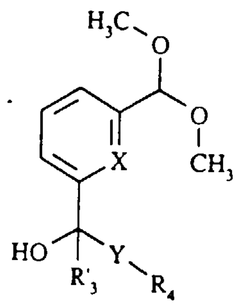

$(IX_A)$

wherein $R'_3$, $R_4$, X and Y are as defined above,

**or :** B) treated with a reagent of formula $(VIII_B)$ :

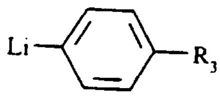

$(VIII_B)$

wherein $R_3$ is as defined above,
under the same conditions is those used for obtaining the compound of formula $(VI_C)$, to yield the compound of formula $(IX_B)$ :

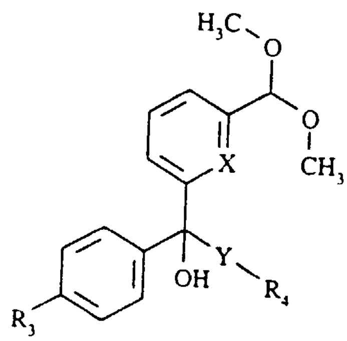

$(IX_B)$

wherein $R_3$, $R_4$, X and Y are as defined above,

the totality of the compounds of formula (VI), $(IX_A)$ and $(IX_B)$ forming the totality of the compounds of formula (IX) :

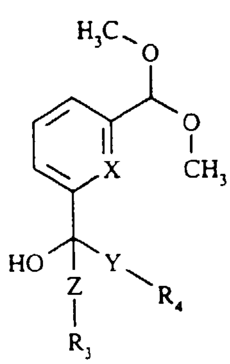

(IX)

wherein $R_3$, $R_4$, X, Y and Z are as defined above,
the alcohol function of which compounds of formula (IX) is protected by tert-butyl-diphenylsilyl chloride, in the

presence of imidazole in a solvent, at room temperature, to yield the compound of formula (X) :

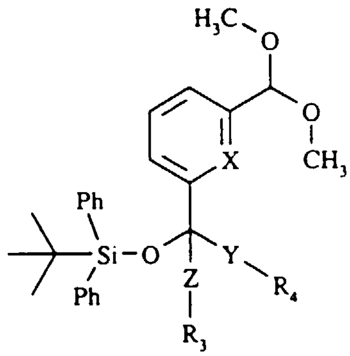

(X)

wherein X, Y Z, $R_3$ and $R_4$ are as defined above,
the acetal function of the compound of formula (X) then being converted in a manner identical to that used for the compound of formula (III) into the aldehyde of formula (XI) :

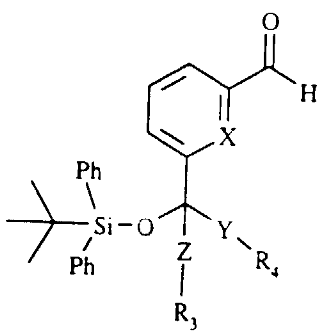

(XI)

wherein X, Y, Z, $R_3$ and $R_4$ are as defined above,
which is then subjected to a Wittig reaction with a compound of formula (XII) :

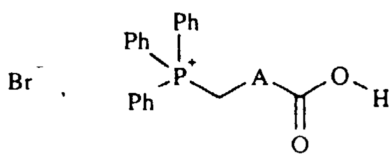

(XII)

wherein A is as defined above,
previously dissolved in an anhydrous solvent, in the presence of lithium bis(trimethylsilyl)amide and hexamethylphosphotriamide (HMPA), at a temperature of -80°C, to obtain the compound of formula (XIIIa) :

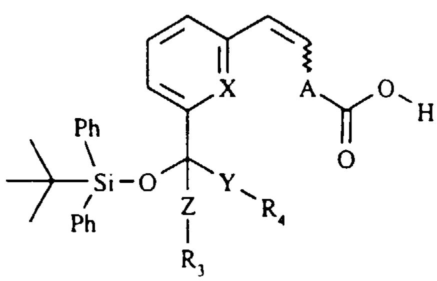

(XIIIa)

wherein A, X, Y, Z, $R_3$ and $R_4$ are as defined above,

which may, if desired, be converted according to a conventional esterification technique into a compound of formula (XIIIb) :

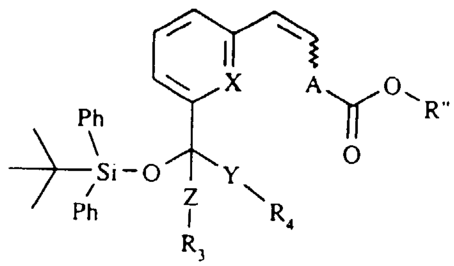

(XIIIb)

wherein A, X, Y, Z, R", $R_3$ and $R_4$ are as defined above,
the corresponding Z and E isomers of which compounds of formulae (XIIIa) and (XIIIb) are separated on a silica column, then optionally subjected to a reaction for the deprotection of the alcohol function by the action of tetraalkylammonium fluoride in a polar solvent, at 0°C, to obtain the alcohols of formulae (XIVa) and (XIVb) respectively :

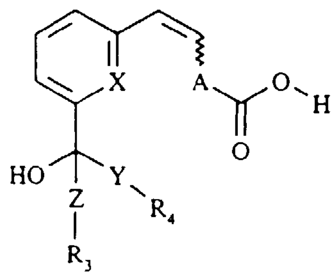

(XIVa)

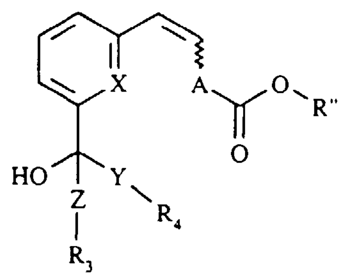

(XIVb)

wherein A, X, Y, Z, R", $R_3$ and $R_4$ are as defined above,
the compound of formula (XIVa) then optionally being converted, after temporary protection of the alcohol function, into the amide of formula (XIVc):

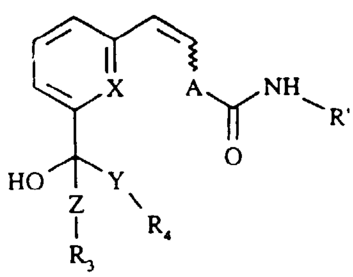

(XIVc)

wherein A, X, Y, Z, R', $R_3$ and $R_4$ are as defined above,
it also being possible for the ester of formula (XIIIb) to be hydrolysed directly, in accordance with the method described for obtaining the acid of formula (XIVa), then optionally converted into its acyl chloride of formula (XIV) :

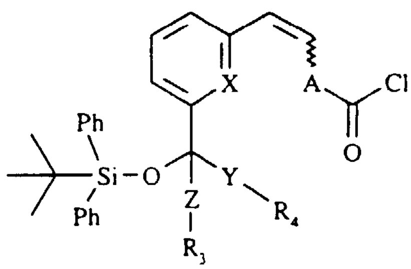

(XIV)

wherein A, X, Y, Z, $R_3$ and $R_4$ are as defined above, by the action of oxalyl chloride in the presence of N,N-dimethylformamide in THF at 0°C, which acyl chloride of formula (XIV) is immediately treated with an N-alkylhydroxylamine hydrochloride of formula (XV) :

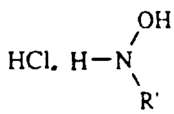

(XV)

wherein R' is as defined above, in basic medium, at 0°C, to yield, after freeing the alcohol function, the hydroxamic acid of formula (XIVd) :

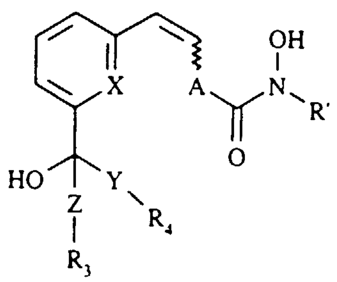

(XIVd)

wherein A, X, Y, Z, R', $R_3$ and $R_4$ are as defined above, it also being possible for the acyl chloride of formula (XIV) to be treated immediately with a hydrazine of formula $H_2N$-NHAr wherein Ar is as defined above to yield, after freeing the alcohol function, the corresponding hydrazide of formula (XIVe) :

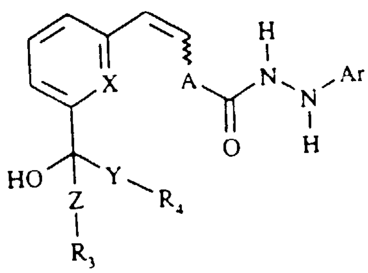

(XIVe)

wherein A, X, Y, Z, Ar, $R_3$ and $R_4$ are as defined above, the ester of formula (XIIIb) likewise leading, in accordance with conventional reduction techniques and after freeing the alcohol function protected in silylated ether form, to the alcohol and ether of formula (XIVf):

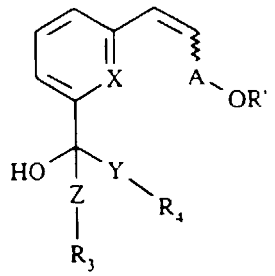

(XIVf)

wherein A, X, Y, Z, R', $R_3$ and $R_4$ are as defined above,
themselves resulting, after temporary protection of the α-aromatic alcohol function, in the halogenated compounds of formula (XIVg) :

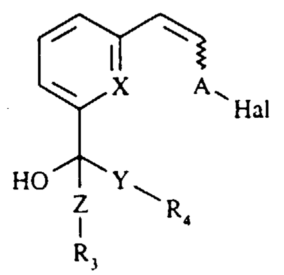

(XIVg)

wherein A, X, Y, Z, $R_3$ and $R_4$ are as defined above and Hal represents a halogen atom selected from fluorine, chlorine, bromine and iodine,
enabling the preparation of the phosphonates of formula (XIVh) :

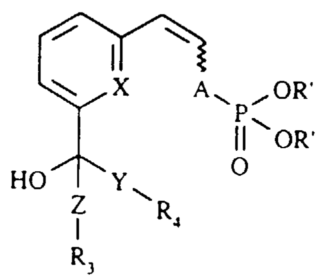

(XIVh)

wherein A, X, Y, Z, R', $R_3$ and $R_4$ are as defined above,
or, by reaction with a compound of formula (XVI) :

$$H\text{-}B_1 \qquad (XVI)$$

wherein $B_1$ represents a -NHR",

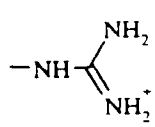

,

optionally substituted imidazolyl, optionally substituted pyrazolyl or optionally substituted tetrazolyl group, R" being

as defined above,
under conventional and appropriate operating conditions, enabling the preparation of the compounds of formula (XVIa) :

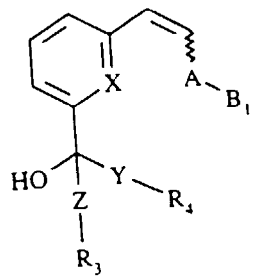

(XVIa)

wherein A, $B_1$, X, Y, Z, $R_3$ and $R_4$ are as defined above,
the amines of formula (XVIb) :

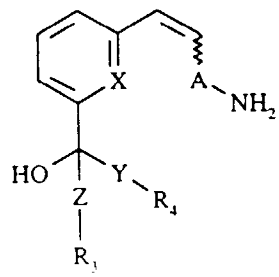

(XVIb)

wherein A, X, Y, Z, $R_3$ and $R_4$ are as defined above,
then being obtained by preparation of the azide, starting from the precursor halogenated compound of formula (XIVg), followed by hydrogenation of that azide,
which amines of formula (XVIb) may be converted by the action of a compound of formula (XVI'):

Hal-$B_2$ (XVI')

wherein $B_2$ represents -$SO_2$Ar or -COR' and Hal, Ar and R' are as defined above,
into compounds of formula (XVIc) :

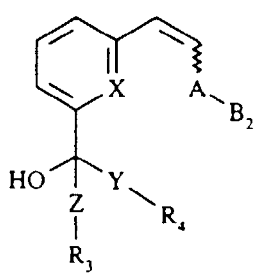

(XVIc)

wherein A, $B_2$, X, Y, Z, $R_3$ and $R_4$ are as defined above,
the totality of the compounds of formulae (XVIa), (XVIb) and (XVIc) together forming the totality of the compounds of formula (XIVi) :

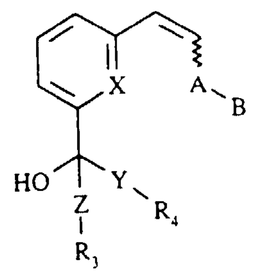

(XIVi)

wherein A, X, Y, Z, $R_3$ and $R_4$ are as defined above and B represents $B_1$, $B_2$ or a $NH_2$ group, it also being possible for the ester of formula (XIIIb) :

1/ - to be hydrolysed to the corresponding acid and then treated with a compound of formula Li-R" wherein R" is as defined above to yield, after freeing the alcohol function which has until then been protected in silylated ether form, the ketones of formula (XIVj) :

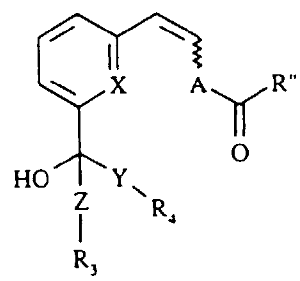

(XIVj)

wherein A, X, Y, Z, R", $R_3$ and $R_4$ are as defined above,

2/ - to be treated with an equivalent of diisobutylaluminium hydride (DIBAL) at low temperature to yield, after freeing the alcohol function, the aldehydes of formula (XIVk) :

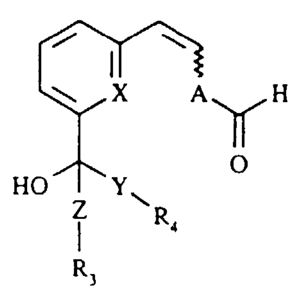

(XIVk)

wherein A, X, Y, Z, $R_3$ and $R_4$ are as defined above,

it being possible for those aldehydes of formula (XIVk) to be

***either :*** α) treated with hydroxylamine to yield the oximes of formula (XIVl) :

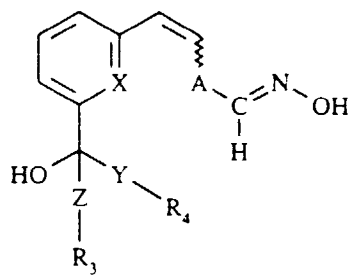

(XIVl)

wherein A, X, Y, Z, $R_3$ and $R_4$ are as defined above,
it being possible for the oximes of formula (XIVl) in turn to be alkylated to compounds of formula (XIVm) :

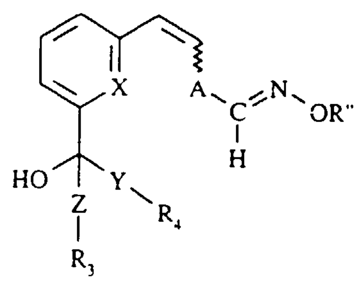

(XIVm)

wherein A, X, Y, Z, R", $R_3$ and $R_4$ are as defined above,
by a reagent of formula W-R" wherein W represents a leaving group and R" is as defined above,

***or :*** β) treated with an amine of formula $H_2N$-R" wherein R" is as defined above to yield the imines of formula (XIVn) :

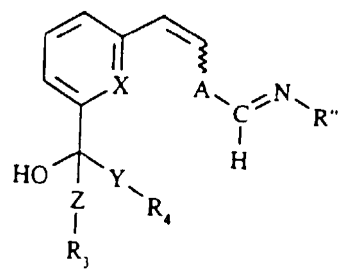

(XIVn)

wherein A, X, Y, Z, R", $R_3$ and $R_4$ are as defined above,

the totality of the compounds of formulae (XIVa) to (XIVn) forming the totality of the compounds of formula (XVII) :

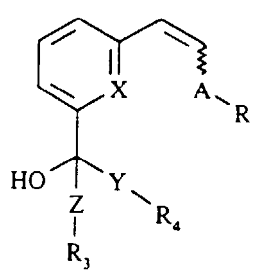

(XVII)

wherein A, X, Y, Z, R, $R_3$ and $R_4$ are as defined above,

which, optionally subjected to hydrogenation according to a conventional technique, yield the compounds of formulae (XVIII) :

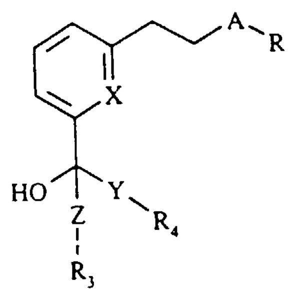

(XVIII)

wherein A, X, Y, Z, R, $R_3$ and $R_4$ are as defined above,

the totality of the compounds of formulae (XVII) and (XVIII) forming the totality of the compounds of formula (I) which, if necessary, are purified according to a conventional purification technique and, if desired, are separated into their isomers by a conventional separation technique, and, optionally, are converted into their N-oxides and/or their pharmaceutically acceptable addition salts with an acid or base.

**9.** Process for the preparation of compounds of formula I according to claim 3 wherein X represents a nitrogen atom, characterised in that the starting material is 2,6-dimethanolpyridine, one of the alcohol functions of which is protected by tert-butyl-diphenyl-silyl chloride to obtain the compound of formula (XIX) :

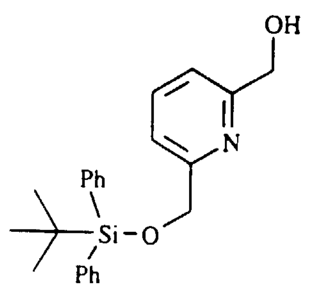

(XIX)

which is then oxidised to the aldehyde of formula (XX) :

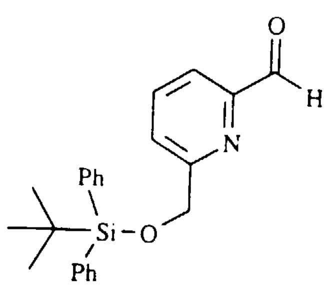

(XX)

which is then subjected to the Wittig reaction described for obtaining the compound of formula (XIIIb), to yield the compound of formula (XXI) :

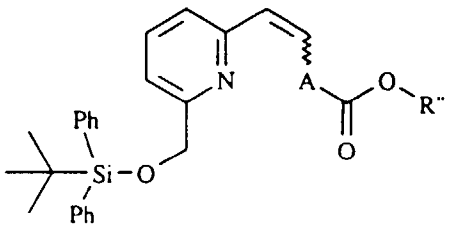

(XXI)

wherein A and R" are as defined above,
the alcohol function of which is deprotected according to the method used for obtaining the compound of formula (XIVa), then oxidised to the aldehyde according to the method described for the compound (XVIII), thus yielding the compound of formula (XXII) :

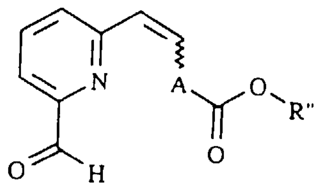

(XXII)

wherein A and R" are as defined above,
which is then treated in an analogous manner to the compound of formula (IV), in accordance with the meanings of Y and $R_4$, to yield the compounds of formula (XXIII) :

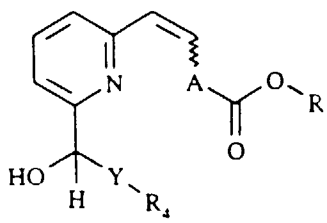

(XXIII)

wherein A, Y, R" and $R_4$ are as defined above,
which are then optionally treated in an analogous manner to the compounds of formula (VI) to obtain the compounds of formula (XXIV) :

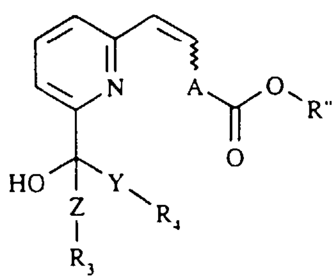

(XXIV)

wherein A, Y, Z, R", $R_3$ and $R_4$ are as defined above,
the ester function of which is optionally modified in an identical manner to the methods used for obtaining the compounds (XIVa) to (XIVn) to yield the compounds of formula (XXV):

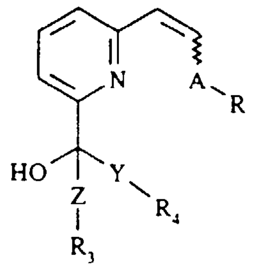

(XXV)

wherein A, Y, Z, R, $R_3$ and $R_4$ are as defined above,
which are optionally subjected to hydrogenation according to the technique described for the hydrogenation of the compounds of formula (XVII) in order to obtain the compounds of formula (XXVI) :

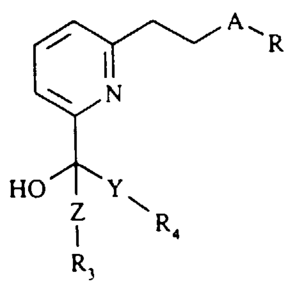

(XXVI)

wherein A, Y, Z, R, $R_3$ and $R_4$ are as defined above,
the totality of the compounds of formulae (XXV) and (XXVI) forming the totality of the compounds of formula (XXVII) :

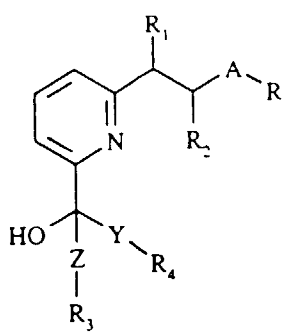

(XXVII)

wherein A, R, $R_1$, $R_2$, $R_3$, $R_4$, Y and Z are as defined above,
a particular case of the compound of formula (I) wherein X represents nitrogen, which, if necessary, are purified according to a conventional purification technique and which, if desired, are separated into their isomers by a conventional separation technique and which, if necessary, are converted into their pharmaceutically acceptable addition salts with an acid or base.

**10.** Pharmaceutical compositions comprising as active ingredient at least one compound according to any one of claims 1 to 7, alone or in combination with one or more pharmaceutically acceptable, inert, non-toxic carriers.

**11.** Pharmaceutical composition according to claim 10 having an anti platelet-aggregation activity and useful in the treatment of disorders resulting from or associated with platelet adhesion pathologies and especially in the case of thromboembolism, by dissolving blood clots, in the case of pulmonary embolism, arterial embolism of the extremities, myocardial infarct, atherosclerosis, malignant cancer, and also in maintaining blood homeostasis, in particular in extracorporeal circulation.